(19) Europäisches Patentamt
European Patent Office
Office européen des brevets

(11) EP 3 547 385 B1

(12) **EUROPEAN PATENT SPECIFICATION**

(45) Date of publication and mention of the grant of the patent:
**04.11.2020 Bulletin 2020/45**

(21) Application number: **17874611.1**

(22) Date of filing: **23.11.2017**

(51) Int Cl.:
***H01L 51/50*** *(2006.01)*

(86) International application number:
**PCT/CN2017/112713**

(87) International publication number:
**WO 2018/095392 (31.05.2018 Gazette 2018/22)**

(54) **ORGANIC MIXTURE, COMPOSITION, AND ORGANIC ELECTRONIC COMPONENT**

ORGANISCHE MISCHUNG, ZUSAMMENSETZUNG UND ORGANISCHES ELEKTRONISCHES BAUELEMENT

MÉLANGE ORGANIQUE, COMPOSITION ET COMPOSANT ÉLECTRONIQUE ORGANIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.11.2016 CN 201611046904**

(43) Date of publication of application:
**02.10.2019 Bulletin 2019/40**

(73) Proprietor: **Guangzhou Chinaray Optoelectronic Materials Ltd.**
**Guangzhou, Guangdong 510663 (CN)**

(72) Inventors:
- **PAN, Junyou**
  **Guangzhou**
  **Guangdong 510663 (CN)**
- **HE, Ruifeng**
  **Guangzhou**
  **Guangdong 510663 (CN)**
- **TAN, Jiahui**
  **Guangzhou**
  **Guangdong 510663 (CN)**
- **HUANG, Hong**
  **Guangzhou**
  **Guangdong 510663 (CN)**

(74) Representative: **Biggi, Cristina**
**Bugnion S.p.A.**
**Viale Lancetti 17**
**20158 Milano (IT)**

(56) References cited:
**CN-A- 101 030 624**
**CN-A- 103 985 822**
**CN-A- 105 679 949**
**US-A1- 2007 078 267**

- **SATOSHI SEO ET AL: "Exciplex-triplet energy transfer: A new method to achieve extremely efficient organic light-emitting diode with external quantum efficiency over 30% and drive voltage below 3 V", JAPANESE JOURNAL OF APPLIED PHYSICS, vol. 53, no. 4, 17 March 2014 (2014-03-17) , pages 042102-1, XP055559909, JP ISSN: 0021-4922, DOI: 10.7567/JJAP.53.042102**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 547 385 B1

## Description

### CROSS-REFERENCE TO RELATED APPLICATIONS

The present application is the national phase of International Application PCT/CN2017/112713, filed on Nov. 23, 2017, which claims priority to Chinese Application No. 201611046904.3, filed on Nov. 23, 2016.

### TECHNICAL FIELD

The present disclosure relates to electronic devices, and in particular to an organic mixture, formulation and an organic electronic device.

### BACKGROUND

With the properties of light weight, active emitting, wide viewing angle, high contrast, high emitting efficiency, low energy consumption, easy preparation for flexible and large-sized panels, etc., organic light-emitting diodes (OLEDs) are regarded as the most promising next-generation display technology in the industry.

In order to promote the large-scale industrialization of the organic light-emitting diodes, further improving the luminescence properties and lifetime of the organic light-emitting diodes is a key issue that needs to be solved urgently, and high-performance organic optoelectronic material systems will still need to be further developed.

The host material is the key element for obtaining efficient and long-lifetime light-emitting diodes. Since the organic light-emitting diodes using phosphorescent materials can achieve nearly 100% internal electroluminescence quantum efficiency, the phosphorescent materials, especially, red and green phosphorescent materials, have become the mainstream material system in the industry. However, the phosphorescent OLEDs have a significant problem of Roll-off effect, i.e., the phenomenon that the emitting efficiency decreases rapidly with the increase of current or voltage, due to the charge imbalance in the device, which is particularly disadvantageous for high brightness applications. In order to solve the above problem, Kim et al. (see Kim et al.Adv. Func. Mater. 2013 DOI: 10.1002/adfm.201300547, and Kim et al.Adv. Func. Mater. 2013,DOI: 10.1002/adfm.201300187) obtained the OLEDs with low Roll-off and high efficiency by using a co-host that can form an exciplex together with another metal complex as the phosphorescent emitter. SATOSHI SEO et al. (SATOSHI SEO et al., JAPANESE JOURNAL OF APPLIED PHYSICS, 2014 DOI: 10.7567/JJAP.53.042102) obtained highly efficient and low-voltage OLEDs by employing energy transfer from an exciplex formed between an electron-transporting material with a $\pi$-deficient quinoxaline moiety and a hole-transporting material with aromatic amine structure to a phosphorescent emitter.

However, the lifetime of such OLED devices containing a co-host still needs to be greatly improved. So far, it is still unclear how to design and combine two different hosts to achieve high-performance OLEDs.

### SUMMARY

In view of the deficiencies of the prior art mentioned above, a purpose of the present disclosure is to provide an organic mixture, to provide an effective technical solution for the material of the OLED device containing a co-host and specifies a co-host design method.

Technical solution of the disclosure is described below.

An organic mixture comprising an organic compound H1 and an organic compound H2 is provided, wherein, 1) $\min((LUMO(H1)-HOMO(H2), LUMO(H2)-HOMO(H1)) \leq \min(E_T(H1), E_T(H2))+0.1eV$, wherein, LUMO(H1), HOMO(H1) and $E_T(H1)$ are the lowest unoccupied molecular orbital, highest occupied molecular orbital and triplet excited state energy levels of the organic compound H1, respectively, and LUMO(H2), HOMO(H2) and $E_T(H2)$ are the lowest unoccupied molecular orbital, highest occupied molecular orbital and triplet excited state energy levels of the organic compound H2, respectively; 2) $(LUMO+1)(H1)-LUMO(H1) \geq 0.1eV$, wherein, (LUMO+1)(H1) is the second lowest unoccupied molecular orbital energy level of the organic compound H1.

In one embodiment, $(LUMO+1)(H1)-LUMO(H1) \geq 0.15$ eV.

In one embodiment, the organic compound H1 is a compound represented by the general formula (1):

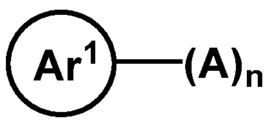

(1)

wherein, $Ar^1$ is selected from H atom, an aromatic group containing 5 to 90 ring atoms or a heteroaromatic group containing 5 to 90 ring atoms; A is an electron-accepting group; n is an integer from 1 to 6; when n is greater than 1, a plurality of the electron-accepting groups A are the same or different.

In some embodiment, the electron-accepting group is F or cyano, while $Ar^1$ is not H atom; or the electron-accepting group comprises a group formed by one or more of the following structures:

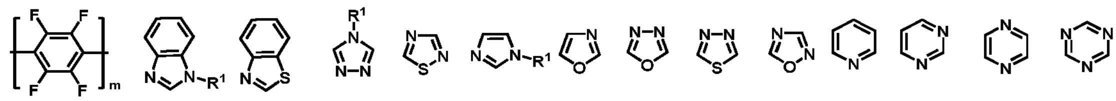

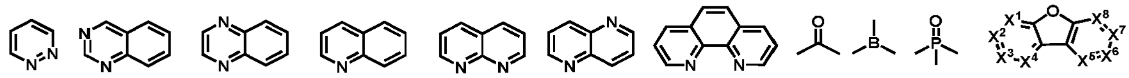

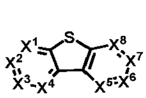
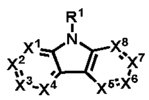
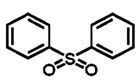
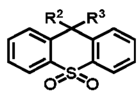
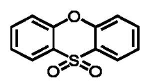
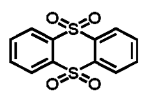
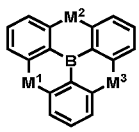

wherein, m is 1, 2 or 3; $X^1$ to $X^8$ are selected from CR or N, and at least one of $X^1$ to $X^8$ is N; $M^1$, $M^2$ and $M^3$ each independently represent N(R), $C(R)_2$, $Si(R)_2$ , O, C=N(R), $C=C(R)_2$, P(R), P(=O)R, S, S=O, $SO_2$ or none; wherein $R^1$, $R^2$, $R^3$ and R each independently represent alkyl, alkoxy, amino, alkenyl, alkynyl, aralkyl, heteroalkyl, aryl or heteroaryl.

In one embodiment, the organic compound H2 is a compound represented by the general formula (2):

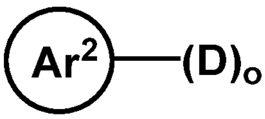
(2)

wherein, $Ar^2$ is selected from H atom, an aromatic group containing 5 to 90 ring atomsor a heteroaromatic group containing 5 to 90 ring atoms; D is an electron-donating group; o is an integer from 1 to 6; when o is greater than 1, a plurality of the electron-donating groups are the same or different.

In some embodiment, the electron-donating group comprises a group formed by one or more of the following structures:

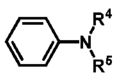
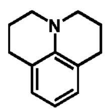
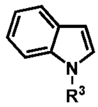
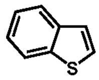
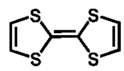
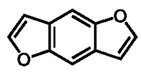

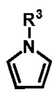
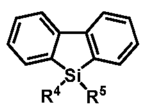
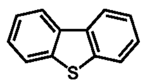
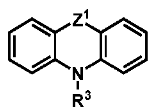
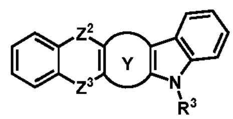

wherein, Y represents an aromatic group containing 5 to 40 carbon atoms or a heteroaromatic group containing 5 to 40 carbon atoms; $Z^1$, $Z^2$ and $Z^3$ each independently represent a single bond, N(R), $C(R)_2$, $Si(R)_2$ , O, S, C=N(R), $C=C(R)_2$ or P(R); and R, $R^3$, $R^4$ and $R^5$ each independently represent alkyl, alkoxy, amino, alkenyl, alkynyl, aralkyl, heteroalkyl, aryl or heteroaryl.

In some embodiment, $Ar^1$ or $Ar^2$ comprises a group including one or more of the following structures:

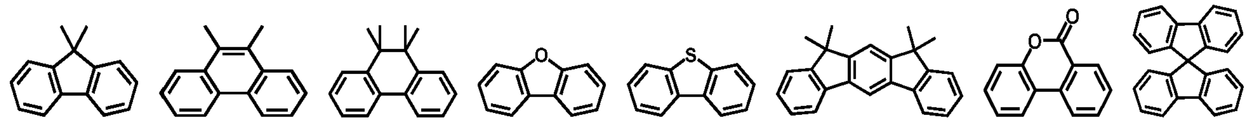

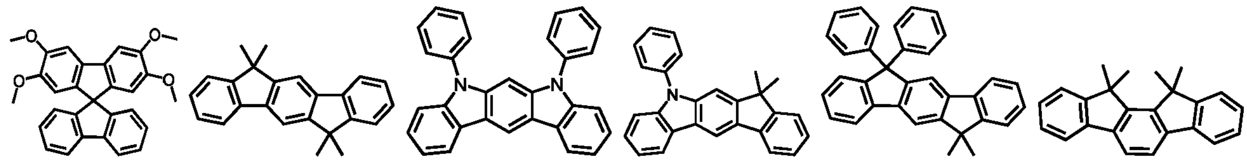

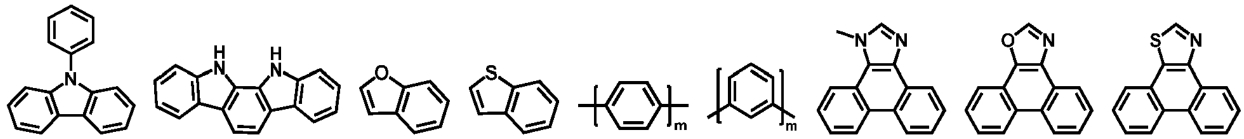

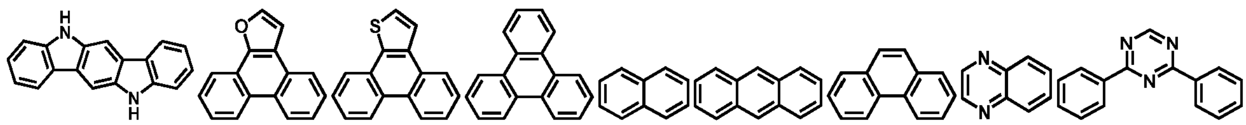

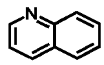

wherein, m is 1, 2 or 3.

In some embodiment, the organic compound H1 is selected from one or more of the compounds represented by the following structural formulas:

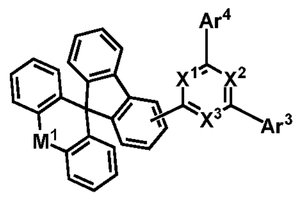 (3) , 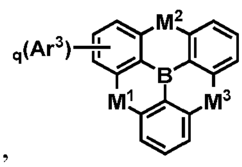 (4) ,

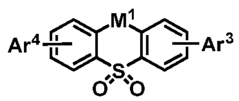 (5) and 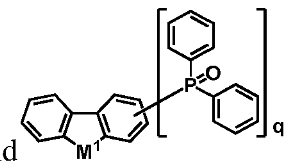 (6) ,

wherein, $Ar^3$ and $Ar^4$ are each independently selected from an aromatic group containing 5 to 60 ring atoms or a heteroaromatic group containing 5 to 60 ring atoms; q is an integer from 1 to 6; $X^1$, $X^2$ and $X^3$ are each independently selected from CR or N, and at least one of $X^1$, $X^2$ and $X^3$ is N; $M^1$, $M^2$ and $M^3$ each independently represent N(R), $C(R)_2$, $Si(R)_2$ , O, C=N(R), $C=C(R)_2$, P(R), P(=O)R, S, S=O, $SO_2$ or none; wherein, R represents alkyl, alkoxy, amino, alkenyl, alkynyl, aralkyl, heteroalkyl, aryl or heteroaryl.

Particularly, the organic compound H1 is selected from one or more of the following compounds:

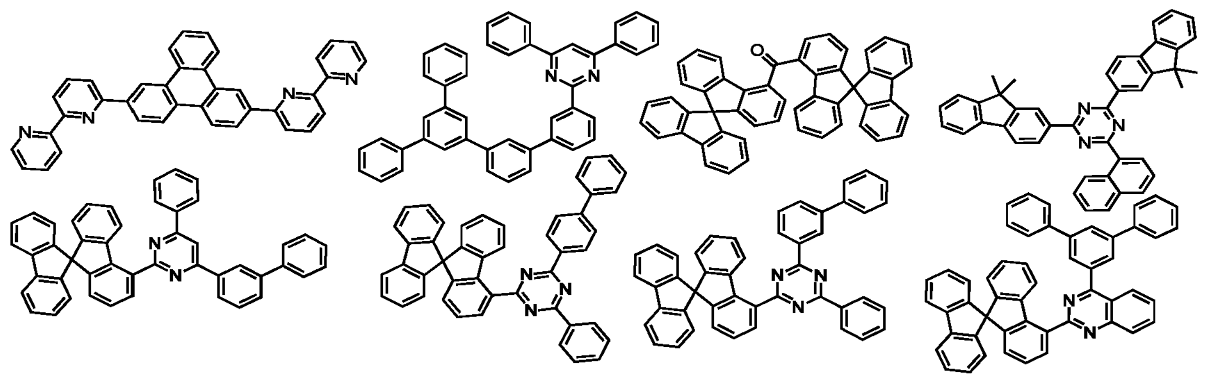

,

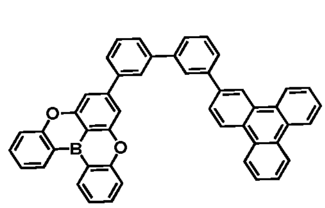 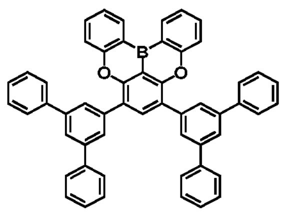 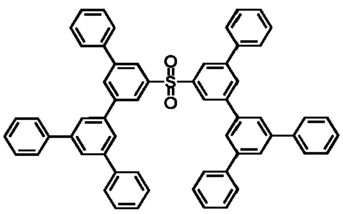 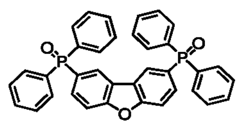

.

Particularly, the organic compound H2 is selected from one or more of the following compounds:

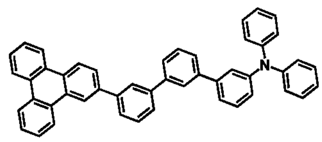 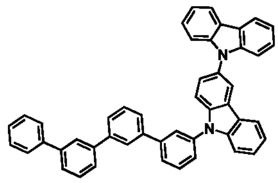 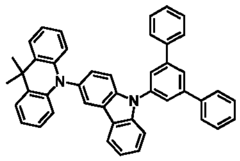 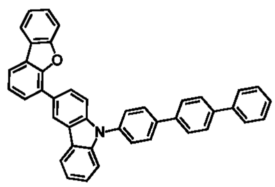

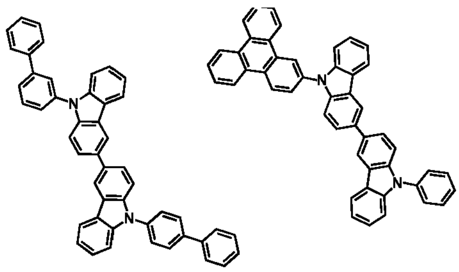 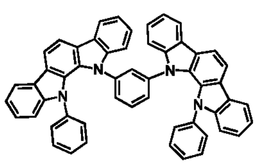 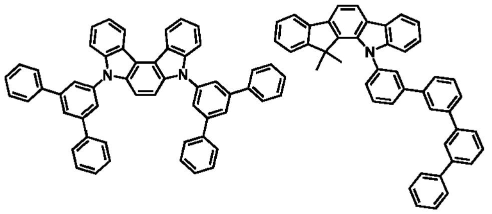

.

In one embodiment, the molar ratio of the organic compound H1 to the organic compound H2 ranges from 2:8 to 8:2.

In one embodiment, the difference in molecular weight between the organic compound H1 and the organic compound H2 does not exceed 100 Dalton.

In one embodiment, the difference in sublimation temperature between the organic compound H1 and the organic compound H2 does not exceed 30 K.

In one embodiment, the organic mixture further comprises a light-emitting material selected from at least one of a fluorescent emitter, a phosphorescent emitter and a TADF material.

A formulation comprising the organic mixture and an organic solvent is also provided.

An organic electronic device comprising a functional layer including the organic mixture is further provided.

In one embodiment, the organic electronic device is an organic light-emitting diode (OLED), an organic photovoltaic cell (OPV), an organic light-emitting electrochemical cell (OLEEC), an organic field effect transistor (OFET), an organic light-emitting field effect transistor, an organic sensor or an organic plasmon emitting diode.

In one embodiment, the functional layer is a light-emitting layer.

In one embodiment, the method for preparing the functional layer includes:

mixing the organic compound H1 and the organic compound H2 uniformly and depositing the same as one source; or evaporating the organic compound H1 and the organic compound H2 as two separate sources.

Advantageous Effects: by applying the organic mixture composed of an electron type compound and a hole type compound according to the present disclosure as a host material to the organic light-emitting diode, high emitting efficiency and device lifetime can be achieved. The possible reasons are, but not limited to: the bipolar mixture has suitable HOMO and LUMO energy levels, which is beneficial to reduce the barrier of electron and hole injection, and easy to achieve the balance of carrier transport, thereby reducing the turn-on voltage and roll-off effect of the OLED device; at the same time, the compound having electron transporting property in the mixture has a larger (LUMO+1)-LUMO, so that the device can be stable at work; and the energy transfer intermediate state of exciplex with smaller singlet and triplet energy level difference is formed between two host materials, so that the energy of the exciton can be more fully utilized, thereby effectively improving the efficiency and lifetime of the device.

## DETAILED DESCRIPTION OF THE EMBODIMENTS

In order to make the purpose, technical solution and advantages of the present disclosure clearer, the embodiments of the present disclosure will be further described in detail below with reference to the accompanying drawings. It should be noted that, the specific embodiment illustrated herein is merely for the purpose of explanation, and should not be deemed to limit the disclosure.

In the present disclosure, formulation and printing ink, or ink, have the same meaning and they can be used interchangeably; host material, matrix material, Host or Matrix material have the same meaning and they can be used interchangeably; metal organic complex, metal organic complex, and organometallic complex have the same meaning and can be used interchangeably; The electron-rich group has the same meaning as the electron-donating group, and the electron-deficient group has the same meaning as the electron-accepting group.

The present disclosure provides an organic mixture comprising an organic compound H1 and an organic compound H2, wherein, 1) min((LUMO(H1)-HOMO(H2), LUMO(H2)-HOMO(H1)) ≤ min($E_T$(H1), $E_T$(H2))+0.1eV, wherein, LUMO(H1), HOMO(H1) and $E_T$(H1) are the lowest unoccupied orbital, highest occupied orbital and triplet energy levels of the organic compound H1, respectively, and LUMO(H2), HOMO(H2) and $E_T$(H2) are the lowest unoccupied orbital, highest occupied orbital and triplet energy levels of the organic compound H2, respectively; 2) (LUMO+1)(H1)-LUMO(H1) ≥ 0.1eV.

In certain embodiments, the organic compound H1 and the organic compound H2 may form a type II heterojunction structure.

In certain embodiments, the organic compound H1 comprises an electron-accepting group.

In an embodiment, min((LUMO(H1)-HOMO(H2), LUMO(H2)-HOMO(H1)) ≤ min($E_T$(H1), $E_T$(H2));

In one embodiment, min((LUMO(H1)-HOMO(H2), LUMO(H2)-HOMO(H1)) ≤ min($E_T$(H1), $E_T$(H2))-0.05eV;

In one embodiment, min((LUMO(H1)-HOMO(H2), LUMO(H2)-HOMO(H1)) ≤ min($E_T$(H1), $E_T$(H2))-0.1eV;

In one embodiment, min((LUMO(H1)-HOMO(H2), LUMO(H2)-HOMO(H1)) ≤ min($E_T$(H1), $E_T$(H2))-0.15eV;

In one embodiment, min((LUMO(H1)-HOMO(H2), LUMO(H2)-HOMO(H1)) ≤ min($E_T$(H1), $E_T$(H2))-0.2eV;

In the present disclosure, the energy level structure of the organic material, i.e., the triplet energy level $E_T$, HOMO and LUMO play a key role. The determination of these energy levels is described below.

The HOMO and LUMO energy levels can be measured by photoelectric effect, such as XPS (X-ray Photoelectron Spectroscopy) and UPS (Ultroviolet Photoelectron Spectroscopy) or by Cyclic Voltammetry (hereinafter referred to as CV). Recently, quantum chemistry method such as density functional theory (hereinafter referred to as DFT), has also become a feasible method for calculating molecular orbital energy levels.

The triplet energy level $E_T$ of organic materials can be measured by low temperature time-resolved luminescence spectroscopy, or by quantum simulation calculation (e.g., by Time-dependent DFT), such as by the commercial software Gaussian 03W (Gaussian Inc.), and the specific simulation method may refer to WO2011141110 or may be as described in the embodiments below.

It should be noted that, the absolute values of HOMO, LUMO and $E_T$ depend on the measurement method or calculation method used, even for the same method, different HOMO/LUMO value may be obtained by different evaluation methods, such as starting point and peak point on the CV curve. Therefore, reasonable and meaningful comparisons should be made using the same measurement method and the same evaluation method. In the description of the embodiments of the present disclosure, the values of HOMO, LUMO and $E_T$ are based on the simulations of Time-dependent DFT, but this does not affect the application of other measurement or calculation methods.

In the present disclosure, the organic compound H1 and the organic compound H2 are capable of forming an exciplex, one possible benefit of which is that the excited state of the system will preferentially occupy the combined excited state with the lowest energy or to facilitate the energy transfer of the triplet excited state of the organic compound H1 or the organic compound H2 to the combined excited state, so as to improve the concentration of the combined excited state in the organic light-emitting layer of the OLED.

In one embodiment, the organic mixture may be used as a phosphorescent host material.

In the present disclosure, (HOMO-1) is defined as the second highest occupied molecular orbital energy level, (HOMO-2) is the third highest occupied molecular orbital energy level, and so on. (LUMO+1) is defined as the second lowest unoccupied molecular orbital energy level, (LUMO+2) is the third lowest unoccupied molecular orbital energy level, and so on.

In the present disclosure, $\min((LUMO_{H1}\text{-}HOMO_{H2}),(LUMO_{H2}\text{-}HOMO_{H1}))$ is less than or equal to the energy level of the triplet excited state of the organic compound H1 and the organic compound H2. The energy to form exciplex between organic compound H1 and organic compound H2 depends on the $\min((LUMO_{H1}\text{-}HOMO_{H2}), (LUMO_{H2}\text{-}HOMO_{H1}))$.

In one embodiments, the organic compound H1 has $\Delta$((LUMO+1)-LUMO) $\geq$ 0.15eV, further, $\Delta$((LUMO+1)-LUMO) $\geq$ 0.2eV, still further, $\Delta$((LUMO+1)-LUMO) $\geq$ 0.3eV, and even further, $\Delta$((LUMO+1)-LUMO) $\geq$ 0.4eV.

In certain embodiments, at least one of the organic compound H1 and the organic compound H2 has $\Delta$((HOMO-(HOMO-1)) $\geq$ 0.2 eV, further, $\Delta$((HOMO-(HOMO-1))$\geq$ 0.25 eV, still further, $\Delta$((HOMO-(HOMO-1))$\geq$ 0.3 eV, still further, $\Delta$((HOMO-(HOMO-1)) $\geq$ 0.35 eV, still further, $\Delta$((HOMO-(HOMO-1))$\geq$ 0.4 eV, and even further, $\Delta$((HOMO-(HOMO-1)) $\geq$ 0.45 eV. Wherein, the large $\Delta$((HOMO-(HOMO-1))) of compounds is favorable for the stability of hole transport.

In an embodiment, the organic compound H2 has $\Delta$((HOMO-(HOMO-1)) $\geq$ 0.2 eV, further, $\Delta$((HOMO-(HOMO-1)) $\geq$ 0.25 eV, still further, $\Delta$((HOMO-(HOMO-1))$\geq$ 0.3 eV, still further, $\Delta$((HOMO-(HOMO-1))$\geq$ 0.35 eV, still further, $\Delta$((HOMO-(HOMO-1))$\geq$ 0.4 eV, and even further, $\Delta$((HOMO-(HOMO-1))$\geq$ 0.45 eV. Wherein, the organic compound H2 mainly plays a key role in hole transport in the mixture, and its hole transport stability is more important.

In some embodiments, the organic compound H1 is at least one of the compounds represented by the general formula (1):

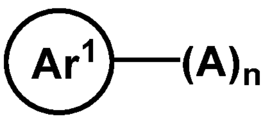
(1)

wherein, $Ar^1$ is selected from H atom, an aromatic group containing 5 to 90 ring atoms or a heteroaromatic group containing 5 to 90 ring atoms; A is an electron-accepting group;

n is an integer from 1 to 6; when n is greater than 1, n of electron-accepting groups A may be the same or different.

In certain embodiments, the above electron-accepting group A may be selected from F and cyano, and $Ar^1$ is not hydrogen atom in this case; or the electron-accepting group A may comprise a structure including the following groups:

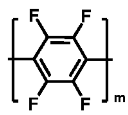 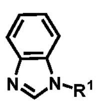 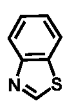 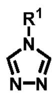 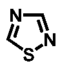 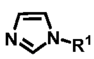 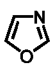 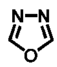 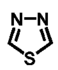 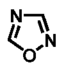 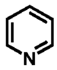 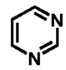 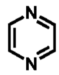 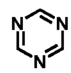

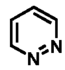 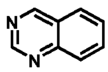 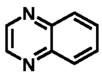 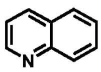 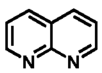 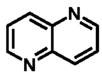 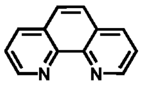 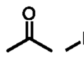 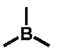 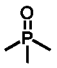 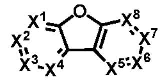

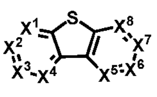 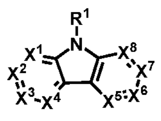 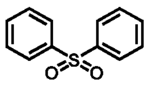 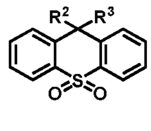 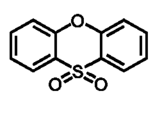 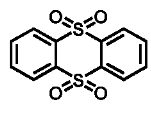 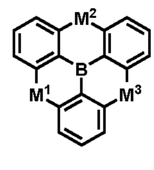

wherein, m is 1, 2 or 3; $X^1$ to $X^8$ are selected from CR or N, and at least one of $X^1$ to $X^8$ is N; $M^1$, $M^2$ and $M^3$ each independently represent N(R), $C(R)_2$, $Si(R)_2$ , O, C=N(R), $C=C(R)_2$, P(R), P(=O)R, S, S=O, $SO_2$ or none; wherein $R^1$, $R^2$, $R^3$ and R each independently represent alkyl, alkoxy, amino, alkenyl, alkynyl, aralkyl, heteroalkyl, aryl or heteroaryl.

In certain embodiments, R, $R^1$, $R^2$ and $R^3$ each independently represent alkyl containing 1 to 20 carbon atoms, cycloalkyl containing 3 to 20 carbon atoms, aromatic hydrocarbyl containing 6 to 40 carbon atoms or aromatic heterocyclyl containing 3 to 40 carbon atoms. In one embodiments, R, $R^1$, $R^2$ and $R^3$ each independently represent alkyl containing 1 to 15 carbon atoms, cycloalkyl containing 3 to 15 carbon atoms, aromatic hydrocarbyl containing 6 to 30 carbon atoms or aromatic heterocyclyl containing 3 to 30 carbon atoms. In one embodiments, R, $R^1$, $R^2$ and $R^3$ each independently

represent alkyl containing 1 to 10 carbon atoms, cycloalkyl containing 3 to 10 carbon atoms, aromatic hydrocarbyl containing 6 to 20 carbon atoms or aromatic heterocyclyl containing 3 to 20 carbon atoms.

In some embodiment, R, $R^1$, $R^2$ and $R^3$ are each independently selected from the group consisting of methyl, isopropyl, t-butyl, isobutyl, hexyl, octyl, 2-ethylhexyl, benzene, biphenyl, naphthalene, anthracene, phenanthrene, benzophenanthrene, pyrene, pyridine, pyrimidine, triazine, fluorene, dibenzothiophene, silafluorene, carbazole, thiophene, furan, thiazole, triphenylamine, triphenylphosphine oxide, tetraphenyl silicane, spirofluorene, spirosilabifluorene groups and the like; and particularly selected from the group consisting of methyl, isopropyl, t-butyl, isobutyl, benzene, biphenyl, naphthalene, anthracene, phenanthrene, benzophenanthrene, fluorene, spirofluorene groups and the like.

In some embodiments, the organic compound H1 represented by the general formula (1) according to the present disclosure is one or more of the following structural formulas:

| | | |
|---|---|---|
| 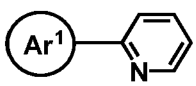 | 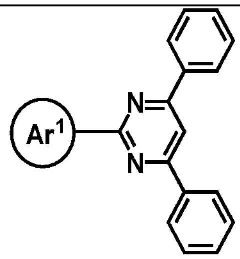 | 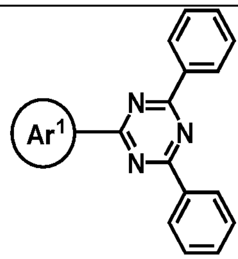 |
| 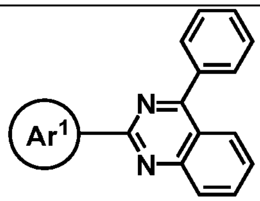 | 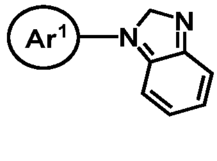 | 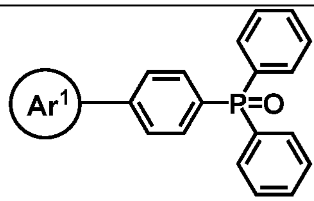 |

In certain embodiments, the organic compound H2 is a compound represented by the general formula (2):

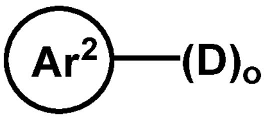
(2)

wherein, $Ar^2$ is selected from H atom, an aromatic group containing 5 to 90 ring atoms or a heteroaromatic group containing 5 to 90 ring atoms; D is an electron-donating group; o is an integer from 1 to 6; when o is greater than 1, o of electron-donating groups D may be the same or different.

In certain embodiments, the above electron-donating group D may be selected from the structure containing any of the following groups:

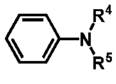 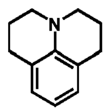 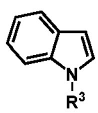 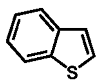 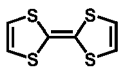 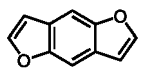

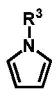 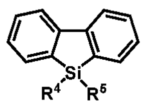 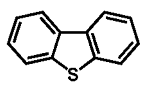 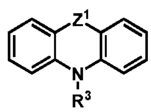 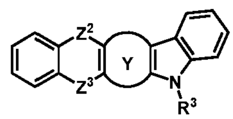

wherein, Y represents an aromatic group containing of 5 to 40 carbon atoms or a heteroaromatic group containing 5 to 40 carbon atoms; $Z^1$, $Z^2$ and $Z^3$ each independently represent a single bond, N(R), $C(R)_2$, $Si(R)_2$ , O, S, C=N(R), $C=C(R)_2$ or P(R); and R, $R^3$, $R^4$ and $R^5$ each independently represent alkyl, alkoxy, amino, alkenyl, alkynyl, aralkyl, heteroalkyl, aryl or heteroaryl.

In an embodiment, the electron-donating group D is selected from one of the following formulas:

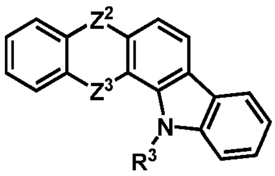,
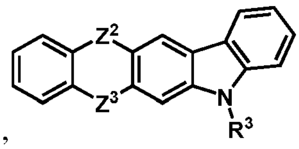,
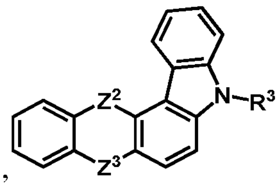,

wherein, Y, $Z^2$, $Z^3$ and $R^3$ are defined as above.

In one embodiment, the electron-donating group D is selected from one of the following formulas:

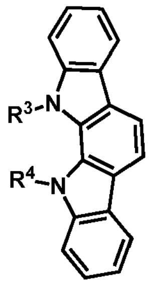
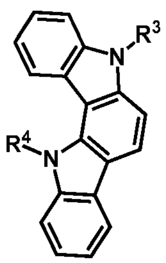
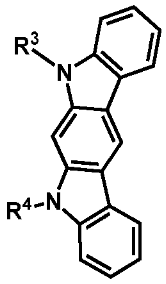
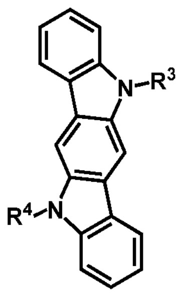
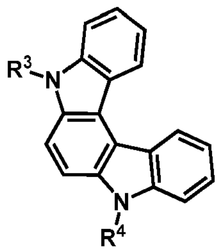
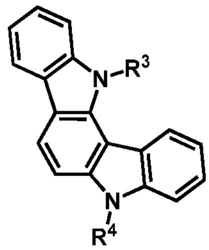,

wherein, $R^3$ and $R^4$ are defined as above.

In some embodiments, the organic compound H2 represented by the general formula (2) according to the present disclosure is one of the following structural formulas:

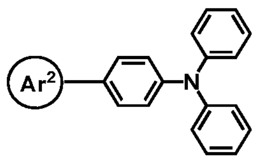
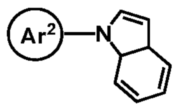
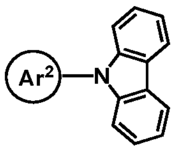
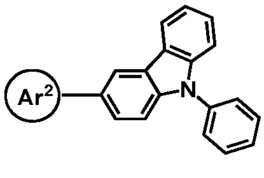
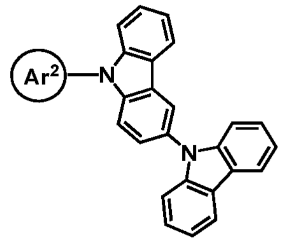
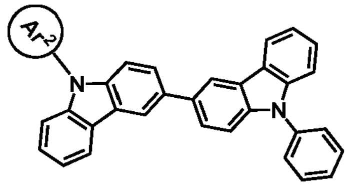
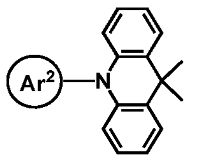
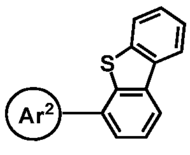
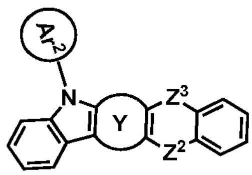

wherein, $Ar^2$, $Z^2$, $Z^3$ and Y are defined as above or below.

In certain embodiments, $Ar^1$ as shown in the general formula (1) and $Ar^2$ as shown in the general formula (2) are aromatic groups containing 6 to 70 ring atoms, or heteroaromatic groups containing 6 to 70 ring atoms. In one embodiment, $Ar^1$ and $Ar^2$ are aromatic groups containing 6 to 60 ring atoms, or heteroaromatic groups containing 6 to 60 ring atoms. In one embodiment, $Ar^1$ and $Ar^2$ are aromatic groups containing 6 to 50 ring atoms, or heteroaromatic groups containing 6 to 50 ring atoms. In one embodiment, $Ar^1$ and $Ar^2$ are aromatic groups containing 6 to 40 ring atoms, or heteroaromatic groups containing 6 to 40 ring atoms.

The aromatic ring system or aromatic group refers to the hydrocarbyl comprising at least one aromatic ring, including monocyclic group and polycyclic ring system. The heteroaromatic ring system or heteroaromatic group refers to the hydrocarbyl comprising at least one heteroaromatic ring (containing heteroatoms), including monocyclic group and polycyclic ring system. The heteroatom is particularly selected from Si, N, P, O, S and/or Ge, especially selected from Si, N, P, O and/or S. Such polycyclic rings may have two or more rings, wherein two carbon atoms are shared by two adjacent rings, i.e., fused ring. At least one of such polycyclic rings is aromatic or heteroaromatic. For the purpose of the present dislosure, the aromatic or heteroaromatic ring systems not only include aromatic or heteroaromatic systems, but also a plurality of aryl or heteroaryl groups in the systems may be interrupted by short non-aromatic units (<10% of non-H atoms, preferably less than 5% of non-H atoms, such as C, N or O atoms). Therefore, systems such as 9,9'-spirobifluorene, 9,9-diarylfluorene, triarylamine, diaryl ether and the like are also considered to be aromatic ring systems for the purpose of this disclosure.

Specifically, examples of the aromatic group include: benzene, naphthalene, anthracene, phenanthrene, perylene, tetracene, pyrene, benzopyrene, triphenylene, acenaphthene, fluorene, and derivatives thereof.

Specifically, examples of the heteroaromatic group include: furan, benzofuran, thiophene, benzothiophene, pyrrole, pyrazole, triazole, imidazole, oxazole, oxadiazole, thiazole, tetrazole, indole, carbazole, pyrroloimidazole, pyrrolopyrrole, thienopyrrole, thienothiophene, furopyrrole, furofuran, thienofuran, benzisoxazole, benzisothiazole, benzimidazole, pyridine, pyrazine, pyridazine, pyrimidine, triazine, quinoline, isoquinoline, cinnoline, quinoxaline, phenanthridine, perimidine, quinazoline, quinazolinone and derivatives thereof.

In one embodiment, $Ar^1$ and $Ar^2$ are each independently selected from the group consisting of benzene, biphenyl, naphthalene, anthracene, phenanthrene, benzophenanthrene, pyrene, pyridine, pyrimidine, triazine, fluorene, dibenzothiophene, silafluorene, carbazole, thiophene, furan, thiazole, triphenylamine, triphenylphosphine oxide, tetraphenyl silicane, spirofluorene, spirosilabifluorene groups and the like; and more preferably from the group consisting of benzene, biphenyl, naphthalene, anthracene, phenanthrene, benzophenanthrene, fluorene,spirofluorene groups and the like.

In one embodiment, $Ar^2$ in chemical formula (1) and $Ar^2$ in chemical formula (2) may comprise one or more combinations of the following structural groups:

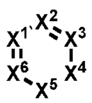 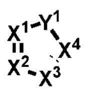 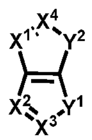 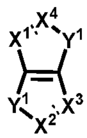 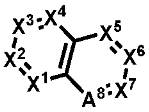 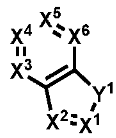 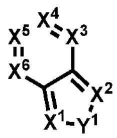 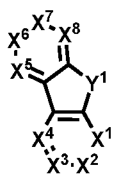

wherein, $X^1$ to $X^8$ each independently represent $CR^3$ or N;

$Y^1$ and $Y^2$ each independently represent $CR^4R^5$, $SiR^4R^5$, $NR^3$, C(=O), S or O;

$R^3$, $R^4$ and $R^5$ are hydrogen atom, or deuterium atom, or linear alkyl containing 1 to 20 carbon atoms, linear alkoxy containing 1 to 20 carbon atoms or linear thioalkoxy groups containing 1 to 20 carbon atoms, or branched or cyclic alkyl containing 3 to 20 carbon atoms, branched or cyclic alkoxy containing 3 to 20 carbon atoms or branched or cyclic thioalkoxy groups containing 3 to 20 carbon atoms ,or silyl group , or substituted keto groups containing 1 to 20 carbon atoms, alkoxycarbonyl groups containing 2 to 20 carbon atoms, aryloxycarbonyl groups containing 7 to 20 carbon atoms, cyano group (-CN), carbamoyl group ($-C(=O)NH_2$), haloformyl group (-C(=O)-X, wherein X represents halogen atom), formyl group (-C(=O)-H), isocyano group, isocyanate group, thiocyanate group, or isothiocyanate group, hydroxyl group, nitro group, CF3 group, Cl, Br, F, a crosslinkable group, or substituted or unsubstituted aromatic ring systems containing 5 to 40 ring atoms or substituted or unsubstituted heteroaromatic ring systems containing 5 to 40 ring atoms, aryloxy groups containing 5 to 40 ring atoms or heteroaryloxy groups containing 5 to 40 ring atoms, or combination of these groups, wherein one or more of the groups $R^3$, $R^4$ and $R^5$ may form a monocyclic or polycyclic aliphatic or aromatic ring system with each other and/or with a ring bonded thereto.

In a particularly embodiment, $Ar^1$ or $Ar^2$ may be selected from structures comprising the following groups:

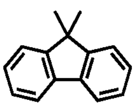 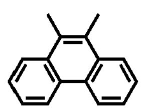 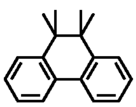 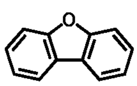 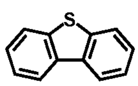 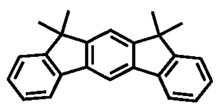 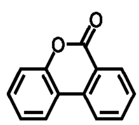 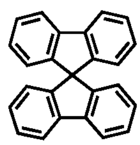

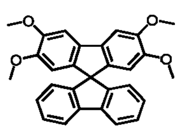 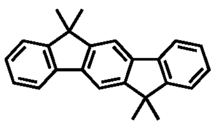 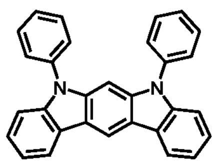 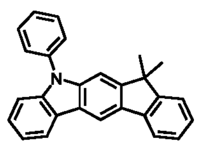 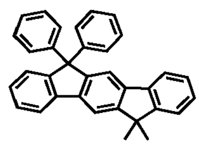 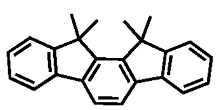

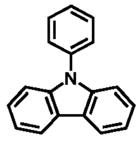 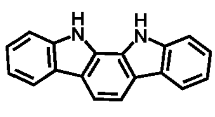 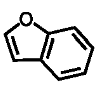 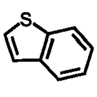 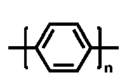 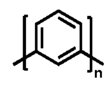 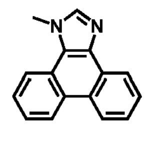 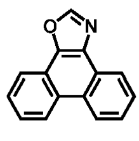 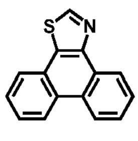

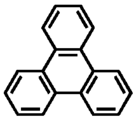 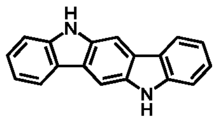 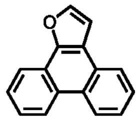 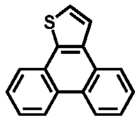 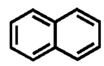 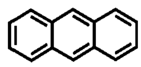 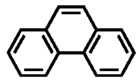 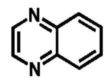

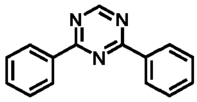 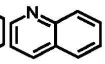

wherein, m is 1, 2 or 3.

In a particularly embodiment, the organic compound H1 is selected from at least one of the compounds represented by general formulas (3) to (6):

| | |
|---|---|
| 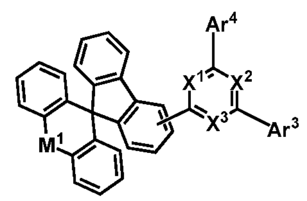 | 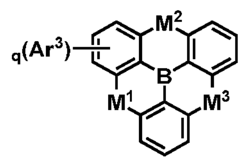 |
| (3) | (4) |
| 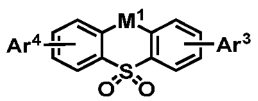 | 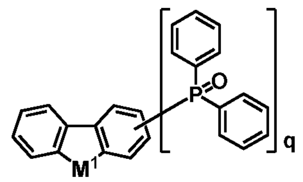 |
| (5) | (6) |

wherein, $Ar^3$ and $Ar^4$ are each independently represent an aromatic group containing 5 to 60 ring atoms or a heteroaromatic group containing 5 to 60 ring atoms; q is an integer from 1 to 6; and $X^1$, $X^2$, $X^3$, $M^1$, $M^2$ and $M^3$ in the general formulas (3) to (6) have the same meanings with those in the general formula (1), and are not described herein again.

In certain embodiments, the organic compound H2 may be selected from at least one of the compounds represented by the following general formulas:

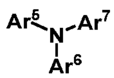 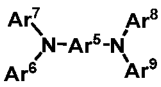 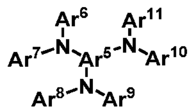 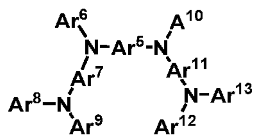 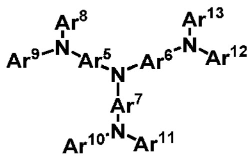

Each of $Ar^5$ to $Ar^{11}$ may be independently selected from the group consisting of cyclic aromatic compound such as benzene, biphenyl, triphenyl, benzo, naphthalene, anthracene, phenalene, phenanthrene, fluorene, pyrene, chrysene, perylene, azulene; or heteroaromatic compound such as dibenzothiophene, dibenzofuran, furan, thiophene, benzofuran, benzothiophene, carbazole, pyrazole, imidazole, triazole, isoxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indolizine, benzoxazole, benzisoxazole, benzothiazole, quinoline, isoquinoline, cinnoline, quinazoline, quinoxaline, naphthalene, phthalein, pteridine, xanthene, acridine, phenazine , phenothiazine, phenoxazine, dibenzoselenophene, benzoselenophene, benzofuropyridine, indolocarbazole, pyridylindole, pyrrolodipyridine, furodipyridine, benzothieopyridine, thienopyridine, benzoselenophenopyridine and selenophenodipyridine; or groups containing 2 to 10 ring structures, which may be the same or different types of aromatic cyclic or heteroaromatic cyclic groups and coupled to each other directly or through at least one of the following groups: such as oxygen atom, nitrogen atom, sulfur atom, silicon atom, phosphorus atom, boron atom, chain structure unit and aliphatic ring group. Wherein, each Ar may be further substituted with the substituent which may be selected from the group consisting of hydrogen, alkyl, alkoxy, amino, alkenyl, alkynyl, aralkyl, heteroalkyl, aryl and heteroaryl.

In certain embodiments, the organic compound H2 may be selected from at least one of the compounds represented by the following general formulas (7) to (10):

| | |
|---|---|
| 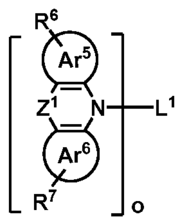 | 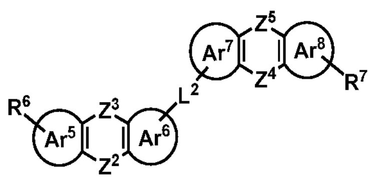 |
| (7) | (8) |
| 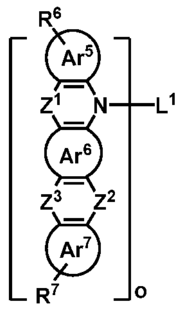 | 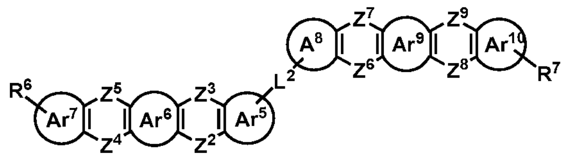 |
| (9) | (10) |

wherein, $L^1$ each independently represents an aromatic group containing 5 to 60 ring atoms or a heteroaromatic group containing 5 to 60 ring atoms;

$L^2$ represents a single bond, an aromatic group containing 5 to 30 ring atoms or a heteroaromatic group containing 5 to 30 ring atons, and $L^2$ may be coupled to any carbon atom in the ring;

$Ar^5$ to $Ar^{10}$ each independently represent an aromatic group containing 5 to 20 ring atoms or a heteroaromatic group containing 5 to 20 ring atoms;

$Z^1$, $Z^2$ and $Z^3$ are defined as above; $Z^4$ to $Z^9$ are defined as $Z^2$, but $Z^4$ and $Z^5$ are not single bonds at the same time, $Z^6$ and $Z^7$ are not single bonds at the same time, and $Z^8$ and $Z^9$ are not single bonds at the same time;

o is an integer from 1 to 6; when o is greater than 1, o of electron-donating groups may be the same or different.

In some embodiments, according to the organic mixture of the present disclosure, H2 is selected from one of the following structural formulas:

| | | |
|---|---|---|
| 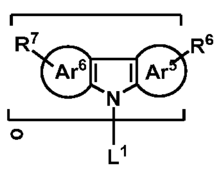 | 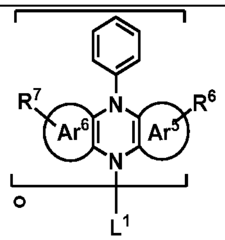 | 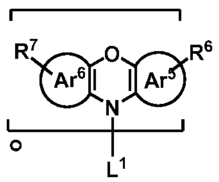 |
| 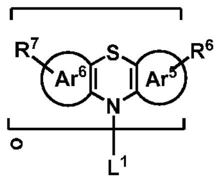 | 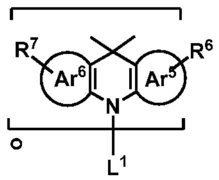 | 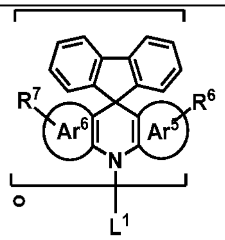 |

wherein, $Ar^5$, $Ar^6$, $R^6$, $R^7$, $L^1$ and o are defined as above.

In other embodiments, according to the organic mixture of the present disclosure, the organic compound H2 is selected from one of the following structural formulas:

| | | |
|---|---|---|
| 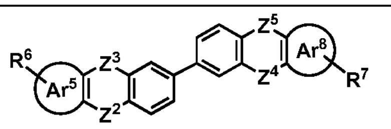 | 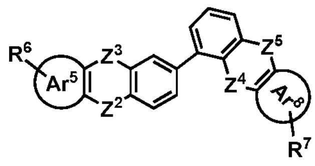 | 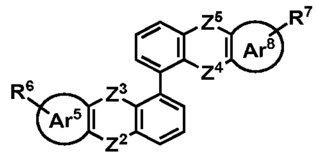 |

wherein, $Ar^5$, $Ar^8$, $Z^2$, $Z^3$, $Z^4$, $Z^5$, $R^6$ and $R^7$ are as defined above.

In other embodiments, according to the organic mixture of the present disclosure, the organic compound H2 is selected from one of the following structural formulas:

| | | |
|---|---|---|
| 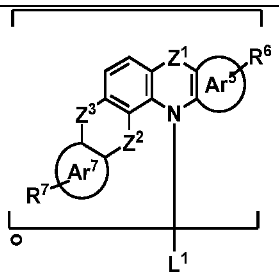 | 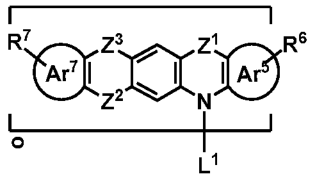 | 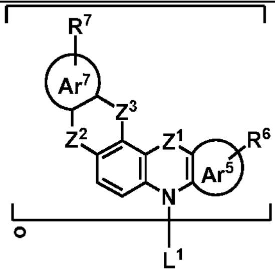 |
| 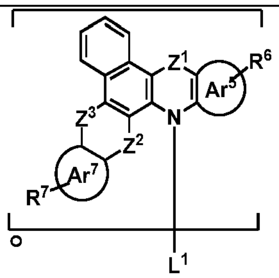 | 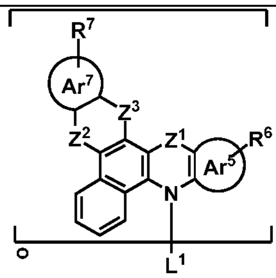 | 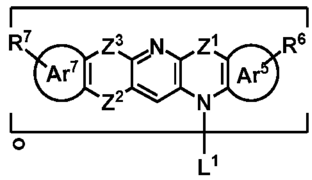 |

wherein, $Ar^5$, $Ar^7$, $Z^1$, $Z^2$, $Z^3$, $R^6$, $R^7$, $L^1$ and o are as defined above.

In other embodiments, according to the organic mixture of the present disclosure, the organic compound H2 is selected from one of the following structural formulas:

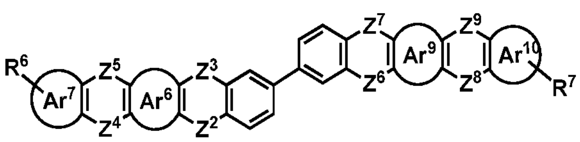

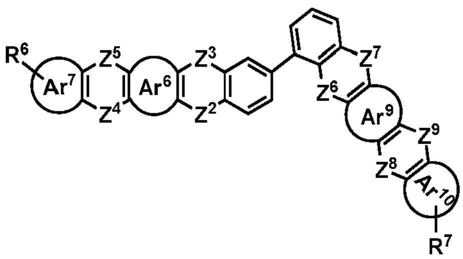

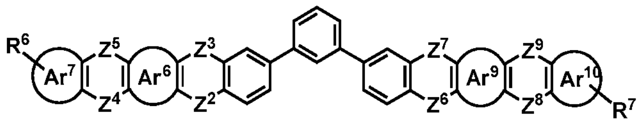

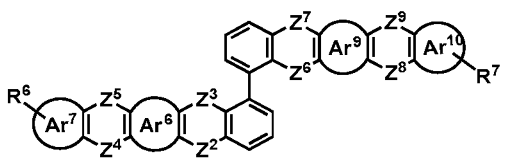

wherein, $Ar^6$, $Ar^7$, $Ar^8$ , $Ar^9$, $Z^2$, $Z^3$, $Z^4$, $Z^5$, $Z^6$, $Z^7$, $Z^8$, $Z^9$, $R^6$ and $R^7$ are as defined above.

Specific examples of the organic compound H1 represented by the general formula (1) and the general formulas (3) to (6) according to the present disclosure are exemplified below, but not limited to:

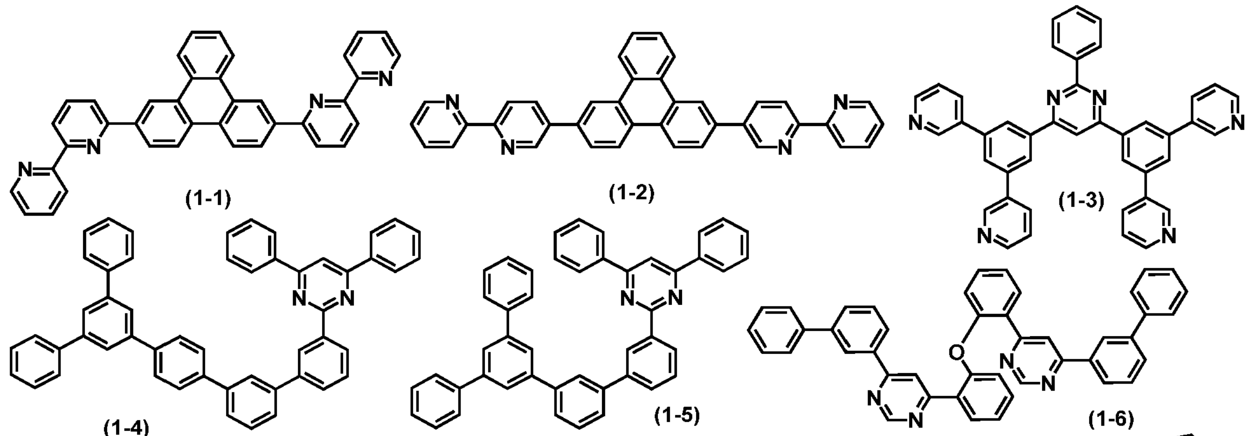

(1-1) (1-2) (1-3) (1-4) (1-5) (1-6)

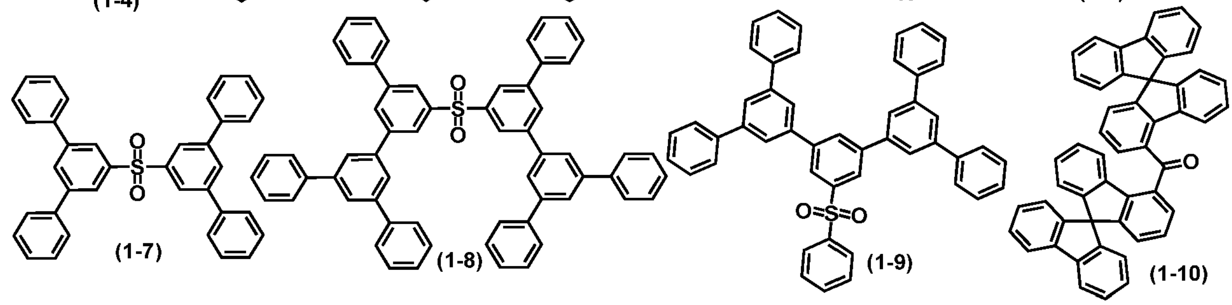

(1-7) (1-8) (1-9) (1-10)

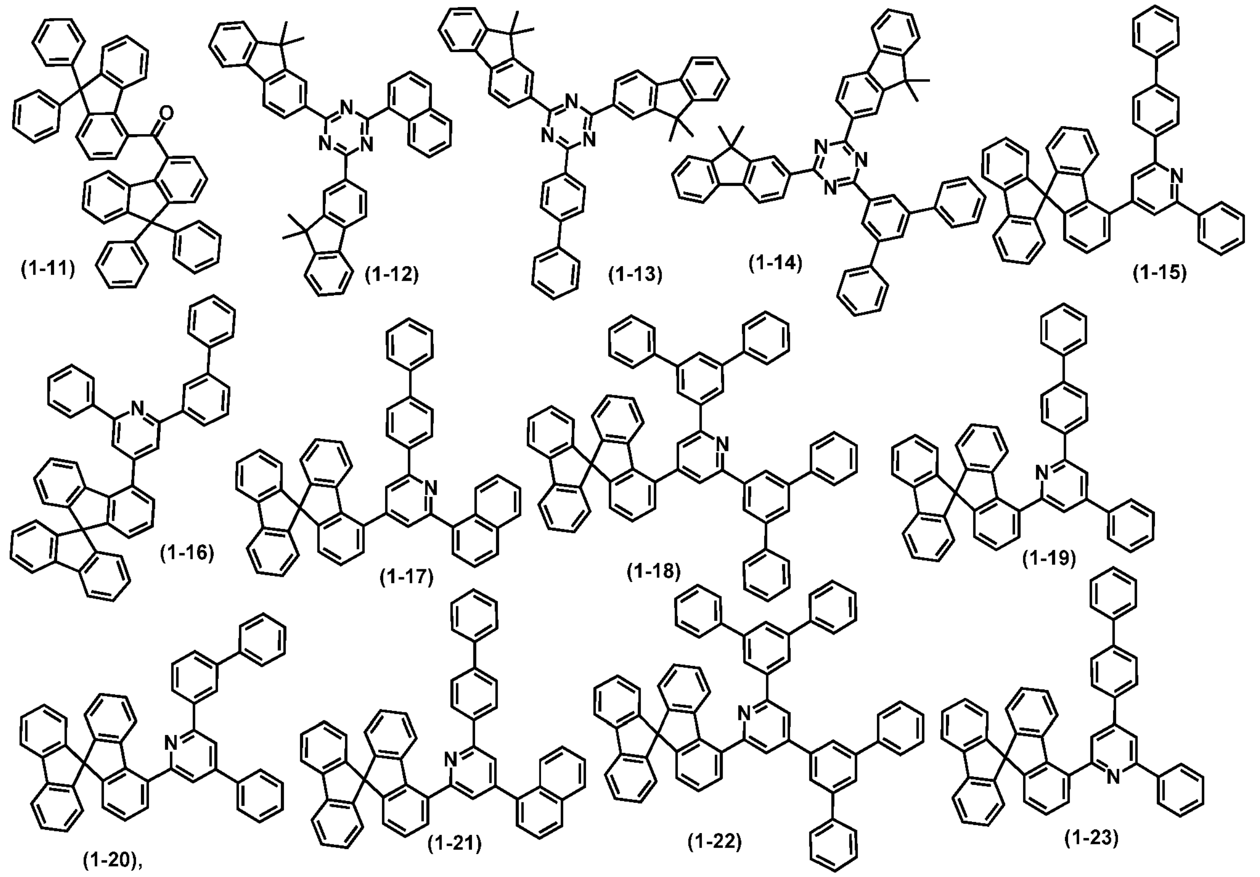

(1-11) (1-12) (1-13) (1-14) (1-15)

(1-16) (1-17) (1-18) (1-19)

(1-20), (1-21) (1-22) (1-23)

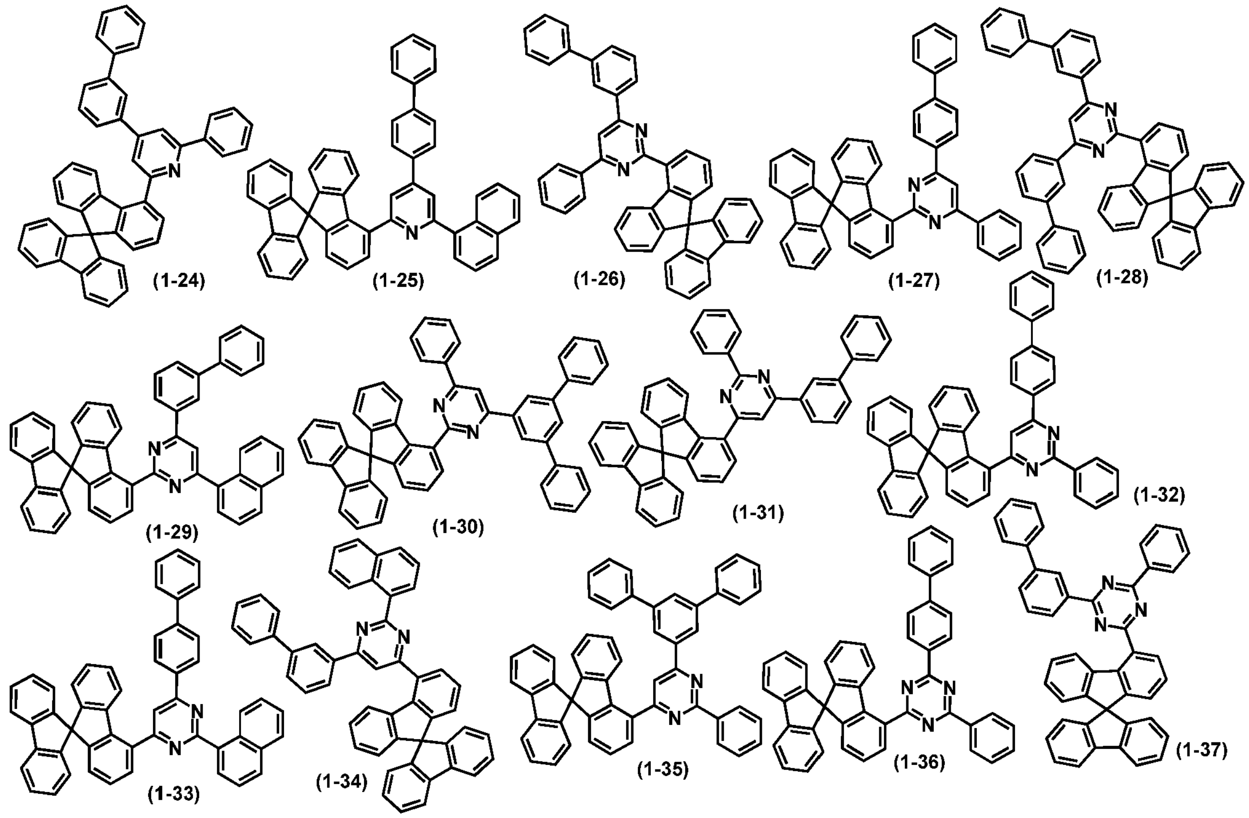

(1-24) (1-25) (1-26) (1-27) (1-28)

(1-29) (1-30) (1-31) (1-32)

(1-33) (1-34) (1-35) (1-36) (1-37)

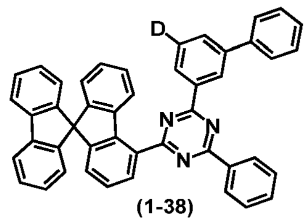

(1-38)

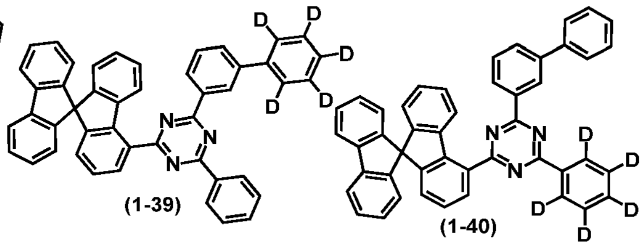

(1-39) (1-40)

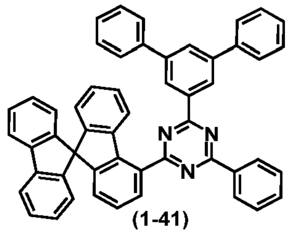

(1-41)

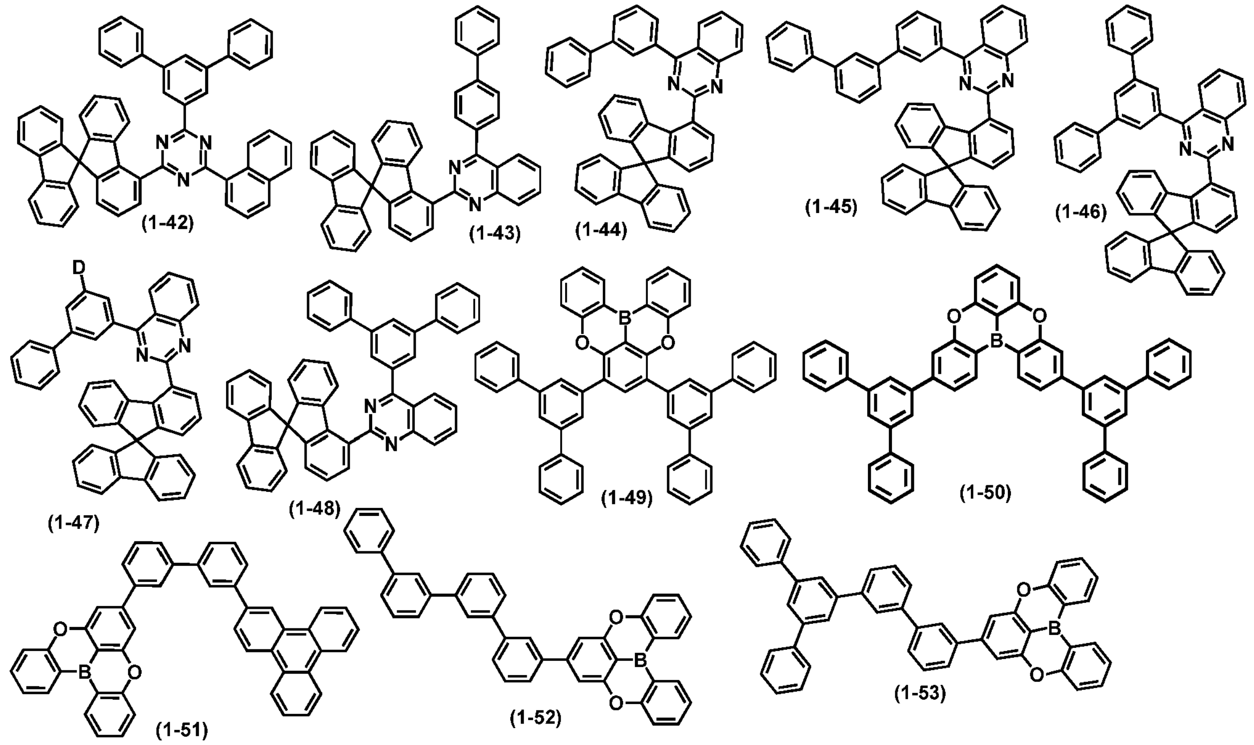

(1-42) (1-43) (1-44) (1-45) (1-46) (1-47) (1-48) (1-49) (1-50) (1-51) (1-52) (1-53)

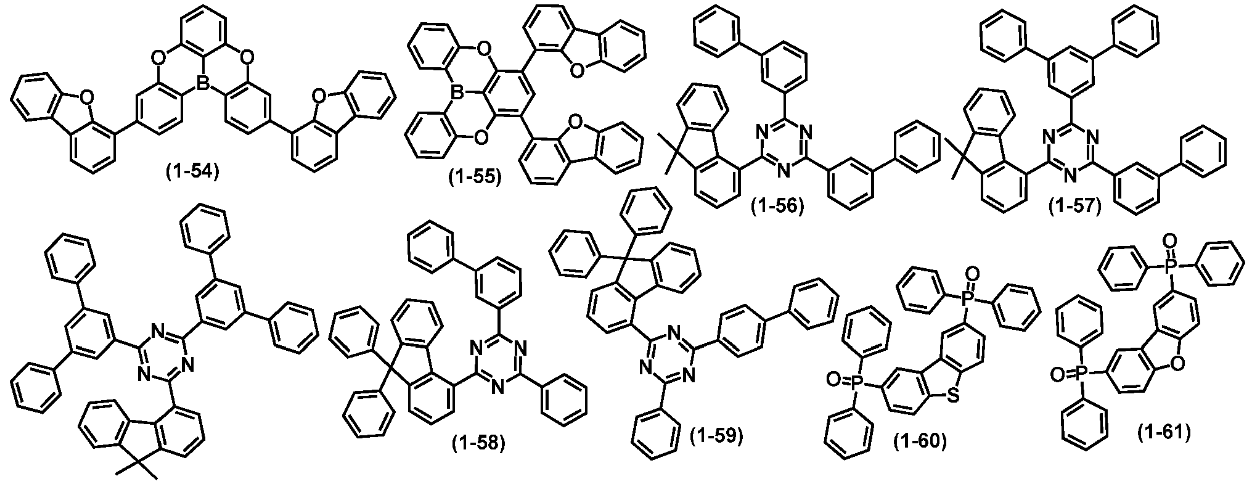

(1-54) (1-55) (1-56) (1-57) (1-58) (1-59) (1-60) (1-61)

Specific examples of the organic compound H2 represented by the general formula (2) and by the general formulas (7) to (10) are exemplified below, but not limited to:

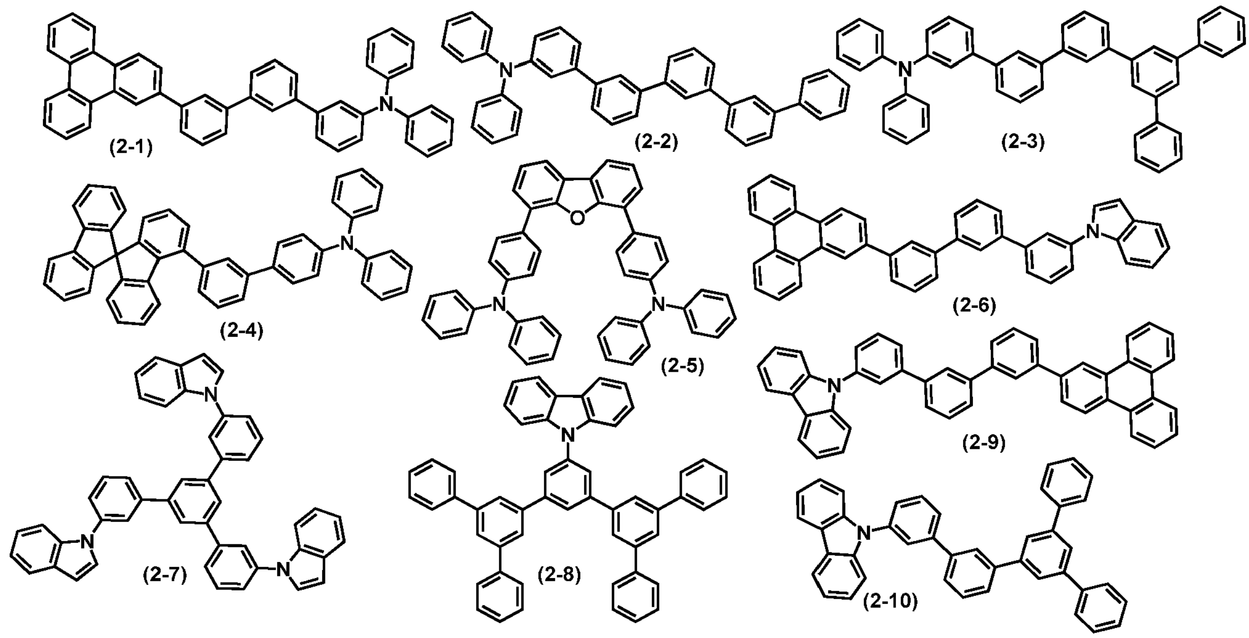

(2-1) (2-2) (2-3)

(2-4) (2-5) (2-6)

(2-7) (2-8) (2-9) (2-10)

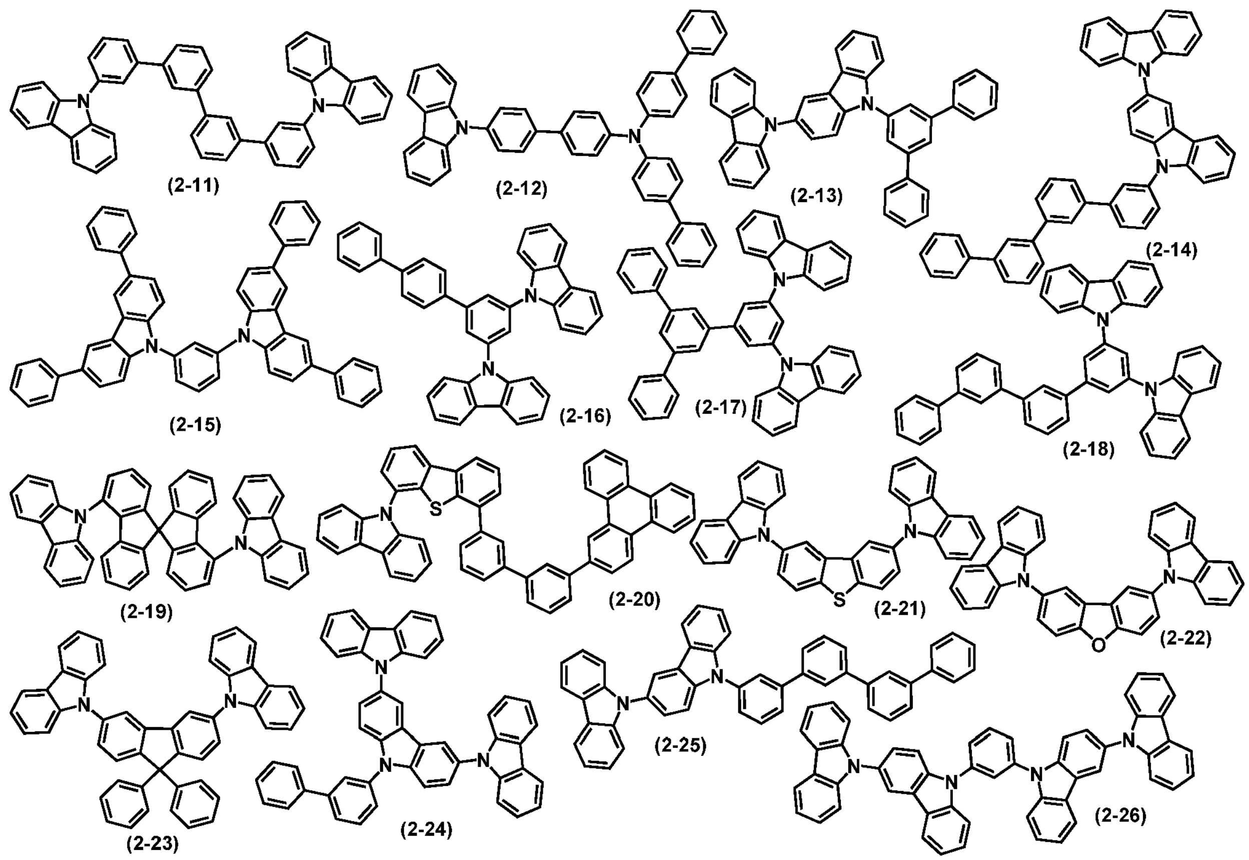

(2-11) (2-12) (2-13) (2-14)

(2-15) (2-16) (2-17) (2-18)

(2-19) (2-20) (2-21) (2-22)

(2-23) (2-24) (2-25) (2-26)

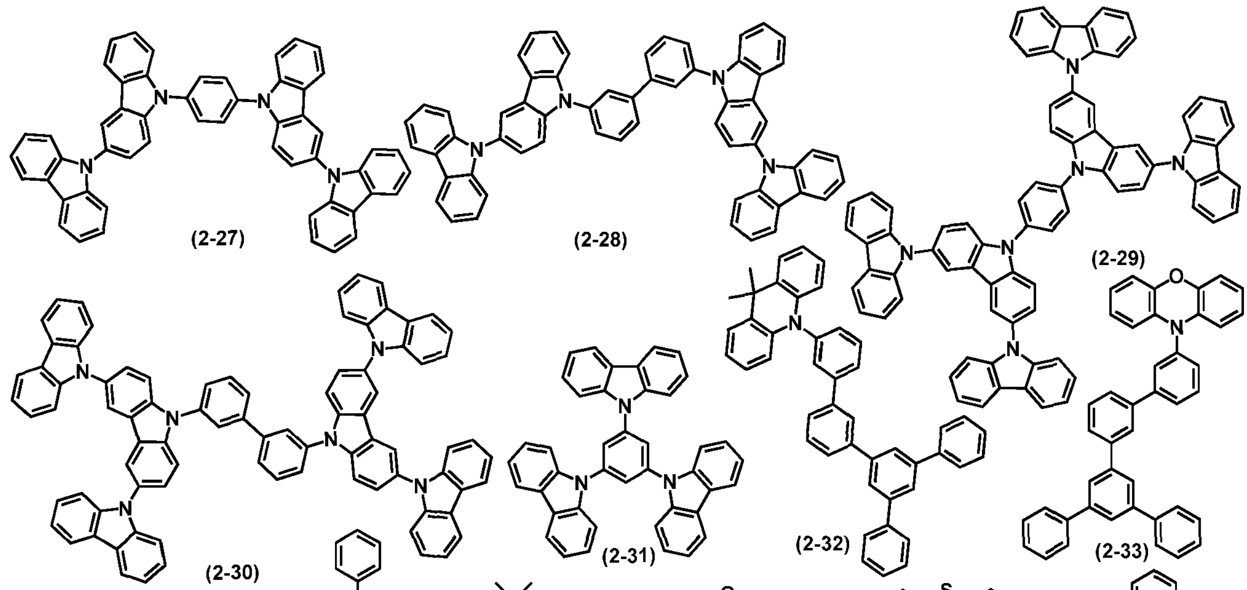

(2-27) (2-28) (2-29)

(2-30) (2-31) (2-32) (2-33)

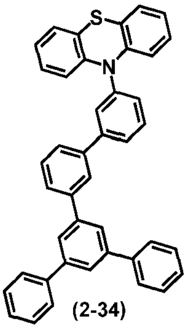

(2-34)

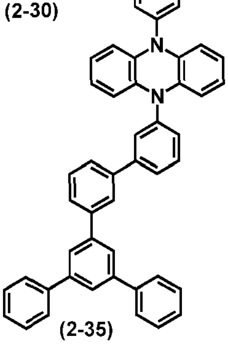

(2-35)

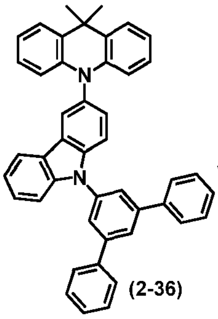

(2-36)

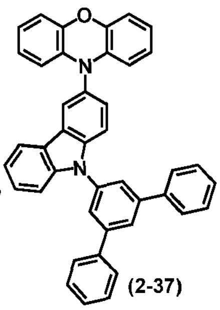

(2-37)

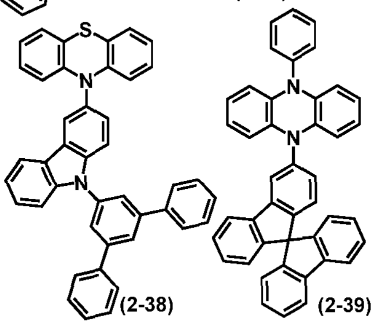

(2-38) (2-39)

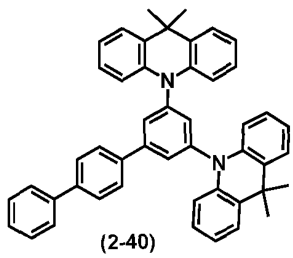
(2-40)

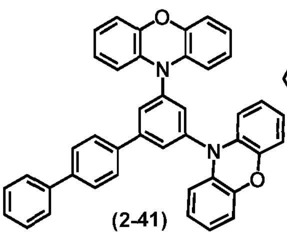
(2-41)

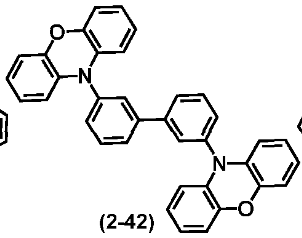
(2-42)

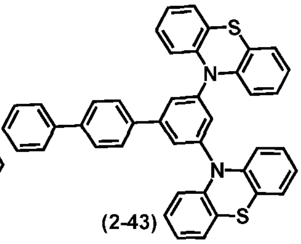
(2-43)

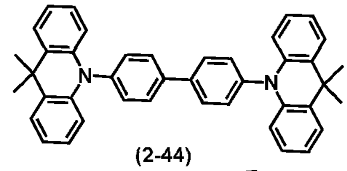
(2-44)

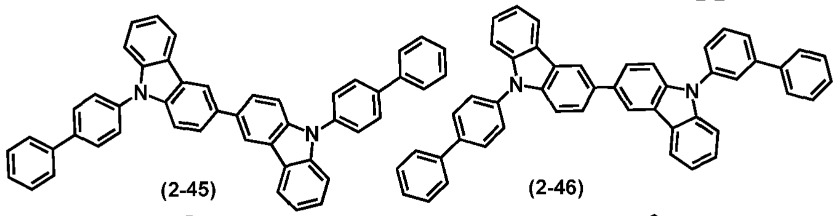
(2-45)

(2-46)

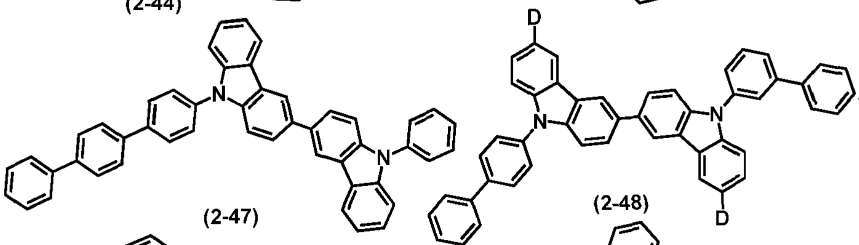
(2-47)

(2-48)

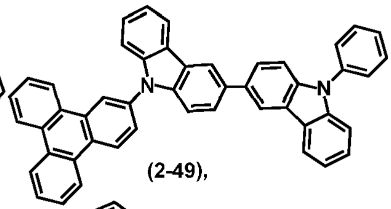
(2-49),

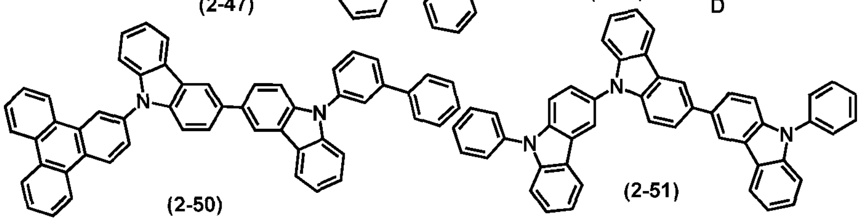
(2-50)

(2-51)

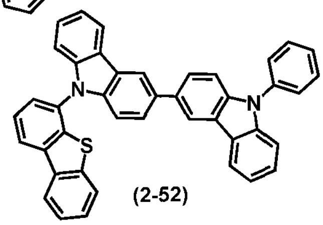
(2-52)

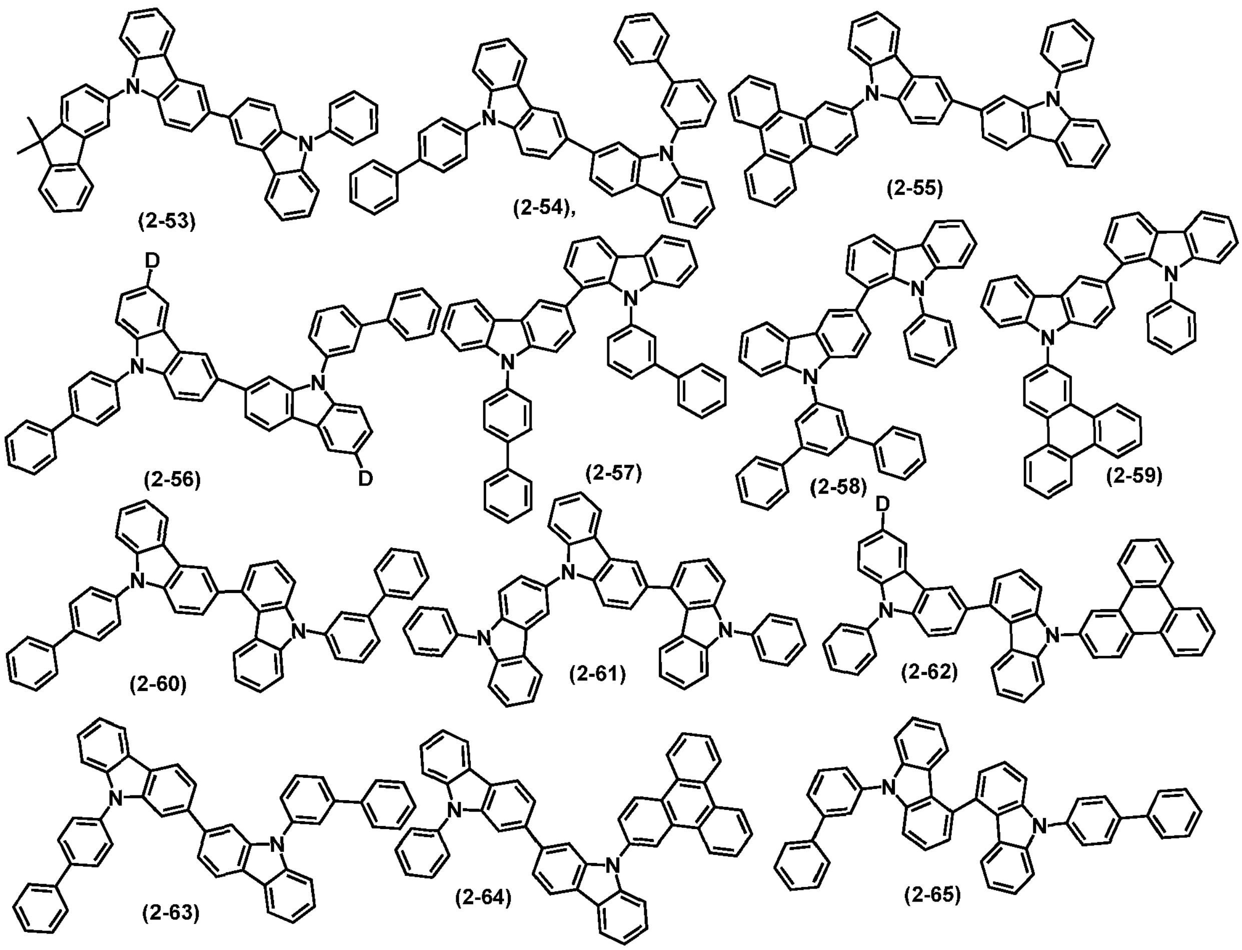

(2-53) (2-54), (2-55)

(2-56) (2-57) (2-58) (2-59)

(2-60) (2-61) (2-62)

(2-63) (2-64) (2-65)

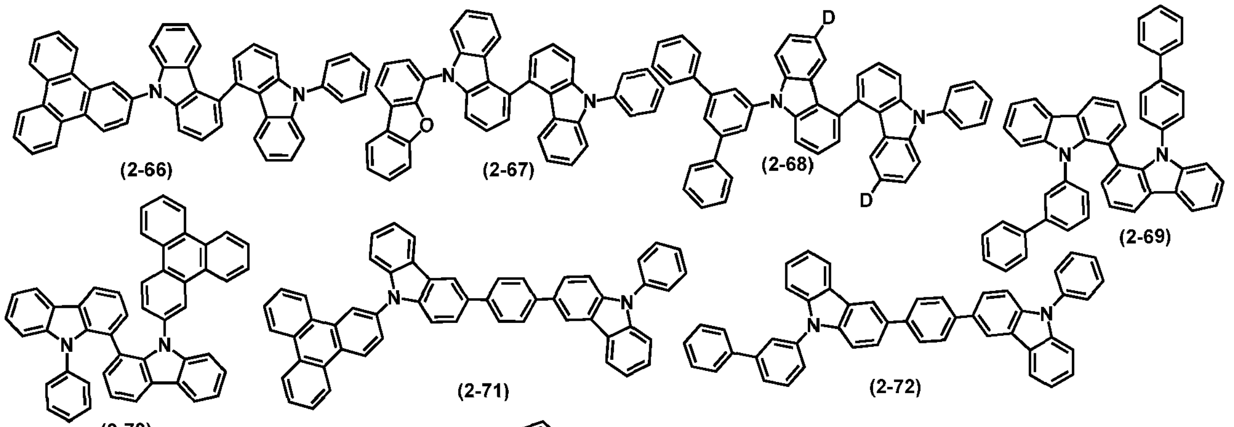

(2-66) (2-67) (2-68) (2-69)

(2-70) (2-71) (2-72)

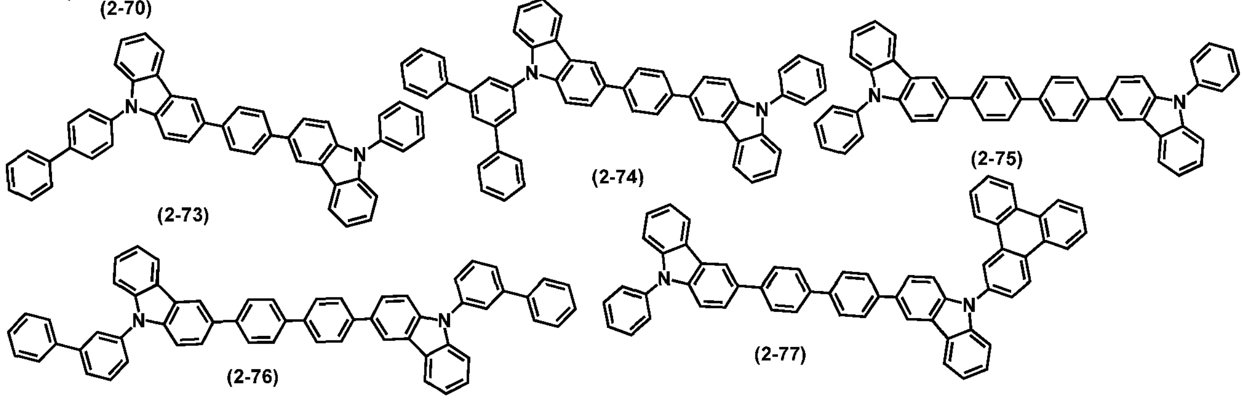

(2-73) (2-74) (2-75)

(2-76) (2-77)

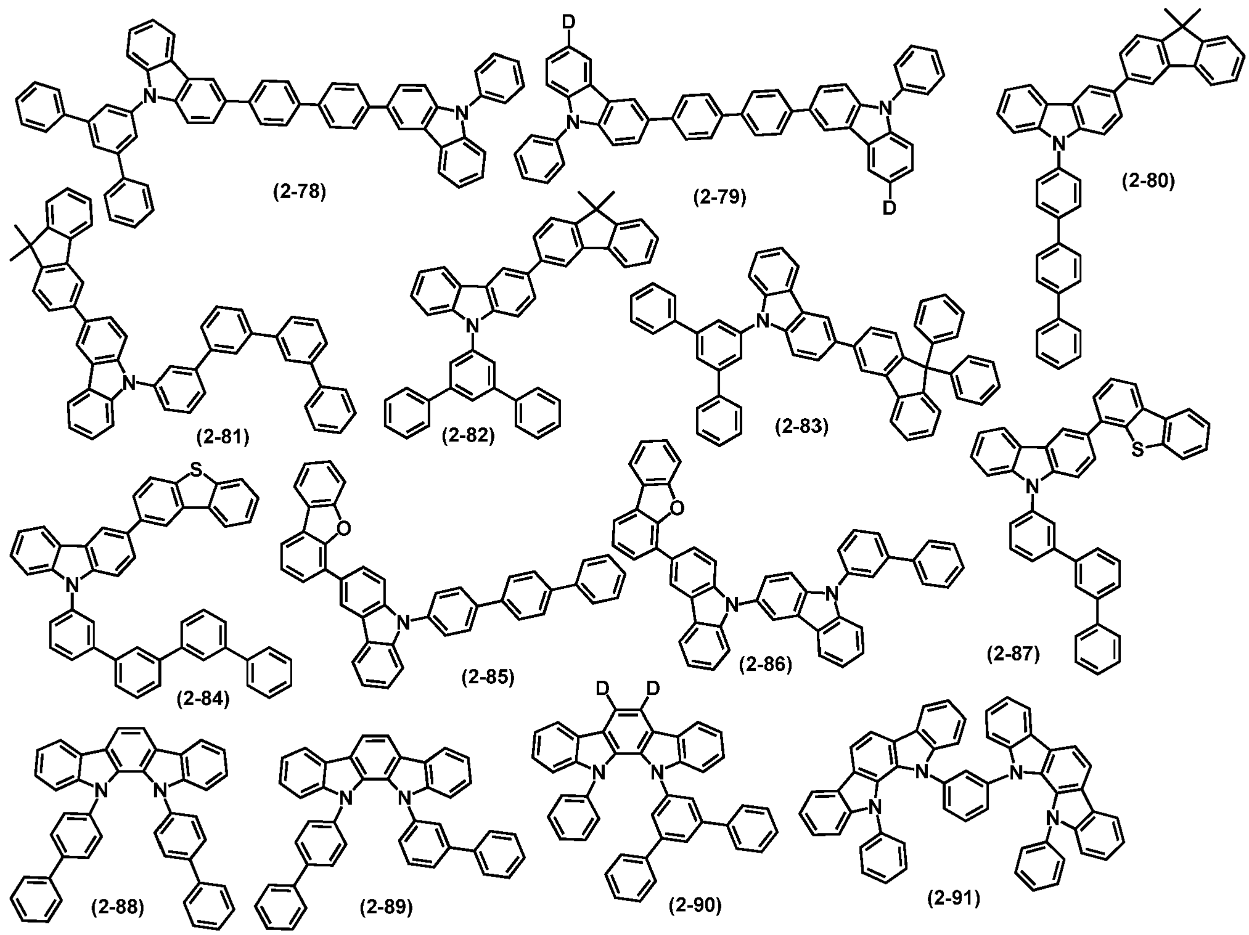

(2-78) (2-79) (2-80)

(2-81) (2-82) (2-83)

(2-84) (2-85) (2-86) (2-87)

(2-88) (2-89) (2-90) (2-91)

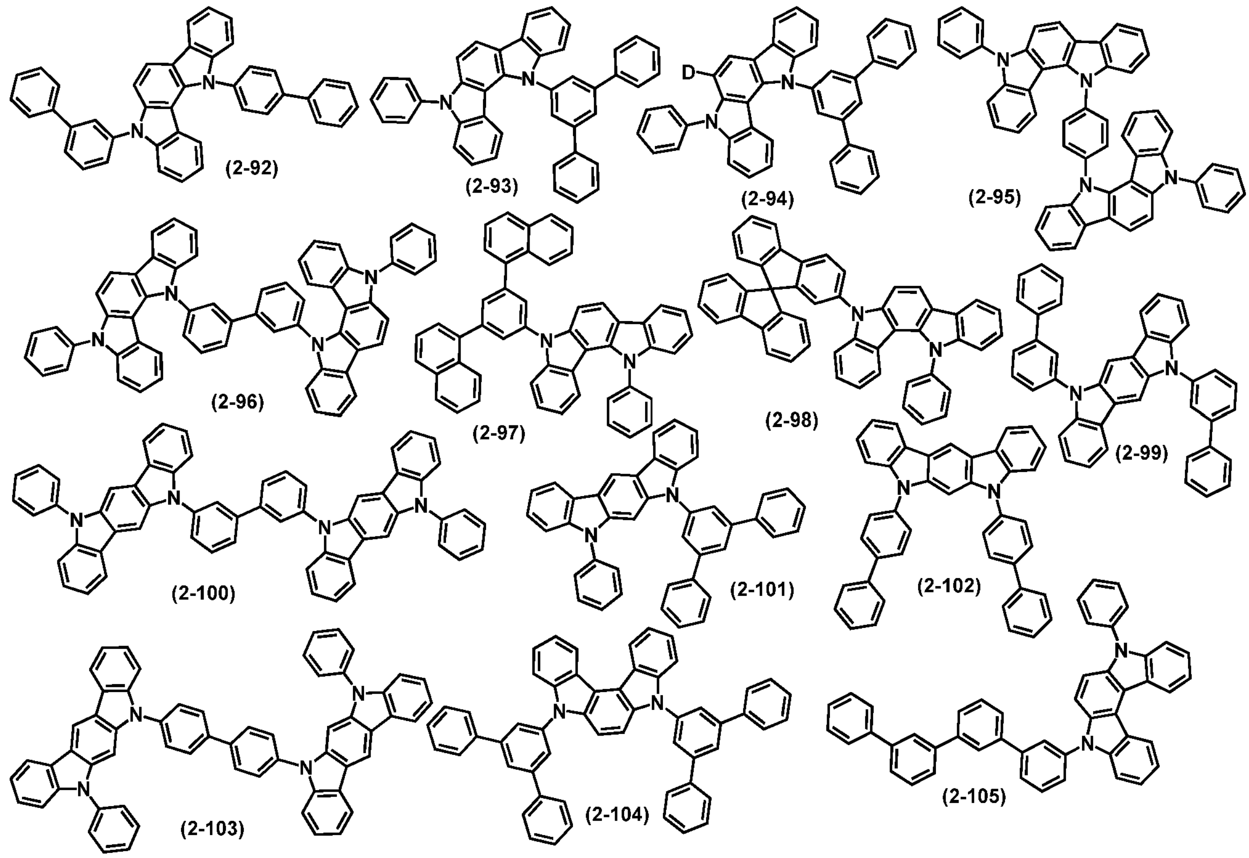

(2-92) (2-93) (2-94) (2-95)

(2-96) (2-97) (2-98) (2-99)

(2-100) (2-101) (2-102)

(2-103) (2-104) (2-105)

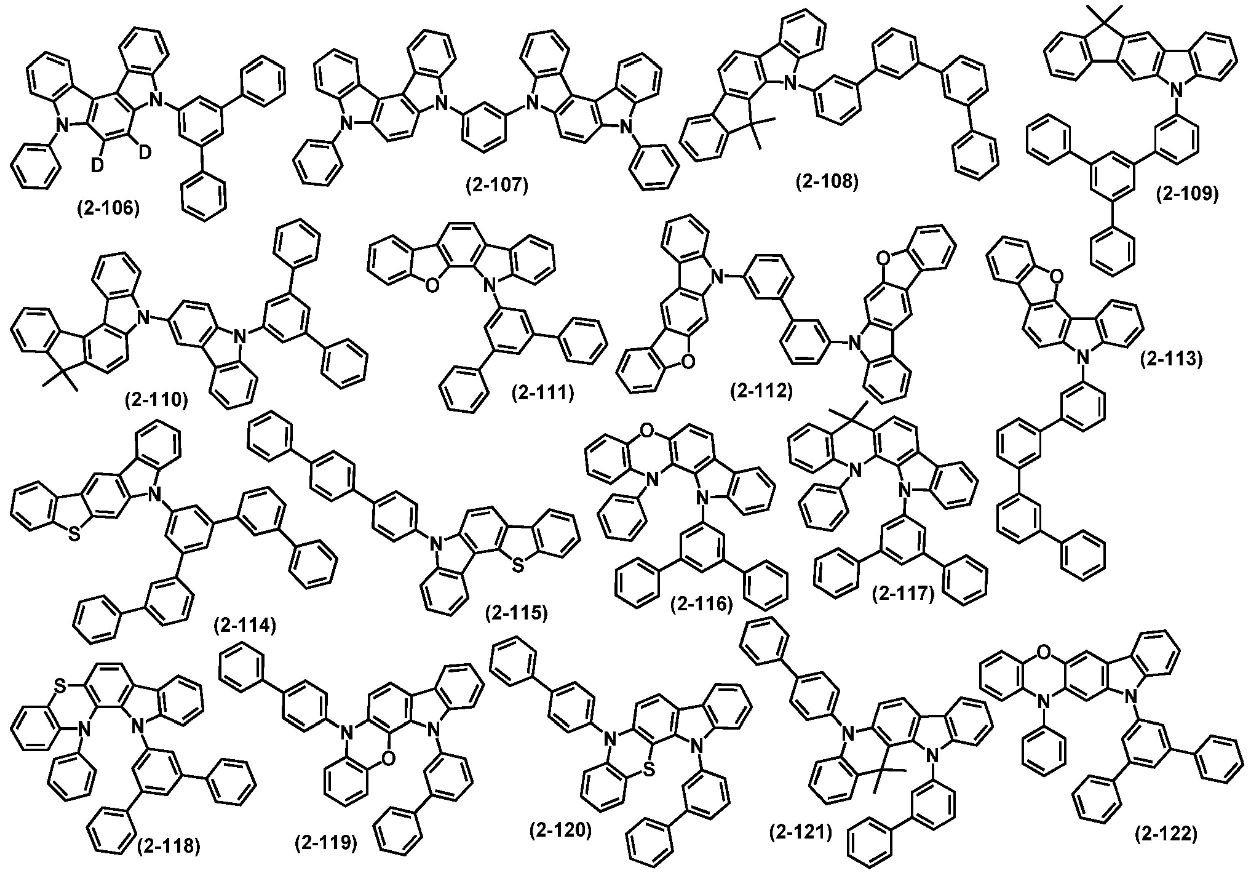

(2-106) (2-107) (2-108) (2-109)

(2-110) (2-111) (2-112) (2-113)

(2-114) (2-115) (2-116) (2-117)

(2-118) (2-119) (2-120) (2-121) (2-122)

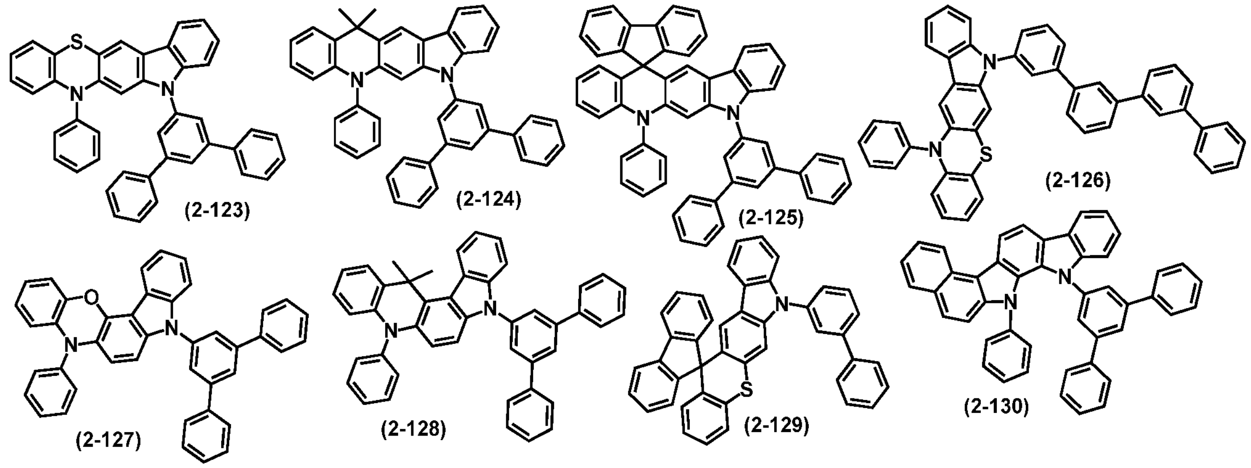

(2-123) (2-124) (2-125) (2-126)

(2-127) (2-128) (2-129) (2-130)

In an embodiment, the molar ratio of the organic compound H1 to the organic compound H2 is ranges from 2:8 to 8:2, further ranges from 3:7 to 7:3, and still further ranges from 4:6 to 6:4.

In one embodiment, the difference in molecular weight between the organic compound H1 and the organic compound H2 does not exceed 100 Dalton, further not exceed 60 Dalton, and still further not exceed 30 Dalton. Wherein, smaller difference in molecular weight between the organic compound H1 and the organic compound H2 is advantageous for the proportional stability of the two materials during the preparation of electronic devices.

In another embodiment, the difference in sublimation temperature between the organic compound H1 and the organic compound H2 does not exceed 30K, further not exceed 20K, and still further not exceed 10K. Wherein, smaller difference in sublimation temperature between the organic compound H1 and the organic compound H2 is advantageous for the proportional stability of the two materials during the preparation of evaporated electronic devices.

In one embodiment, the organic compound H1 and the organic compound H2 are small molecule materials.

In one embodiment, the organic mixture according to the present disclosure is used for evaporated OLED devices. For this purpose, the organic compound H1 and the organic compound H2 have a molecular weight of no greater than 1000 mol/kg,further no greater than 900 mol/kg, still further no greater than 850 mol/kg, still further no greater than 800 mol/kg, and even further no greater than 700 mol/kg.

In one embodiment, the organic compound H1 and the organic compound H2 have a glass transition temperature $T_g \geq 100°C$, $T_g \geq 120°C$ in one embodiment, $T_g \geq 140°C$ in a one embodiment, $T_g \geq 160°C$ in one embodiment, and $T_g \geq 180°C$ in one embodiment.

The term "small molecule" as defined herein refers to a molecule that is not a polymer, oligomer, dendrimer, or blend. In particular, there are no repeating structures in small molecules. The molecular weight of the small molecule is no greater than 3000 g/mole, further no greater than 2000 g/mole, and still further no greater than 1500 g/mole.

Polymer includes homopolymer, copolymer, and block copolymer. In addition, in the present disclosure, the polymer also includes dendrimer. The synthesis and application of dendrimers are described in Dendrimers and Dendrons, Wiley-VCH Verlag GmbH & Co. KGaA, 2002, Ed. George R. Newkome, Charles N. Moorefield, Fritz Vogtle.

Conjugated polymer is a polymer whose backbone is primarily consisted of the sp2 hybrid orbital of C atoms. Taking poly acetylene and poly (phenylene vinylene) as examples, the C atoms on the backbones of which may also be substituted by other non-C atoms, and which are still considered to be conjugated polymers when the sp2 hybridization on the backbones is interrupted by some natural defects. In addition, the conjugated polymer in the present disclosure may also comprise aryl amine, aryl phosphine and other heteroarmotics, organometallic complexes, and the like on the backbone.

The disclosure further relates to a mixture comprising the organic mixture described above and at least another organic functional material. The organic functional material is at least one selected from the group consisting of a hole (also called electron hole) injection or transport material (HIM/HTM), a hole blocking material (HBM), an electron injection or transport material (EIM/ETM), an electron blocking material (EBM), an organic host material, a singlet emitter (fluorescent emitter), a triplet emitter (phosphorescent emitter), an organic thermally activated delayed fluorescent material (TADF material) and especially a light-emitting organometallic complex. Various organic functional materials are described in detail, for example, in WO2010135519A1, US20090134784A1, and WO2011110277 A1. The organic functional material may be a small molecule material or a polymer material. In certain embodiments, the organic functional material is a light-emitting material selected from a fluorescent emitter, a phosphorescent emitter and a TADF material.

In some embodiment, the mixture comprises the organic mixture described above and a phosphorescent emitter. The organic mixture described above may be used as a host, wherein the phosphorescent emitter has a weight percentage of no greater than 30%, further no greater than 25%, and still further no greater than 20%.

In some embodiment, the mixture comprises the organic mixture described above and a fluorescent emitter. The organic mixture described above may be used as a fluorescent host material, wherein the fluorescent emitter has a weight percentage of no greater than 15%, further no greater than 10%, and still further no greater than 8%.

In another embodiment, the mixture comprises the organic mixture described above and a fluorescent host material. The organic mixture described above may be used as a fluorescent material which has a weight percentage of no greater than 15%, further no greater than 10%, and still further no greater than 8%.

In an embodiment, the mixture comprises the organic mixture described above, a phosphorescent emitter and a host material. In this embodiment, the organic mixture described above may be used as an auxiliary light-emitting material, and its weight ratio to the phosphorescent emitter is from 1:2 to 2:1. In another embodiment, the energy level of the exciplex of the organic mixture is higher than that of the phosphorescent emitter.

In another embodiment, the mixture comprises the organic mixture described above and a TADF material. The organic mixture described above may be used as a TADF host material, wherein the TADF material has a weight percentage of no greater than 15%, further no greater than 10%, and still further no greater than 8%.

The fluorescent emitting material or singlet emitter, phosphorescent emitting material or triplet emitter, and TADF material are described in more detail below (but not limited thereto).

1. Singlet Emitter

Singlet emitter tends to have a longer conjugated $\pi$-electron system. To date, there have been many examples, such as, styrylamine and derivatives thereof disclosed in JP2913116B and WO2001021729A1, and indenofluorene and derivatives thereof disclosed in WO2008/006449 and WO2007/140847.

In one embodiment, the singlet emitter can be selected from the group consisting of mono-styrylamine, di-styrylamine, tri-styrylamine, tetra-styrylamine, styrene phosphine, styrene ether and arylamine.

A mono-styrylamine is a compound comprising an unsubstituted or substituted styryl group and at least one amine, especially an aromatic amine. A di-styrylamine is a compound comprising two unsubstituted or substituted styryl groups and at least one amine, especially an aromatic amine. A tri-styrylamine is a compound comprising three unsubstituted or substituted styryl groups and at least one amine, especially an aromatic amine. A tetra-styrylamine is a compound comprising four unsubstituted or substituted styryl groups and at least one amine, especially an aromatic amine. A preferred styrene is stilbene, which may be further substituted. The definitions of the corresponding phosphines and ethers are similar to those of amines. An aryl amine or aromatic amine refers to a compound comprising three unsubstituted or substituted aromatic cyclic or heterocyclic systems directly attached to nitrogen. Especially, at least one of these aromatic or heterocyclic ring systems is preferably selected from fused ring systems and particularly has at least 14 aromatic ring atoms. Among the preferred examples are aromatic anthramine, aromatic anthradiamine, aromatic pyrene amine, aromatic pyrene diamine, aromatic chrysene amine and aromatic chrysene diamine. An aromatic anthramine refers to a compound in which a diarylamino group is directly attached to anthracene, particularly at position 9. An aromatic anthradiamine refers to a compound in which two diarylamino groups are directly attached to anthracene, particularly at positions 9, 10. Aromatic pyrene amine, aromatic pyrene diamine, aromatic chrysene amine and aromatic chrysene diamine are similarly defined, wherein the diarylarylamino group is particularlyattached to position 1 or 1 and 6 of pyrene.

The examples of singlet emitters based on vinylamine and arylamine are also some examples which may be found in the following patent documents: WO 2006/000388, WO 2006/058737, WO 2006/000389, WO 2007/065549, WO 2007/115610, US 7250532 B2, DE 102005058557 A1, CN 1583691 A, JP 08053397 A, US 6251531 B1, US 2006/210830 A EP 1 957 606 A1 and US 2008/0113101 A1.

Examples of singlet emitters based on distyrylbenzene and derivatives thereof may be found in US 5121029.

Further singlet emitters may be selected from the group consisting of: indenofluorene-amine and indenofluorene-diamine such as disclosed in WO 2006/122630, benzoindenofluorene-amine and benzoindenofluorene-diamine such as disclosed in WO 2008/006449, dibenzoindenofluoreneamine and dibenzoindenofluorene-diamine such as disclosed in WO2007/140847.

Other materials may be used as singlet emitters are polycyclic aromatic compounds, especially the derivatives of the following compounds: anthracenes such as 9,10-di(2-naphthylanthracene), naphthalene, tetraphenyl, oxyanthene, phenanthrene, perylene (such as 2,5,8,11-tetra-t-butylatedylene), indenoperylene, phenylenes (such as 4,4'- (bis (9-ethyl-3-carbazovinylene) -1,1'-biphenyl), periflanthene, decacyclene, coronene, fluorene, spirobifluorene, arylpyren (e.g., US20060222886), arylenevinylene (e.g., US5121029, US5130603), cyclopentadiene such as tetraphenylcyclopentadiene, rubrene, coumarine, rhodamine, quinacridone, pyrane such as 4(dicyanoethylene)-6-(4-dimethylaminostyryl-2-methyl) -4H-pyrane (DCM), thiapyran, bis (azinyl) imine-boron compounds (US 2007/0092753 A1), bis (azinyl) methene compounds, carbostyryl compounds, oxazone, benzoxazole, benzothiazole, benzimidazole, and diketopyrrolopyrrole. Examples of some singlet emitter materials may be found in the following patent documents: US 20070252517 A1, US 4769292, US 6020078, US 2007/0252517 A1, US 2007/0252517 A1.

Examples of suitable singlet emitters are listed below:

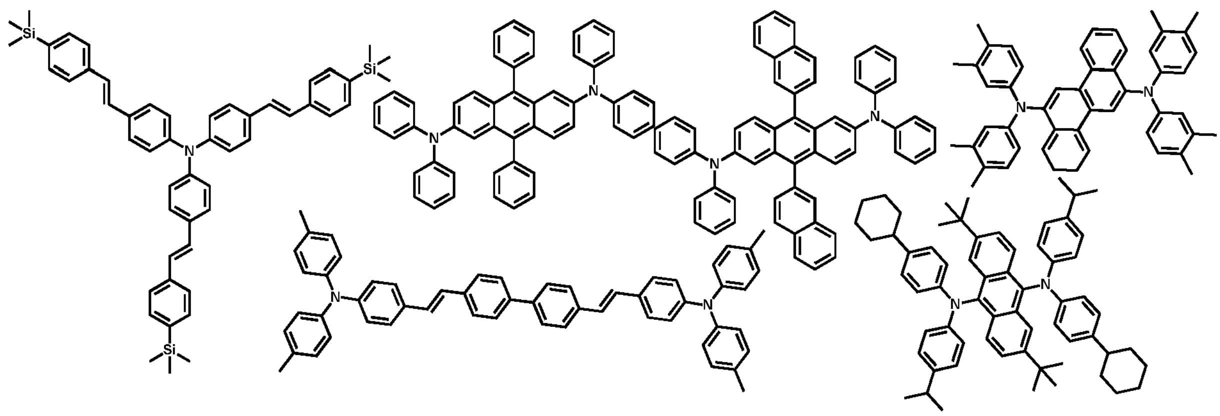

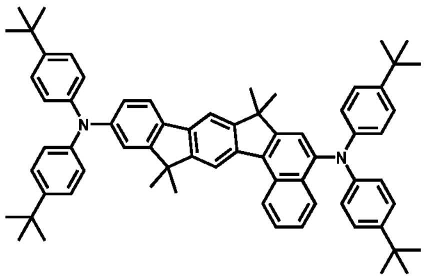

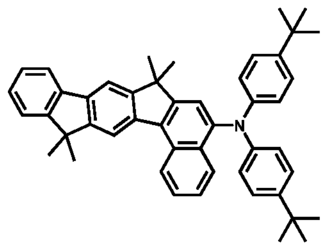

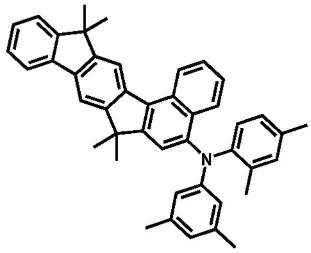

2. Thermally activated delayed fluorescent materials (TADF):

Traditional organic fluorescent materials can only emit light using 25% singlet excitonic luminescence formed by electrical excitation, and the devices have relatively low internal quantum efficiency (up to 25%). The phosphorescent material enhances the intersystem crossing due to the strong spin-orbit coupling of the heavy atom center, the singlet exciton and the triplet exciton luminescence formed by the electric excitation can be effectively utilized, so that the internal quantum efficiency of the device can reach 100%. However, the application of phosphor material in OLEDs is limited by the problems such as high cost, poor material stability and serious roll-off of the device efficiency, etc. Thermally-activated delayed fluorescent materials are the third generation of organic light-emitting materials developed after organic fluorescent materials and organic phosphorescent materials. This type of material generally has a small singlet-triplet energy level difference ($\Delta Est$), and triplet excitons can be converted to singlet excitons by anti-intersystem crossing. Thus, singlet excitons and triplet excitons formed under electric excitation can be fully utilized. The device can achieve 100% internal quantum efficiency.

The TADF material needs to have a small singlet-triplet energy level difference, typically $\Delta Est < 0.3eV$, further $\Delta Est < 0.2eV$, still further $\Delta Est < 0.1eV$, and even further $\Delta Est < 0.05eV$. In one embodiment, TADF has good fluorescence quantum efficiency. Some TADF materials can be found in the following patent documents: CN103483332(A), TW201309696(A), TW201309778(A), TW201343874(A), TW201350558(A), US20120217869(A1), WO2013133359(A1), WO2013154064(A1), Adachi, et.al. Adv. Mater., 21, 2009, 4802, Adachi, et.al. Appl. Phys. Lett., 98, 2011, 083302, Adachi, et.al. Appl. Phys. Lett., 101, 2012, 093306, Adachi, et.al. Chem. Commun., 48, 2012, 11392, Adachi, et.al. Nature Photonics, 6, 2012, 253, Adachi, et.al. Nature, 492, 2012, 234, Adachi, et.al. J. Am. Chem. Soc, 134, 2012, 14706, Adachi, et.al. Angew. Chem. Int. Ed, 51, 2012, 11311, Adachi, et.al. Chem. Commun., 48, 2012, 9580, Adachi, et.al. Chem. Commun., 48, 2013, 10385, Adachi, et.al. Adv. Mater., 25, 2013, 3319, Adachi, et.al. Adv. Mater., 25, 2013, 3707, Adachi, et.al. Chem. Mater., 25, 2013, 3038, Adachi, et.al. Chem. Mater., 25, 2013, 3766, Adachi, et.al. J. Mater. Chem. C., 1, 2013, 4599,Adachi, et.al. J. Phys. Chem. A., 117, 2013, 5607.

Some examples of suitable TADF light-emitting materials are listed in the following table:

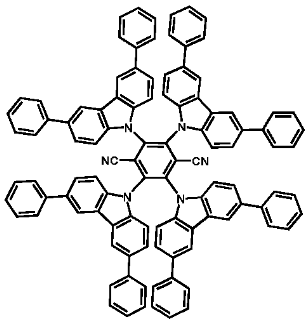
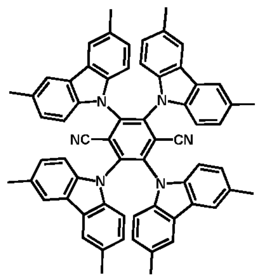
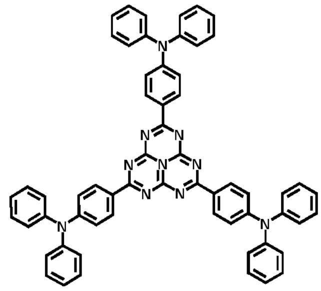
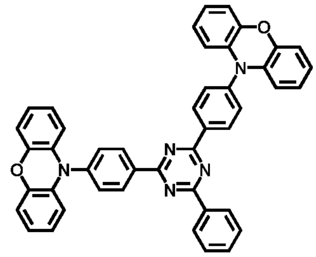
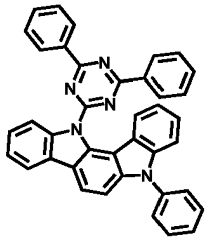
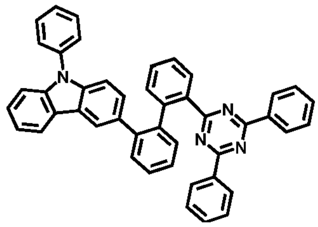
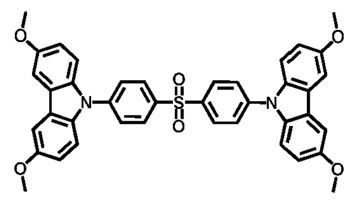
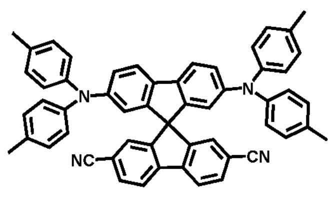
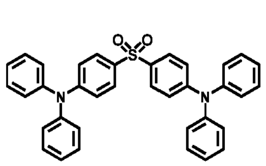
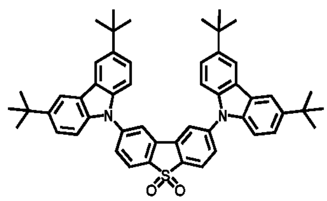
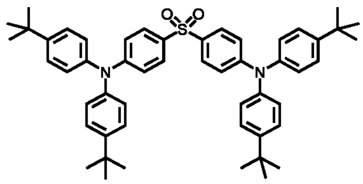
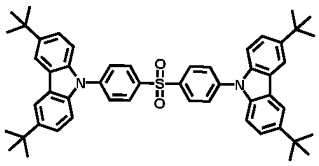
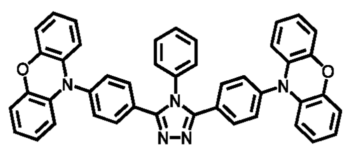
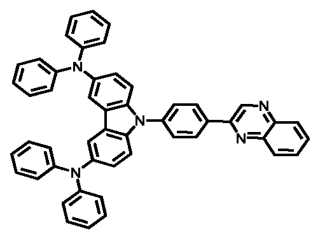
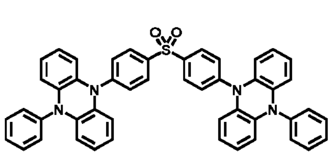
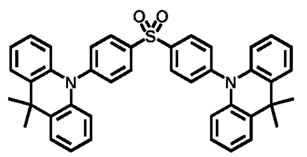
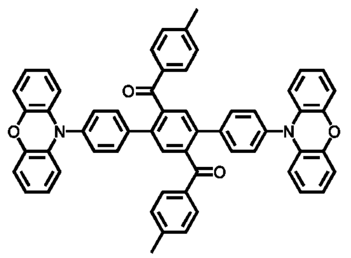
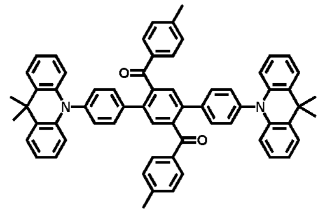
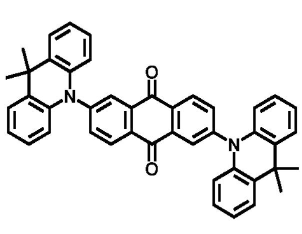
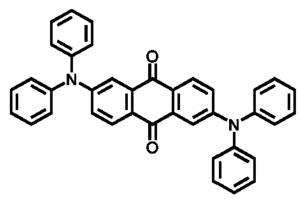
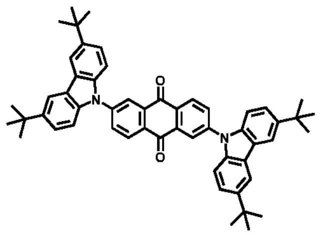

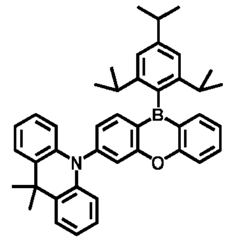
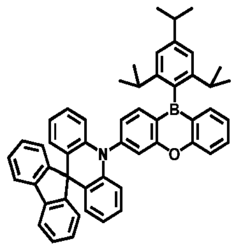
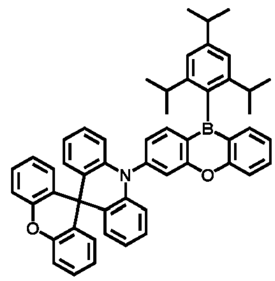
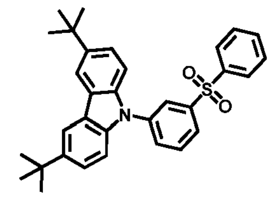

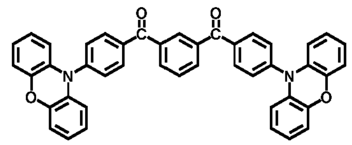
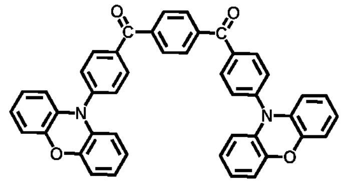
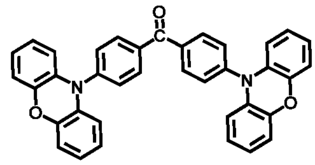

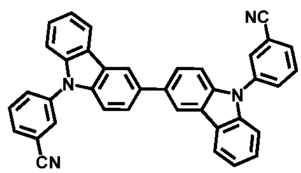
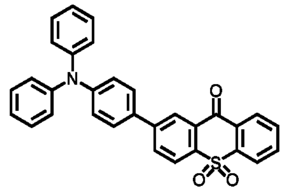
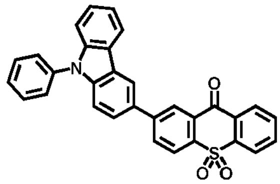
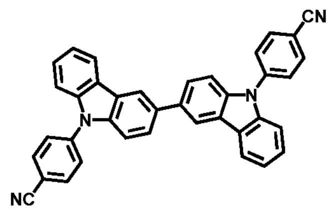

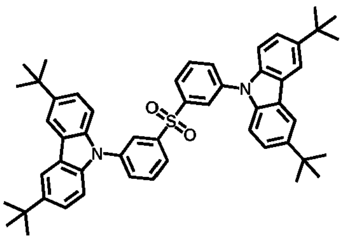

3. Triplet Emitter

Triplet emitters are also called phosphorescent emitters. In one embodiment, the triplet emitter is a metal complex with general formula $M(L)_n$, wherein M is a metal atom, and each occurrence of L may be the same or different and is an organic ligand which is bonded or coordinated to the metal atom M through one or more positions; n is an integer greater than 1, particularly 1,2,3,4,5 or 6. Optionally, these metal complexes are attached to a polymer through one or more positions, particularly through organic ligands.

In one embodiment, the metal atom M is selected from a transitional metal element or a lanthanide element or a lanthanoid element, and particularly selected from Ir, Pt, Pd, Au, Rh, Ru, Os, Sm, Eu, Gd, Tb, Dy , Re, Cu or Ag, and specially selected from Os, Ir, Ru, Rh, Re, Pd or Pt.

In one embodiment, the triplet emitter comprises chelating ligands, i.e. ligands, coordinated with the metal via at least two binding sites, and particularly, the triplet emitter comprises two or three identical or different bidentate or multidentate ligands. The chelating ligands are helpful to improve the stability of the metal complexes.

Examples of the organic ligands may be selected from the group consisting of phenylpyridine derivatives, 7,8-benzoquinoline derivatives, 2 (2 -thienyl) pyridine derivatives, 2 (1-naphthyl) pyridine derivatives, and 2 phenylquinoline derivatives. All of these organic ligands may be substituted, for example, substituted by fluoromethyl or trifluoromethyl. Auxiliary ligands may be particularly selected from acetylacetone or picric acid.

In one embodiment, the metal complexes that can be used as triplet emitters have the following form:

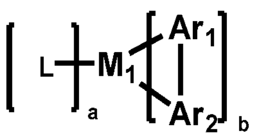

wherein, $M_1$ is a metal and selected from transitional metal elements, lanthanoid elements, or lanthanoid elements;

Each occurrence of An may be the same or different, wherein An is a cyclic group and comprises at least one donor atom (i.e., an atom having one lone pair of electrons, such as nitrogen or phosphorus) through which the cyclic group is coordinately coupled with metal; Each occurrence of $Ar_2$ may be the same or different, wherein $Ar_2$ is a cyclic group and comprises at least one carbon atom through which the cyclic group is coupled with metal; An and $Ar_2$ are covalently bonded together, and each of them may carry one or more substituents, and they may be coupled together by substituents again; Each occurrence of L may be the same or different, wherein L is an auxiliary ligand, particularly a bidentate chelating ligand,especially a monoanionic bidentate chelating ligand; b is 1, 2 or 3, further 2 or 3, specially 3; a is 0, 1 or 2, further 0 or 1, specially 0.

Some examples of triplet emitter materials and applications thereof can be found in the following patent documents and references: WO 200070655, WO 200141512, WO 200202714, WO 200215645, EP 1191613, EP 1191612, EP 1191614, WO 2005033244, WO 2005019373, US 2005/0258742, WO 2009146770, WO 2010015307, WO 2010031485, WO 2010054731, WO 2010054728, WO 2010086089, WO 2010099852, WO 2010102709, US 20070087219 A1, US 20090061681 A1, US 20010053462 A1, Baldo, Thompson et al. Nature 403, (2000), 750-753, US 20090061681 A1, US 20090061681 A1, Adachi et al. Appl. Phys. Lett. 78 (2001), 1622-1624, J. Kido et al. Appl. Phys. Lett. 65 (1994), 2124, Kido et al. Chem. Lett. 657, 1990, US 2007/0252517 A1, Johnson et al., JACS 105, 1983, 1795, Wrighton, JACS 96, 1974, 998, Ma et al., Synth. Metals 94, 1998, 245, US 6824895, US 7029766, US 6835469, US 6830828, US 20010053462 A1, WO 2007095118 A1, US 2012004407A1, WO 2012007088A1, WO2012007087A1, WO 2012007086A1, US 2008027220A1, WO 2011157339A1, CN 102282150A, WO 2009118087A1.

Some suitable examples of triplet emitters are listed in the following table:

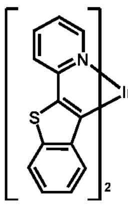
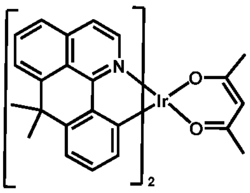
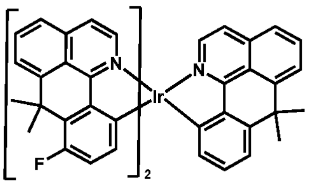
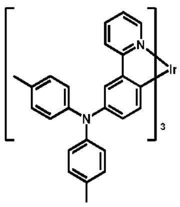
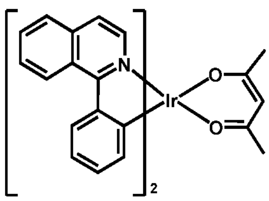

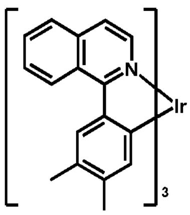
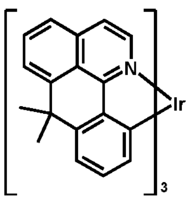
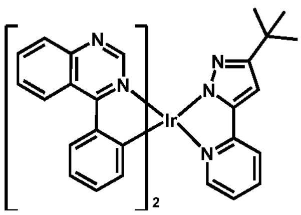
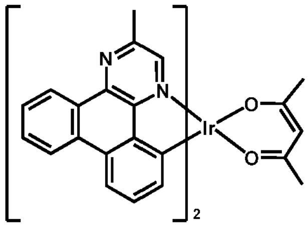
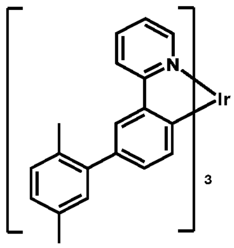

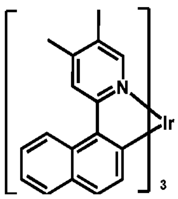
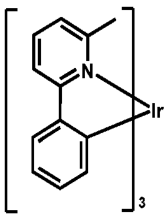
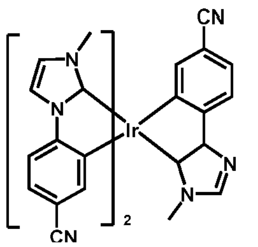
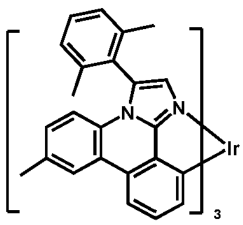
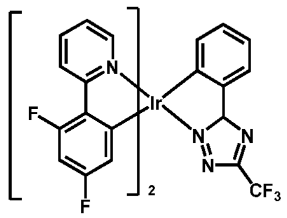

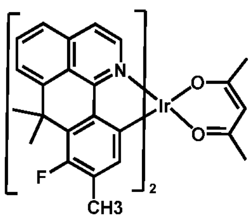
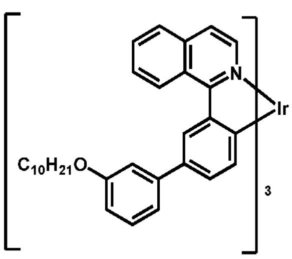
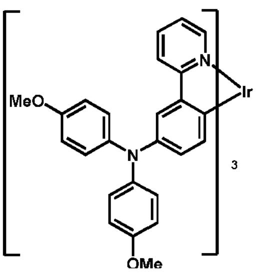
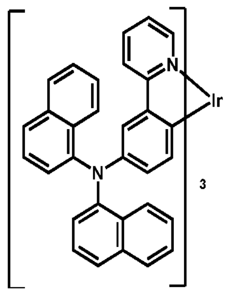

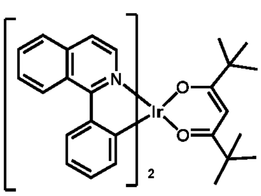 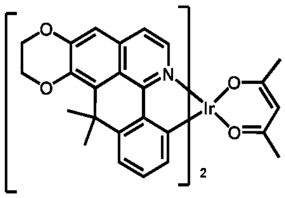 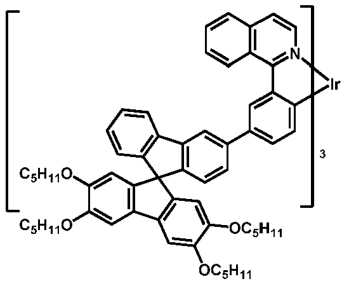 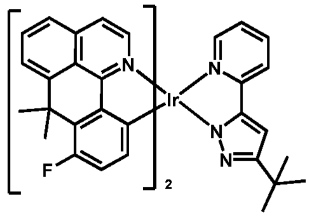

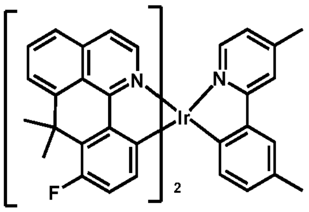 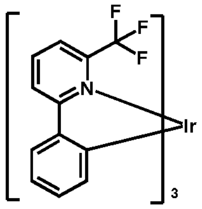 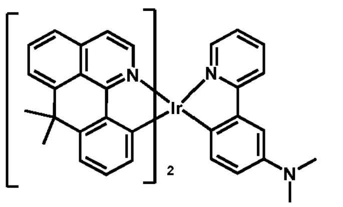 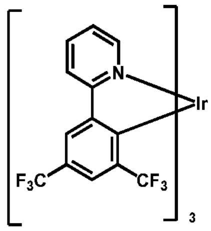

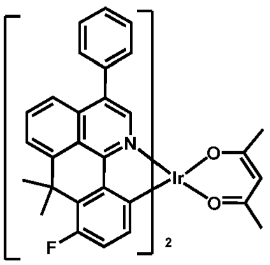 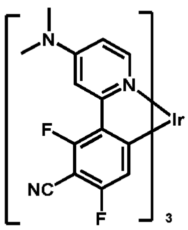 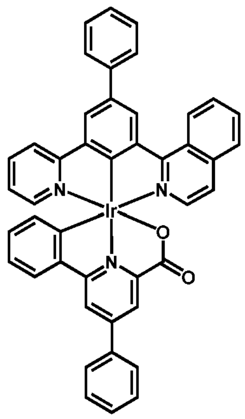 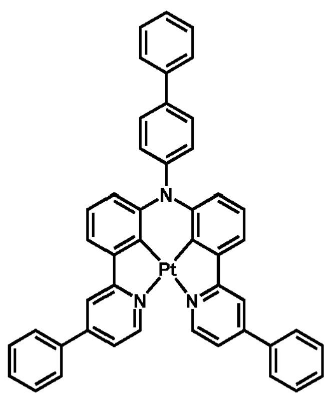 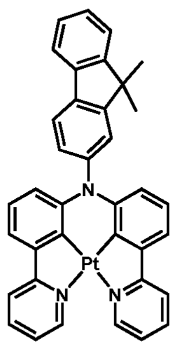

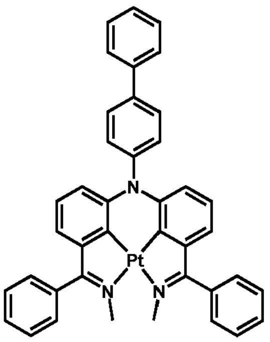 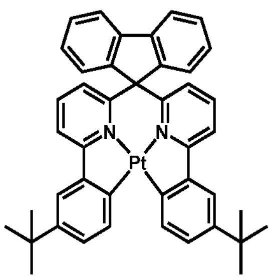 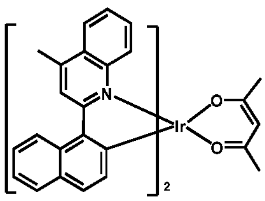 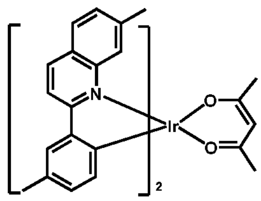

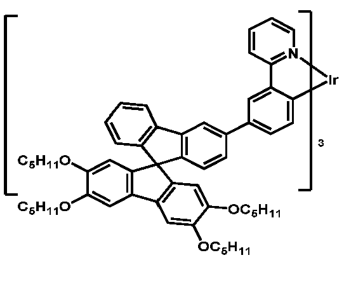 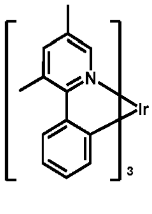 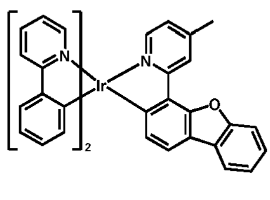 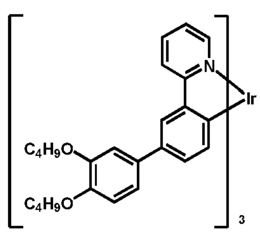

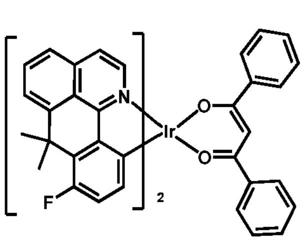 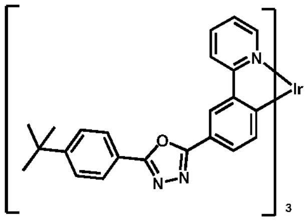 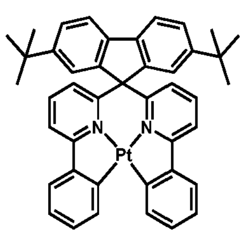 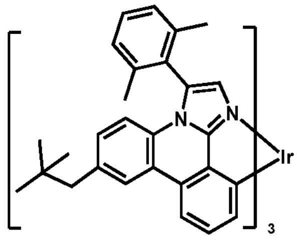

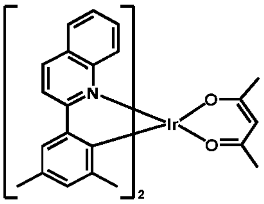

Another purpose of the present disclosure is to provide material solutions for printing OLED.

For this purpose, at least one of the organic compound H1 and the organic compound H2 has a molecular weight no less than 700mol/kg, further no less than 900mol/kg, still further no less than 900mol/kg, still further no less than 1000mol/kg, and even further no less than 1100mol/kg.

In certain embodiments, the solubility of the organic mixture in toluene at 25 °C is no less than 10mg/ml, further no less than 15mg/ml, and still further no less than 20mg/ml.

In addition, the present disclosure further relates to a formulation or an ink comprising the organic mixture as described above, and at least one organic solvent.

The viscosity and surface tension of ink are important parameters when the ink is used in the printing process. The suitable surface tension parameters of ink are suitable for a particular substrate and a particular printing method.

In one embodiment, the surface tension of the ink according to the present disclosure at working temperature or at 25°C is in the range of about 19 dyne/cm to 50 dyne/cm, further in the range of 22 dyne/cm to 35 dyne/cm, and still further in the range of 25 dyne/cm to 33 dyne/cm.

In another embodiment, the viscosity of the ink according to the present disclosure at the working temperature or at 25° C is in the range of about 1 cps to 100 cps, further in the range of 1 cps to 50 cps, still further in the range of 1.5 cps to 20 cps, and even further in the range of 4.0 cps to 20 cps. The formulation so formulated will be suitable for inkjet printing.

The viscosity can be adjusted by different methods, such as by proper solvent selection and the concentration of functional materials in the ink. The ink according to the present disclosure comprising the metal organic compound or polymer can facilitate the adjustment of the printing ink in an appropriate range according to the printing method used. In general, the weight ratio of the functional material contained in the formulation according to the disclosure is in the range of 0.3 wt% to 30 wt%, further in the range of 0.5 wt% to 20 wt%, still further in the range of 0.5 wt% to 15 wt%, still further in the range of 0.5 wt% to 10 wt%, and even further in the range of 1 wt% to 5 wt%.

In some embodiments, according to the ink of the present disclosure, the at least one organic solvent is selected from aromatic or heteroaromatic based solvents, in particular from aromatic solvents or aromatic ketone solvents or aromatic ether solvents substituted by aliphatic chain/ring.

Examples suitable for solvents of the present disclosure include, but not limited to, aromatic or heteroaromatic based solvents: p-diisopropylbenzene, pentylbenzene, tetrahydronaphthalene, cyclohexyl benzene, chloronaphthalene, 1,4-dimethylnaphthalene, 3-isopropylbiphenyl, p-cymene, dipentylbenzene, tripentylbenzene, pentyltoluene, o-xylene, m-xylene, p-xylene, o-diethylbenzene, m-diethylbenzene, p-diethylbenzene, 1,2,3,4-tetramethylbenzene, 1,2,3,5-tetramethylbenzene, 1,2,4,5-tetramethylbenzene, butylbenzene, dodecylbenzene, dihexylbenzene, dibutylbenzene, p-diisopropylbenzene, 1-methoxynaphthalene, cyclohexylbenzene, dimethylnaphthalene, 3-isopropylbiphenyl, p-cymene, 1-methylnaphthalene, 1,2,4-trichlorobenzene, 1,3-dipropoxybenzene, 4,4-difluorodiphenylmethane, 1,2-dimethoxy-4-(1-propenyl)benzene, diphenylmethane, 2-phenylpyridine, 3-phenylpyridine, N-methyldiphenylamine 4-isopropylbiphenyl, $\alpha,\alpha$--dichlorodiphenylmethane, 4-(3-phenylpropyl)pyridine, benzylbenzoate, 1,1-di(3,4-dimethylphenyl)ethane, 2-isopropylnaphthalene, dibenzylether, and the like; solvents based on ketones: 1-tetralone, 2-tetralone, 2-(phenylepoxy)tetralone, 6-(methoxyl)tetralone, acetophenone, phenylacetone, benzophenone , and derivatives thereof, such as 4-methylacetophenone, 3-methylacetophenone, 2-methylacetophenone, 4-methylphenylacetone, 3-methylphenylacetone, 2-methylphenylacetone, isophorone, 2,6,8-trimethyl-4-nonanone, fenchone, 2-nonanone, 3-nonanone, 5-nonanone, 2-demayone, 2,5-hexanedione, phorone, di-n-amyl ketone; aromatic ether solvents: 3-phenoxytoluene, butoxybenzene, benzylbutylbenzene, p-anisaldehyde dimethyl acetal, tetrahydro-2-phenoxy-2H-pyran, 1,2-dimethoxy 4-(1-propenyl)benzene, 1,4-benzodioxane, 1,3-dipropylbenzene, 2,5-dimethoxytoluene, 4-ethylphenetole, 1,2,4-trimethoxybenzene, 4-(1-propenyl)-1,2-dimethoxybenzene, 1,3-dimethoxybenzene, glycidyl phenyl ether, dibenzyl ether, 4-tert-butylanisole, trans-p-propenylanisole, 1,2-dimethoxybenzene, 1-methoxynaphthalene, diphenyl ether, 2-phenoxymethyl ether, 2-phenoxytetrahydrofuran, ethyl-2-naphthyl ether, pentyl ether, hexyl ether, dioctyl ether, ethylene glycol dibutyl ether, diethylene glycol diethyl ether, diethylene glycol butyl methyl ether, diethylene glycol dibutyl ether, triethylene glycol dimethyl ether, triethylene glycol ethyl methyl ether, triethylene glycol butyl methyl ether, tripropylene glycol dimethyl ether, tetraethylene glycol dimethyl ether; and ester solvents: alkyl octoate, alkyl sebacate, alkyl stearate, alkyl benzoate, alkyl phenylacetate, alkyl cinnamate, alkyl oxalate, alkyl maleate, alkyl lactone, alkyl oleate, and the like.

Further, according to the ink of the present disclosure, the at least one solvent may be selected from the group consisting of aliphatic ketones, such as 2-nonanone, 3-nonanone, 5-nonanone, 2-decanone, 2,5-hexanedione, 2,6,8-trimethyl-4-demayone, phorone, di-n-pentyl ketone, and the like; or aliphatic ethers, such as amyl ether, hexyl ether, dioctyl ether, ethylene glycol dibutyl ether, diethylene glycol diethyl ether, diethylene glycol butyl methyl ether, diethylene glycol dibutyl ether, triethylene glycol dimethyl ether, triethyl ether alcohol ethyl methyl ether, triethylene glycol butyl methyl ether, tripropylene glycol dimethyl ether, tetraethylene glycol dimethyl ether, and the like.

In other embodiments, the printing ink further comprises another organic solvent. Examples of another organic solvent comprise, but not limited to, methanol, ethanol, 2-methoxyethanol, dichloromethane, trichloromethane, chlorobenzene, o-dichlorobenzene, tetrahydrofuran, anisole, morpholine, toluene, o-xylene, m-xylene, p-xylene, 1,4-dioxane, acetone, methyl ethyl ketone, 1,2-dichloroethane, 3-phenoxy toluene, 1,1,1-trichloroethane, 1,1,2,2-tetrachloroethane, ethyl acetate, butyl acetate, dimethylformamide, dimethylacetamide, dimethyl sulfoxide, tetrahydronaphthalene, decalin, indene, and/or mixtures thereof.

In an embodiment, the formulation according to the disclosure is a solution.

In another embodiment, the formulation according to the disclosure is a suspension.

The formulation in embodiments of the present disclosure may comprise 0.01-20 wt%, further 0.1-15 wt%, still further 0.2-10 wt%, and even further 0.25-5 wt% of the organic mixture according to the disclosure.

The disclosure also relates to the use of the formulation as a coating or printing ink in the preparation of organic electronic devices, specially by the preparation method of printing or coating.

The appropriate printing technology or coating technology includes, but is not limited to inkjet printing, nozzle printing, typography, screen printing, dip coating, spin coating, blade coating, roller printing, twist roller printing, lithography, flexography, rotary printing, spray coating, brush coating or transfer printing, slot die coating, and the like. Particularly are gravure printing, nozzle printing and inkjet printing. The solution or the suspension liquid may further includes one or more components, such as a surfactant compound, a lubricant, a wetting agent, a dispersant, a hydrophobic agent, a binder, to adjust the viscosity and the film forming property and to improve the adhesion property. The detailed information relevant to the printing technology and requirements of the printing technology to the solution, such as solvent, concentration, and viscosity, may be referred to Handbook of Print Media: Technologies and Production Methods, Helmut Kipphan, ISBN 3-540-67326-1.

Based on the above organic mixture, the present disclosure also provides an application of the above organic mixture in organic electronic devices. The organic electronic devices may be selected from, but not limited to, an organic light-emitting diode (OLED), an organic photovoltaic cell (OPV), an organic light-emitting electrochemical cell (OLEEC), an organic field effect transistor (OFET), an organic light-emitting field effect transistor, an organic laser, an organic spintronic device, organic sensor, and an organic plasmon emitting diode, and the like, specially OLED. In embodiments of the present disclosure, the organic compound is used in the light-emitting layer of the OLED device.

The disclosure further relates to an organic electronic device comprising at least one organic mixture as described above. Generally, the organic electronic device includes at least one cathode, one anode, and one functional layer located between the cathode and the anode, wherein the functional layer comprises at least one organic mixture as described above. The organic electronic devices may be selected from, but not limited to, an organic light-emitting diode (OLED), an organic photovoltaic cell (OPV), an organic light-emitting electrochemical cell (OLEEC), an organic field effect transistor (OFET), an organic light-emitting field effect transistor, an organic laser, an organic spintronic device, an organic sensor, and an organic plasmon emitting diode, etc., specially an organic electroluminescent device such as an OLED, an OLEEC and an organic light-emitting field effect transistor.

In certain particularly embodiments, the light-emitting layer of the organic electroluminescent device comprises the organic mixture, or comprises the organic mixture and a phosphorescent emitter, or comprises the organic mixture and a host material, or comprise the organic mixture, a phosphorescent emitter and a host material.

In the above light-emitting device, particularly the OLED, a substrate, an anode, at least one light-emitting layer and a cathode are included.

The substrate can be opaque or transparent. A transparent substrate can be used to make a transparent light-emitting device. See, e.g., Bulovic et al. Nature 1996, 380, p29 and Gu et al. ppl. Phys. Lett. 1996, 68, p2606. The substrate can be rigid or elastic. The substrate may be plastic, metal, a semiconductor wafer or glass. Particularly the substrate has a smooth surface. The substrate without any surface defects is the particular ideal selection. In one

embodiment, the substrate is flexible and may be selected from a polymer thin film or a plastic which have the glass transition temperature $T_g$ is 150°C or larger, further larger than 200°C, still further larger than 250°C, still further larger than 300°C. Suitable examples of the flexible substrate are polyethylene terephthalate (PET) and polyethylene 2,6-naphthalate (PEN).

The anode may include a conductive metal, a metallic oxide, or a conductive polymer. The anode can inject holes easily into the hole injection layer (HIL), the hole transport layer (HTL), or the light-emitting layer. In an embodiment, the absolute value of the difference between the work function of the anode and the HOMO energy level or the valence band energy level of the emitter in the light-emitting layer or of the p-type semiconductor material as the HIL or HTL or the electron blocking layer (EBL) is smaller than 0.5 eV, further smaller than 0.3 eV, still further smaller than 0.2 eV. Examples of the anode material include, but are not limited to Al, Cu, Au, Ag, Mg, Fe, Co, Ni, Mn, Pd, Pt, ITO, aluminum-doped zinc oxide (AZO), and the like. Other suitable anode materials are known and may be easily selected by one of ordinary skilled in the art. The anode material may be deposited by any suitable technologies, such as the suitable physical vapor deposition method which includes a radio frequency magnetron sputtering, a vacuum thermal evaporation, an electron beam, and the like. In some embodiments, the anode is patterned and structured. A patterned ITO conductive substrate may be purchased from market to prepare the device according to the present disclosure.

The cathode may include a conductive metal or metal oxide. The cathode can inject electrons easily into the electron injection layer (EIL) or the electron transport layer (ETL), or directly injected into the light-emitting layer. In an embodiment, the absolute value of the difference between the work function of the cathode and the LUMO energy level or the valence band energy level of the emitter in the light-emitting layer or of the n type semiconductor material as the electron injection layer (EIL) or the electron transport layer (ETL) or the hole blocking layer (HBL) is smaller than 0.5 eV, further smaller than 0.3 eV, still further smaller than 0.2 eV. In principle, all materials capable of using as the cathode of the OLED may be used as the cathode material of the device of the present disclosure. Examples of the cathode material include, but are not limited to, Al, Au, Ag, Ca, Ba, Mg, LiF/Al, MgAg alloy, BaF2/Al, Cu, Fe, Co, Ni, Mn, Pd, Pt, ITO, and the like. The cathode material may be deposited by any suitable technologies, such as the suitable physical vapor deposition method which includes a radio frequency magnetron sputtering, a vacuum thermal evaporation, an electron beam, and the like.

OLED can also comprise other functional layers such as hole injection layer (HIL), hole transport layer (HTL), electron blocking layer (EBL), electron injection layer (EIL), electron transport layer (ETL), and hole blocking layer (HBL). Materials which are suitable for use in these functional layers are described in detail above and in WO2010135519A1, US20090134784A1 and WO2011110277A1.

In one embodiment, the light-emitting layer of light-emitting device according to the present disclosure comprises the mixture according to the present disclosure. The light-emitting layer can be prepared by the following two methods:

(1) The mixture comprising H1 and H2 is prepared by evaporation as one source. The mixture may be prepared using the formulation according to the present disclosure by printing, or the mixture may be prepared by vacuum evaporation as one source.

Or (2) H1 and H2 are prepared by evaporation as two separate sources.

The emission wavelength of the light-emitting device according to the present disclosure is between 300 and 1000 nm, further between 350 and 900 nm, and still further between 400 and 800 nm.

The present disclosure also relates to the application of the electroluminescent device according to the present disclosure in various electronic equipments, comprising but not limited to display equipment, lighting equipment, light source, and sensor, and the like.

The present disclosure will be described below with reference to the preferred embodiments, but the present disclosure is not limited to the following embodiments. It should be understood that the appended claims summarized the scope of the present disclosure.

## DETAILED EXAMPLES

1. The synthesis method for the compound of the present disclosure is exemplified below, but the present disclosure is not limited to the following examples.

(1) Synthesis of Compound (1-36):

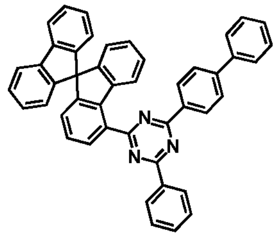

(1-36)

1)

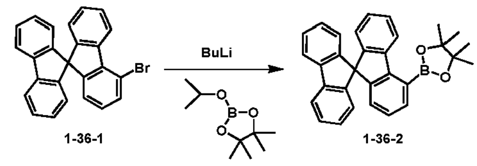


Compound (1-36-1) (31.6 g, 80 mmol) and 200 mL of anhydrous tetrahydrofuran were added to a 500 mL three-necked flask under nitrogen atmosphere, cooled to -78°C, and 85 mmol of n-butyllithium was slowly added dropwise, the mixture was reacted for 2 hours, then 90 mmol of isopropoxyboronic acid pinacol ester was added one time to allow the reaction temperature to rise to room temperature naturally. The reaction was further performed for 12 hours and then quenched by the addition of pure water. The reaction solution was rotary evaporated to remove most of the solvent, and then extracted with dichloromethane and washed with water for 3 times. The organic phase was collected, spin dried, and then recrystallized, with a yield of 90%.

2)

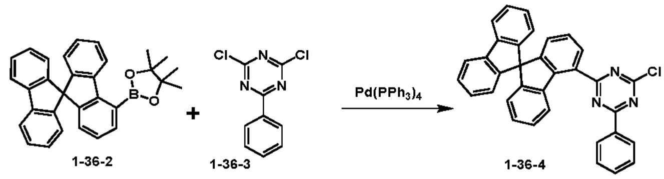


Compound 1-36-2 (26.5 g, 60 mmol) and Compound 1-36-3 (13.6 g, 60 mmol), tetrakis(triphenylphosphine)palladium (3.45 g, 3 mmol), tetrabutylammonium bromide (2.6 g, 8 mmol), sodium hydroxide (3.2 g, 80 mmol), water (20 mL) and toluene (150 mL) were added to a 250 mL three-necked flask under nitrogen atmosphere, and the mixture was heated to 80°C and reacted under stirring for 12 hours, and then the reaction was ended. The reaction solution was rotary evaporated to remove most of the solvent, and then dissolved with dichloromethane and washed with water for 3 times. The organic solution was collected, mixed with silica gel, and then purified by column chromatography, with a yield of 70%.

3)

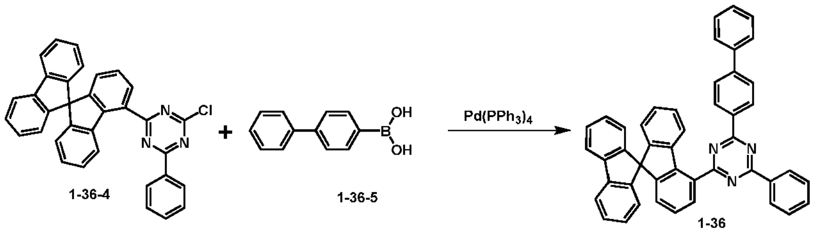


Compound 1-36-4 (10.1 g, 20 mmol) and Compound 1-36-5 (4g, 20 mmol), tetrakis(triphenylphosphine)palladium (1.15 g, 1 mmol), tetrabutylammonium bromide (1.3 g, 4 mmol), sodium hydroxide (1.6 g, 40 mmol), water (10 mL) and toluene (60 mL) were added to a 150 mL three-necked flask under nitrogen atmosphere, and the mixture was heated to 80° C and reacted under stirring for 12 hours, and then the reaction was ended. The reaction solution was rotary evaporated to remove most of the solvent, and then dissolved with dichloromethane and washed with water for 3 times.

The organic solution was collected, mixed with silica gel, and then purified by column chromatography, with a yield of 80%.

(2) Synthesis of Compound (1-37):

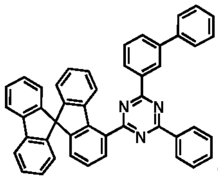

(1-37)

1)

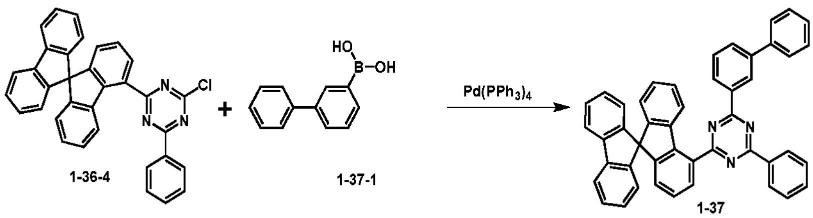


Compound 1-36-4 (10.1 g, 20 mmol) and Compound 1-37-1 (5.6 g, 20 mmol), tetrakis(triphenylphosphine)palladium (1.15 g, 1 mmol), tetrabutylammonium bromide (1.3 g, 4 mmol), sodium hydroxide (1.6 g, 40 mmol), water (10 mL) and toluene (60 mL) were added to a 150 mL three-necked flask under nitrogen atmosphere, and the mixture was heated to 80°C and reacted under stirring for 12 hours, and then the reaction was ended. The reaction solution was rotary evaporated to remove most of the solvent, and then dissolved with dichloromethane and washed with water for 3 times. The organic solution was collected, mixed with silica gel, and then purified by column chromatography, with a yield of 80%.

(3) Synthesis of Compound (1-44):

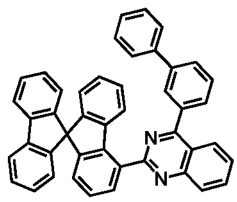

(1-44)

1)

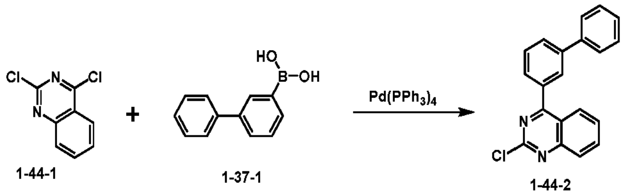


Compound 1-44-1 (8g, 40 mmol) and Compound 1-37-1 (11.2g, 40 mmol), tetrakis(triphenylphosphine)palladium (2.3 g, 2 mmol), tetrabutylammonium bromide (2.6 g, 8mmol), sodium hydroxide (3.2 g, 80 mmol), water (20 mL) and toluene (120 mL) were added to a 250 mL three-necked flask under nitrogen atmosphere, and the solution was heated to 80°C and reacted under stirring for 12 hours, and then the reaction was ended. The reaction solution was rotary evaporated to remove most of the solvent, and then dissolved with dichloromethane and washed with water for

3 times. The organic solution was collected, mixed with silica gel, and then purified by column chromatography, with a yield of 80%.

2)

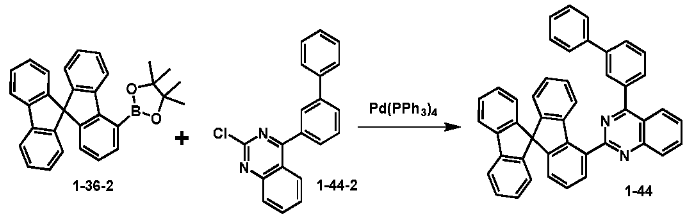


Compound 1-36-2 (8.8g, 20 mmol) and Compound 1-44-2 (6.3g, 20 mmol), tetrakis(triphenylphosphine)palladium (1.15 g, 1 mmol), tetrabutylammonium bromide (1.3 g, 4 mmol), sodium hydroxide (1.6 g, 40 mmol), water (10 mL) and toluene (60 mL) were added to a 150 mL three-necked flask under nitrogen atmosphere, and the solution was heated to 110°C and reacted under stirring for 12 hours, and then the reaction was ended. The reaction solution was rotary evaporated to remove most of the solvent, and then dissolved with dichloromethane and washed with water for 3 times. The organic solution was collected, mixed with silica gel, and then purified by column chromatography, with a yield of 75%.

(4) Reference Example Ref-1:

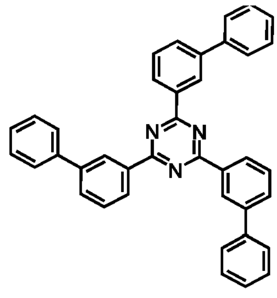
(Ref-1)

1)

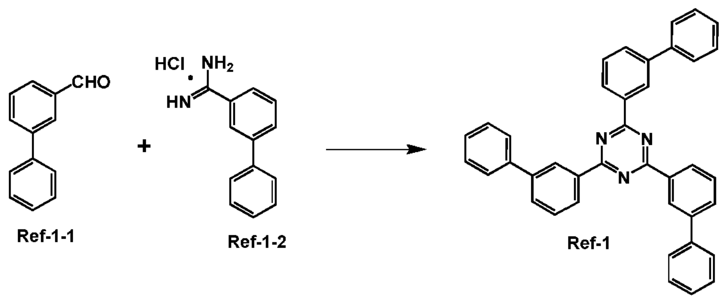

Compound Ref-1-1 (9.1 g, 50 mmol), Compound Ref-1-2 (23.2 g, 100 mmol), tetrakis(triphenylphosphine)palladium (2.6 g, 2.5 mmol), cesium carbonate (32.6 g, 100 mmol), water (100 mL) and 1,4-dioxane (200 mL) were added to a 500 mL two-necked flask under nitrogen atmosphere, and the solution was refluxed with heating and reacted under stirring for 12 hours, and then the reaction was ended.The aqueous layer was separated, the reaction solution was filtered with suction, and the filter residue was recrystallized, with a yield 70%.

(5) Synthesis of Compound (2-46):

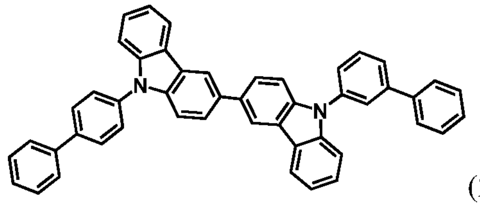

(2-46)

1)

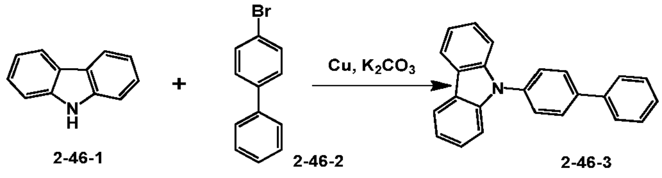


2-46-1 2-46-2 2-46-3

Compound (2-46-1) (16.7 g, 100 mmol) and Compound (2-46-2) (24.5 g, 105 mmol), copper powder (0.65 g, 10 mmol), potassium carbonate (13.8 g, 100 mmol) and 18-crown-6 (2.65 g, 5 mmol) and o-dichlorobenzene (200 mL) were added to a 500 mL two-necked flask under nitrogen atmosphere, and the solution was heated to 150° C and reacted under stirring for 24 hours, and then the reaction was ended. The reaction solution was distilled under reduced pressure to remove most of the solvent, and then dissolved with dichloromethane and washed with water for 3 times. The organic solution was collected, mixed with silica gel, and purified by column chromatography, with a yield of 80%.

2)

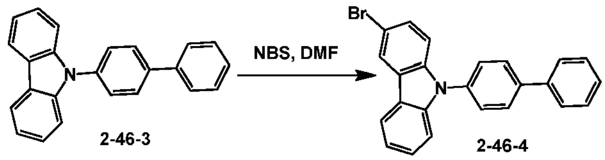


2-46-3 2-46-4

Compound 2-46-3 (19.1 g, 60 mmol) and 100 mL of N,N-dimethylformamide were added into a 250 mL single-necked flask, and 60 mmol N,N-dimethylformamide solution of NBS was added dropwise in an ice bath. The solution was reacted under stirring for 12 hours in the dark, and then the reaction was ended. The reaction solution was poured into 500 mL of water, filtered with suction, and the filter residue was recrystallized, with a yield 90%.

3)

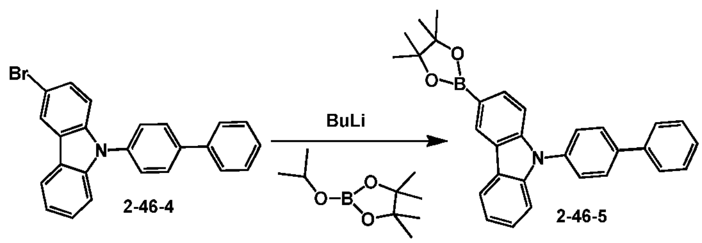


2-46-4 2-46-5

Compound (2-46-4) (15.9 g, 40 mmol) and 300 mL of anhydrous tetrahydrofuran were added to a 500 mL three-necked flask under nitrogen atmosphere, cooled to -78°C, and 50 mmol of n-butyllithium was slowly added dropwise, the solution was reacted for 2 hours, then 55 mmol of isopropoxyboronic acid pinacol ester was added one time to allow

the reaction temperature to rise to room temperature naturally. The reaction was further performed for 12 hours and then quenched by the addition of pure water. The reaction solution was rotary evaporated to remove most of the solvent, and then extracted with dichloromethane and washed with water for 3 times. The organic phase was collected, spin dried, and then recrystallized, with a yield of 80%.

4)

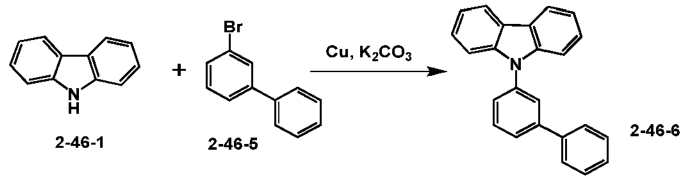


Compound (2-46-1) (16.7 g, 100 mmol) and Compound (2-46-5) (24.5 g, 105 mmol), copper powder (0.65 g, 10 mmol), potassium carbonate (13.8 g, 100 mmol) and 18-crown-6 (2.65 g, 5 mmol) and o-dichlorobenzene (200 mL) were added to a 500 mL two-necked flask under nitrogen atmosphere, and the solution was heated to 150° C and reacted under stirring for 24 hours, and then the reaction was ended. The reaction solution was distilled under reduced pressure to remove most of the solvent, and then dissolved with dichloromethane and washed with water for 3 times. The organic solution was collected, mixed with silica gel, and purified by column chromatography, with a yield of 75%.

5)

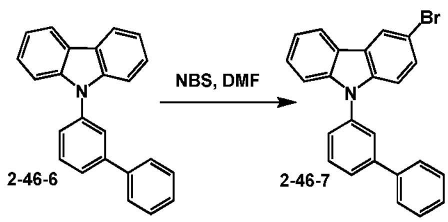


Compound 2-46-6 (19.1 g, 60 mmol) and 100 mL of N,N-dimethylformamide were added into a 250 mL single-necked flask, and 60 mmol N,N-dimethylformamide of NBS was added dropwise in an ice bath. The solution was reacted under stirring for 12 hours in the dark, and then the reaction was ended. The reaction solution was poured into 500 mL of water, filtered with suction, and the filter residue was recrystallized, with a yield 88%.

6)

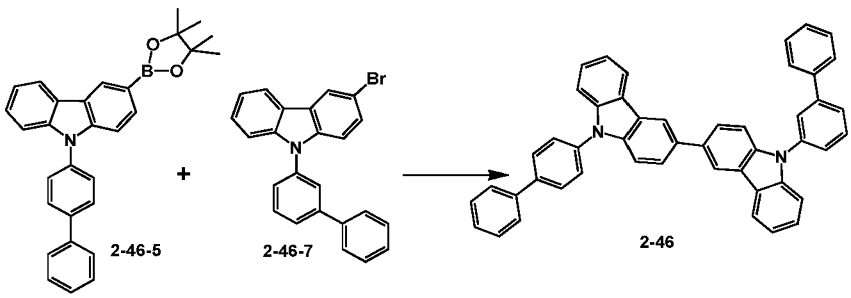


Compound 2-46-5 (4.45 g, 20 mmol) and Compound 2-46-7 (3.98 g, 20 mmol), tetrakis(triphenylphosphine)palladium (1.15 g, 1 mmol), tetrabutylammonium bromide (2.6 g, 8 mmol), sodium hydroxide (3.2 g, 80 mmol), water (10 mL) and toluene (100 mL) were added to a 250 mL three-necked flask under nitrogen atmosphere, and the mixture was heated to 80°C and reacted under stirring for 12 hours, and then the reaction was ended. The reaction solution was rotary evaporated to remove most of the solvent, and then dissolved with dichloromethane and washed with water for 3 times. The organic solution was collected, mixed with silica gel, and then purified by column chromatography, with a yield of 80%.

(6) Synthesis of Compound (2-49):

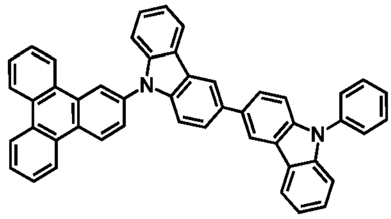

(2-49)

1)

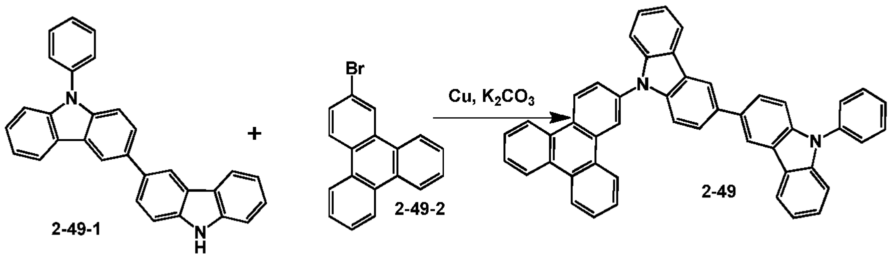


Compound (2-49-1) (10 g, 60 mmol) and Compound (3-49-2) (18.4 g, 60 mmol), copper powder (0.39 g, 6 mmol), potassium carbonate (8.28 g, 60 mmol) and 18-crown-6 (2.65 g, 5 mmol) and o-dichlorobenzene (150 mL) were added to a 300 mL two-necked flask under nitrogen atmosphere, and the mixture was heated to 150°C and reacted under stirring for 24 hours, and then the reaction was ended. The reaction solution was distilled under reduced pressure to remove most of the solvent, and then dissolved with dichloromethane and washed with water for 3 times. The organic solution was collected, mixed with silica gel, and purified by column chromatography, with a yield of 85%.

(7) Synthesis of Compound (2-92):

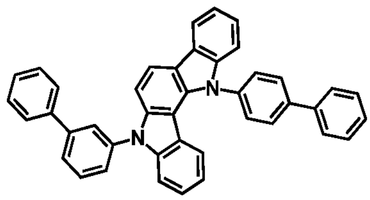

(2-92)

1)

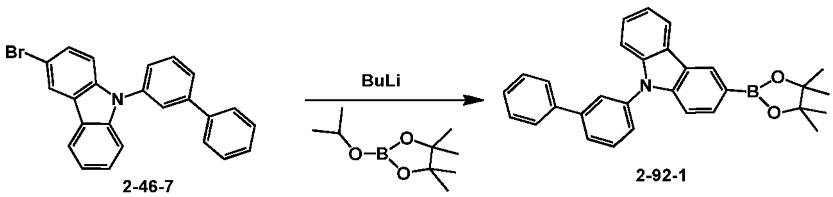


Compound (2-46-7) (31.5 g, 80 mmol) and 300 mL of anhydrous tetrahydrofuran were added to a 500 mL three-necked flask under nitrogen atmosphere, cooled to -78°C, and 85 mmol of n-butyllithium was slowly added dropwise, the solution was reacted for 2 hours, then 90 mmol of isopropoxyboronic acid pinacol ester was added one time to allow the reaction temperature to rise to room temperature naturally. The reaction was further performed for 12 hours and then quenched by the addition of pure water. The reaction solution was rotary evaporated to remove most of the solvent,

and then extracted with dichloromethane and washed with water for 3 times. The organic phase was collected, spin dried, and then recrystallized, with a yield of 90%.

2)

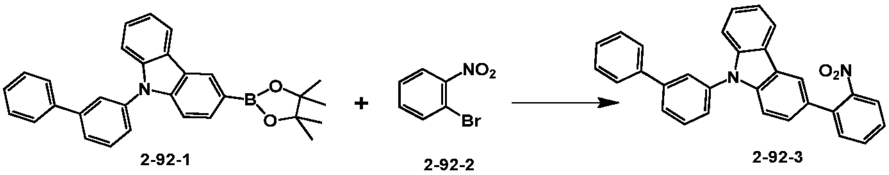

2-92-1 2-92-2 2-92-3

Compound 2-92-1 (26.7g, 60 mmol) and Compound 2-92-2 (12.1g, 60 mmol), tetrakis(triphenylphosphine)palladium (3.45 g, 3mmol), tetrabutylammonium bromide (7.8 g, 24mmol), sodium hydroxide (4.8 g, 120 mmol), water (15 mL) and toluene (120 mL) were added to a 250 mL three-necked flask under nitrogen atmosphere, and the solution was heated to 80°C and reacted under stirring for 12 hours, and then the reaction was ended. The reaction solution was rotary evaporated to remove most of the solvent, and then dissolved with dichloromethane and washed with water for 3 times. The organic solution was collected, mixed with silica gel, and then purified by column chromatography, with a yield of 80%.

3)

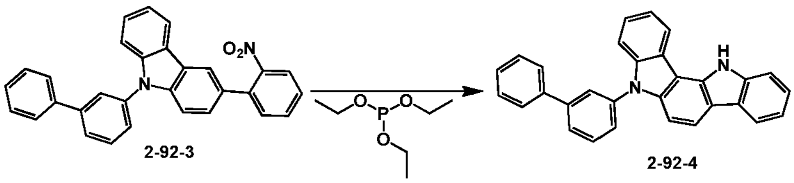

2-92-3 2-92-4

Compound 9-1-11 (13.2 g, 30 mmol) and triethylphosphine (10 g, 60 mmol) were added to a 150 mL two-necked flask under nitrogen atmosphere, and the mixture was heated to 190°C and reacted under stirring for 12 hours, and then the reaction was ended. The reaction solution was distilled under reduced pressure to remove most of the solvent, and then dissolved with dichloromethane and washed with water for 3 times. The organic solution was collected, mixed with silica gel, and then purified by column chromatography, with a yield of 85%.

4)

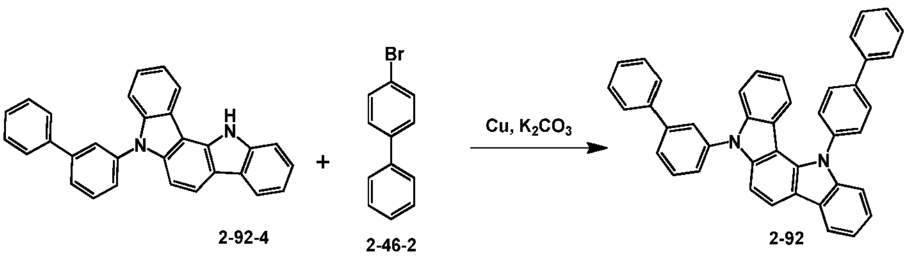


2-92-4 2-46-2 2-92

Compound (2-49-4) (8.16 g, 20 mmol) and Compound (3-46-2) (4.66 g, 60 mmol), copper powder (0.26 g, 4 mmol), potassium carbonate (5.5 g, 40 mmol) and 18-crown-6 (2.12 g, 4 mmol) and o-dichlorobenzene (100 mL) were added to a 250 mL two-necked flask under nitrogen atmosphere, and the mixture was heated to 150°C and reacted under stirring for 24 hours, and then the reaction was ended. The reaction solution was distilled under reduced pressure to remove most of the solvent, and then dissolved with dichloromethane and washed with water for 3 times. The organic solution was collected, mixed with silica gel, and purified by column chromatography, with a yield of 85%.

(8) Reference Example Ref-2:

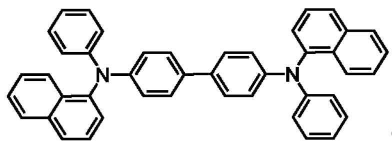

(Ref-2)

1)

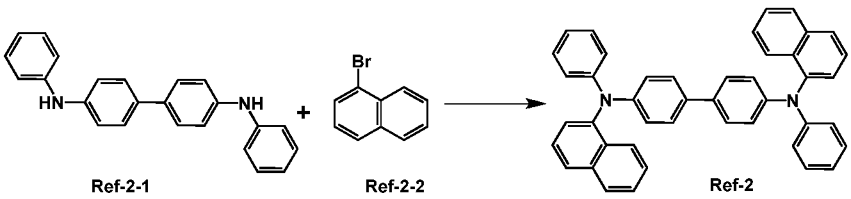


Compound Ref-2-1 (16.8 g, 50 mmol) and Compound Ref-2-2 (20.7 g, 100 mmol), (2.3 g, 2.5 mmol), tri-tert-butylphosphine (1 g, 5 mmol) and sodium tert-butoxide (9.6 g, 100 mmol) and toluene (150 mL) were added into a 250 mL two-necked flask under nitrogen atmosphere, and the mixture was refluxed with heating and reacted under stirring for 12 hours, and then the reaction was ended. The reaction solution was distilled under reduced pressure to remove most of the solvent, and then dissolved with dichloromethane and washed with water for 3 times. The organic solution was collected, mixed with silica gel, and purified by column chromatography, with a yield of 70%.

(9) Reference Example Ref-3:

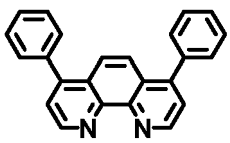

,

purchased from Jilin OLED Material Tech Co., Ltd.

(10) Reference Example Ref-4:

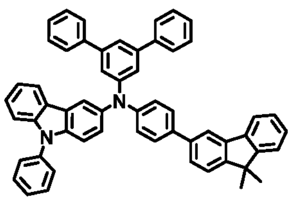

The synthesis of Reference Example Ref-4 can be found in patent WO 2015041428 in prior art.

2. Energy Structure of Organic Compounds

The energy levels of organic materials can be obtained by quantum calculations, such as using TD-DFT (Time Dependent-Density Functional Theory) by Gaussian03W (Gaussian Inc.), and the specific simulation methods can be found in WO2011141110. Firstly, the molecular geometry is optimized by semi-empirical method "Ground State/Semi-empirical/Default Spin/AM1" (Charge 0/Spin Singlet), and then the energy structure of organic molecules is calculated by TD-DFT (time-density functional theory) "TD-SCF/DFT/Default Spin/B3PW91" and the basis set "6-31G (d)" (Charge 0/Spin Singlet). The HOMO and LUMO levels are calculated according to the following calibration formulas, S1 and T1 are used directly.

$$HOMO(eV) = ((HOMO(G)\times 27.212)-0.9899)/1.1206$$

$$LUMO(eV) = ((LUMO(G)\times 27.212)-2.0041)/1.385$$

wherein, HOMO(G) and LUMO(G) in the unit of Hartree are the direct calculation results of Gaussian 03W. The results were shown in Table 1:

Table 1

| Materials | HOMO [eV] | LUMO [eV] | T1 [eV] | S1 [eV] |
|---|---|---|---|---|
| HATCN | -9.04 | -5.08 | 2.32 | 3.17 |
| SFNFB | -5.26 | -2.19 | 2.59 | 3.22 |
| (1-36) | -6.01 | -2.86 | 2.92 | 3.29 |
| (1-37) | -6.01 | -2.84 | 2.95 | 3.26 |
| (1-44) | -5.92 | -2.85 | 2.75 | 3.18 |
| (Ref-1) | -6.46 | -2.79 | 3.00 | 3.49 |
| (Ref-3) | -6.36 | -2.56 | 2.69 | 3.56 |
| (2-46) | -5.44 | -2.22 | 2.92 | 3.12 |
| (2-49) | -5.44 | -2.36 | 2.69 | 3.17 |
| (Ref-2) | -5.24 | -2.34 | 2.46 | 3.13 |
| (Ref-4) | -5.16 | -2.21 | 2.69 | 2.98 |
| $Ir(p\text{-}ppy)_3$ | -5.17 | -2.32 | 2.67 | 2.90 |
| $NaTzF_2$ | -6.19 | -2.82 | 2.55 | 3.52 |

| Mixtures | | | Δ( (HOMO-(HOMO-1)) [eV] | Δ( (LUMO+1)-LUMO) [eV] | min((LUMO(H1) -HOMO(H2), LUMO(H2)- | min($E_T$(H1), $E_T$(H2))[eV] |
|---|---|---|---|---|---|---|
| 1 | H1 | (1-36) | 0.28 | 0.20 | 2.58 | 2.92 |
| | H2 | (2-46) | 0.42 | 0.05 | | |
| 2 | H1 | (1-36) | 0.28 | 0.20 | 2.58 | 2.69 |
| | H2 | (2-49) | 0.42 | 0.03 | | |
| 3 | H1 | (1-37) | 0.28 | 0.19 | 2.60 | 2.92 |
| | H2 | (2-46) | 0.42 | 0.05 | | |
| 4 | H1 | (1-37) | 0.28 | 0.19 | 2.60 | 2.69 |
| | H2 | (2-49) | 0.42 | 0.03 | | |
| 5 | H1 | (Ref-1) | 0.01 | 0 | 2.45 | 2.46 |
| | H2 | (Ref-2) | 0.36 | 0.02 | | |
| 6 | H1 | (Ref-3) | 0.33 | 0.02 | 2.60 | 2.69 |
| | H2 | (Ref-4) | 0.78 | 0.10 | | |

### 3. Preparation and Characterization of OLED Devices

In the present embodiment, compounds (1-36) and (2-46), (1-36) and (2-49), (1-37) and (2-46), (1-37) and (2-49), (Ref-1) and (Ref-2) with mass ratio of 1:1 were used as the host material respectively, $Ir(ppy)_3$ as the light-emitting material, HATCN as the hole injection material, SFNFB as the hole transport material, NaTzF2 as the electron transport material, and Liq as the electron injection material, to prepare an electroluminescent device with a device structure of ITO/HATCN/SFNFB/host material: $Ir(ppy)_3$(10%)/$NaTzF_2$:Liq/Liq/Al.

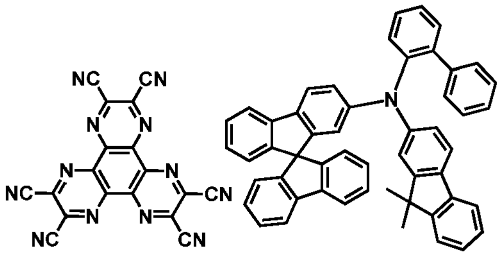
HATCN SFNFB
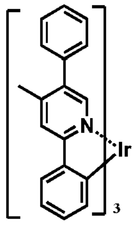
$Ir(p\text{-}ppy)_3$
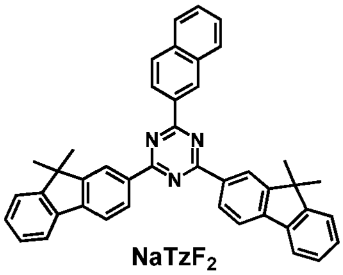
$NaTzF_2$
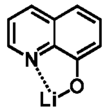
Liq

The above materials such as HATCN, SFNFB, $Ir(p\text{-}ppy)_3$, $NaTzF_2$ and Liq are all commercially available, such as from Jilin OLED Material Tech Co., Ltd (www.jl-oled.com), or all the synthesis methods thereof are all known which can be found in the references of the art and will not be described here.

The preparation process of the above OLED device will be described in detail through a specific embodiment. The structure of the OLED device (as shown in Table 2) is: ITO/HATCN/SFNFB/host material: Ir(p-ppy)3 (10%)/NaTzF2:Liq/Liq /Al, and the preparation steps are as follows:

a. Cleaning of ITO (Indium Tin Oxide) conductive glass substrate: cleaning the substrate with a variety of solvents (such as one or more of chloroform, acetone or isopropanol), and then treating with ultraviolet and ozone;

b. HATCN (30 nm), SFNFB (50 nm), host material: 10% Ir(p-ppy)3 (40 nm), NaTzF2:Liq (30 nm), Liq (1 nm), Al (100 nm) were formed by thermal evaporation in high vacuum ($1\times10^{-6}$ mbar);

c. Encapsulating: encapsulating the device with UV-curable resin in a nitrogen glove box. Wherein, the host material may be prepared in the following forms:

(1) Simple blending, i.e., the two host materials were weighed according to a certain ratio, doped together, ground at room temperature, and the resulting mixture was placed in an organic source for evaporation.

(2) Or Organic alloy, i.e., the two host materials were weighed according to a certain ratio, doped together, heated and stirred until the mixture was melted under a vacuum lower than $10^{-3}$ torr. The mixture was cooled and then ground, and the resulting mixture was placed in an organic source for evaporation.

Table 2

| OLED devices | Host materials | T90@1000nits |
|---|---|---|
| OLED1 | (1-36): (2-46)=1:1 Simple blending | 3.2 |
| OLED2 | (1-36): (2-46)=1:1 Organic alloy | 4.7 |
| OLED3 | (1-36): (2-49)=1:1 Simple blending | 2.5 |
| OLED4 | (1-36): (2-49)=1:1 Organic alloy | 3.8 |
| OLED5 | (1-37): (2-46)=1:1 Simple blending | 4.8 |
| OLED6 | (1-37): (2-46)=1:1 Organic alloy | 6.5 |
| OLED7 | (1-37): (2-49)=1:1 Simple blending | 3.6 |
| OLED8 | (1-37): (2-49)=1:1 Organic alloy | 5.2 |
| OLED9 | (Ref-1): (Ref-2)=1:1 Simple blending | 1 |
| OLED10 | (Ref-1): (Ref-2)=1:1 Organic alloy | 1.2 |
| OLED11 | (Ref-3): (Ref-4)=1:1 Simple blending | 1.4 |
| OLED12 | (Ref-3): (Ref-4)=1:1 Simple blending | 1.6 |

The current-voltage (J-V) characteristics of each OLED device were characterized by characterization equipment while important parameters such as efficiency, lifetime (as shown in Table 2), and external quantum efficiency were recorded. In Table 2, all lifetimes are relative to OLED9. It can be seen that the light-emitting lifetimes of the devices OLED2, OLED4, OLED6, OLED8 and OLED10 based on organic alloy are relatively high compared with the same type of devices, wherein the lifetime of OLED6 is 6.5 times that of Ref OLED. In the case where the condition 1) is satisfied but the condition 2) is not satisfied, the lifetimes of OLED 11 and OLED 12 are significantly lower than those of OLED 7 and OLED 8, and it can be seen that the lifetime of the OLED device prepared by using the organic mixture which simultaneously satisfies conditions 1) and 2) has been greatly improved.

The organic compound H1 and the organic compound H2 according to the disclosure are easy to form exciplexes and have balanced electron transmission properties, the organic compound H1 has high stability of electron transmission, and accordingly the efficiency and lifetime of related electronic components can be effectively improved, and a feasible solution for improving overall performance of the electronic components is provided.

It is apparent that the above examples are merely examples for clear descriptions, but not intended to limit the embodiments. Other modifications and changes in various forms can be made by those skilled in the art based on the above description. There is no need and no way to exhaust all of the embodiments.

## Claims

1. An organic mixture comprising an organic compound H1 and an organic compound H2, wherein 1) min((LUMO(H1)-HOMO(H2), LUMO(H2)-HOMO(H1)) ≤ min($E_T$(H1), $E_T$(H2))+0.1eV, wherein, LUMO(H1), HOMO(H1) and $E_T$(H1) are the lowest unoccupied molecular orbital, highest occupied molecular orbital and triplet excited state energy levels of the organic compound H1, respectively, and LUMO(H2), HOMO(H2) and $E_T$(H2) are the lowest unoccupied molecular orbital, highest occupied molecular orbital and triplet excited state energy levels of the organic compound H2, respectively;
**characterized in that** 2) (LUMO+1)(H1)-LUMO(H1)>0.1eV, wherein, (LUMO+1)(H1) is the second lowest unoccupied molecular orbital energy level of the organic compound H1.

2. The organic mixture according to claim 1, wherein (LUMO+1)(H1)-LUMO(H1) ≥0.15 eV.

3. The organic mixture according to any one of claims 1 to 2, wherein the organic compound H1 is a compound represented by the formula (1):

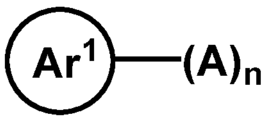

(1)

wherein, $Ar^1$ is selected from H atom, an aromatic group containing 5 to 90 ring atoms or a heteroaromatic group containing 5 to 90 ring atoms; A is an electron-accepting group; n is an integer from 1 to 6; when n is greater than 1, a plurality of the electron-accepting groups are the same or different.

4. The organic mixture according to claim 3, wherein the electron-accepting group is F or cyano, while $Ar^1$ is not H atom; or the electron-accepting group comprises a group formed by one or more of the following structures:

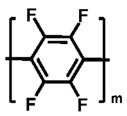 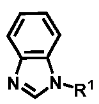 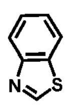 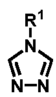 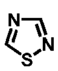 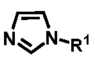 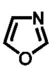 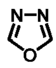 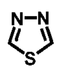 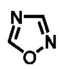 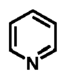 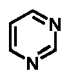 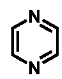 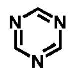

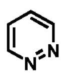 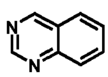 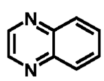 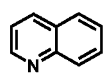 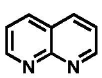 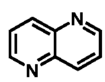 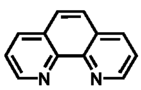 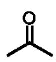 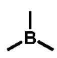 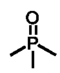 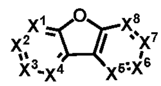

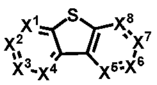
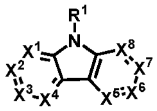
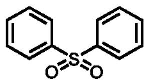
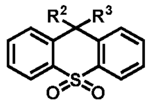
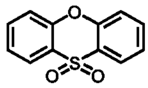
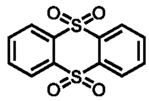
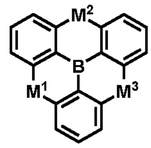

wherein, m is 1, 2 or 3; $X^1$ to $X^8$ are selected from CR or N, and at least one of $X^1$ to $X^8$ is N; $M^1$, $M^2$ and $M^3$ each independently represent N(R), $C(R)_2$, $Si(R)_2$ , O, C=N(R), $C=C(R)_2$, P(R), P(=O)R, S, S=O, $SO_2$ or none; wherein $R^1$, $R^2$, $R^3$ and R each independently represent alkyl, alkoxy, amino, alkenyl, alkynyl, aralkyl, heteroalkyl, aryl or heteroaryl.

**5.** The organic mixture according to any one of claims 1 to 4, wherein the organic compound H2 is a compound represented by the formula (2):

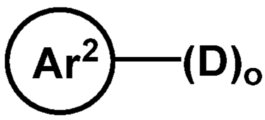
(2)

wherein, $Ar^2$ is selected from H atom, an aromatic group containing 5 to 90 ring atoms or a heteroaromatic group containing 5 to 90 ring atoms; D is an electron-donating group; o is an integer from 1 to 6; when o is greater than 1, a plurality of the electron-donating groups are the same or different.

**6.** The organic mixture according to claim 5, wherein the electron-donating group comprises a group formed by one or more of the following structures:

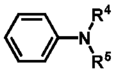
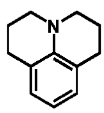
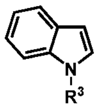
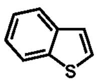
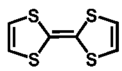
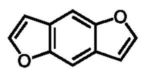

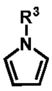
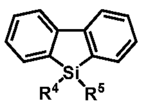
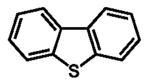
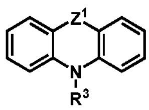
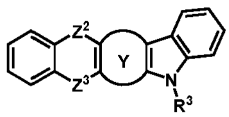

wherein, Y represents an aromatic group containing 5 to 40 carbon atoms or a heteroaromatic group containing 5 to 40 carbon atoms; $Z^1$, $Z^2$ and $Z^3$ each independently represent a single bond, N(R), $C(R)_2$, $Si(R)_2$ , O, S, C=N(R), $C=C(R)_2$ or P(R); wherein, R, $R^3$, $R^4$ and $R^5$ each independently represent alkyl, alkoxy, amino, alkenyl, alkynyl, aralkyl, heteroalkyl, aryl or heteroaryl.

**7.** The organic mixture according to any one of claims 3-6, wherein $Ar^1$ or $Ar^2$ comprises a group formed by one or more of the following structures:

| | | |
|---|---|---|
| 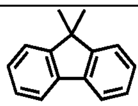 | 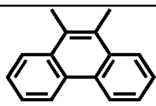 | 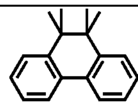 |
| 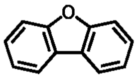 | 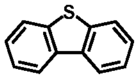 | 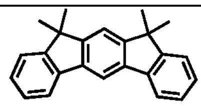 |
| 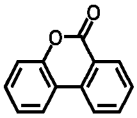 | 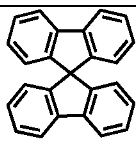 | 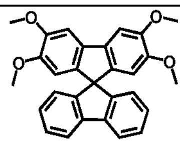 |

| | | |
|---|---|---|
| 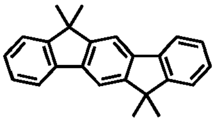 | 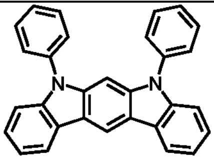 | 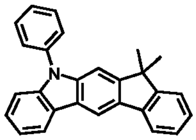 |
| 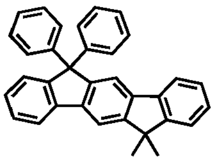 | 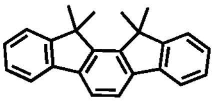 | 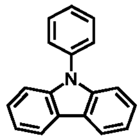 |
| 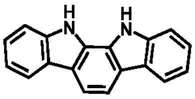 | 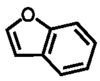 | 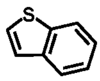 |
| 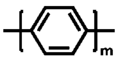 | 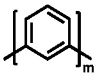 | 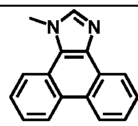 |
| 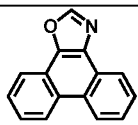 | 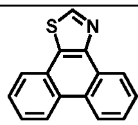 | 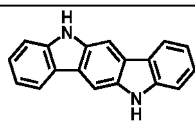 |
| 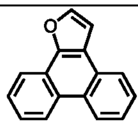 | 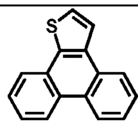 | 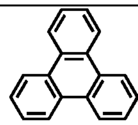 |
| 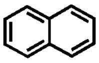 | 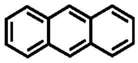 | 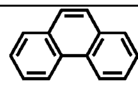 |
| 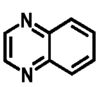 | 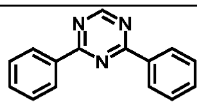 | 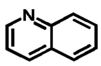 |

wherein, m is 1, 2 or 3.

**8.** The organic mixture according to any one of claims 2 to 7, wherein the organic compound H1 is selected from one or more of the compounds represented by the following general formulas (3) to (6):

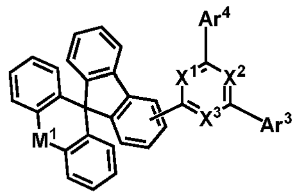 (3) , 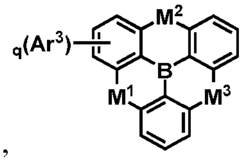 (4) ,

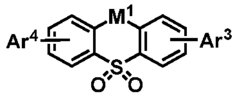 (5) and 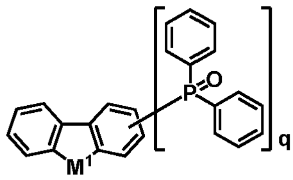 (6) ,

wherein, $Ar^3$ and $Ar^4$ each independently represent an aromatic group containing 5 to 60 ring atoms or a heteroaromatic group with containing 5 to 60 ring atoms; q is an integer from 1 to 6; $X^1$, $X^2$ and $X^3$ are each independently selected from CR or N, and at least one of $X^1$, $X^2$ and $X^3$ is N; $M^1$, $M^2$ and $M^3$ each independently represent N(R), $C(R)_2$, $Si(R)_2$, O, C=N(R), $C=C(R)_2$, P(R), P(=O)R, S, S=O, $SO_2$ or none; wherein, R represents alkyl, alkoxy, amino, alkenyl, alkynyl, aralkyl, heteroalkyl, aryl or heteroaryl.

**9.** The organic mixture according to any one of claims 2 to 8, wherein the organic compound H1 is selected from one or more of the following compounds:

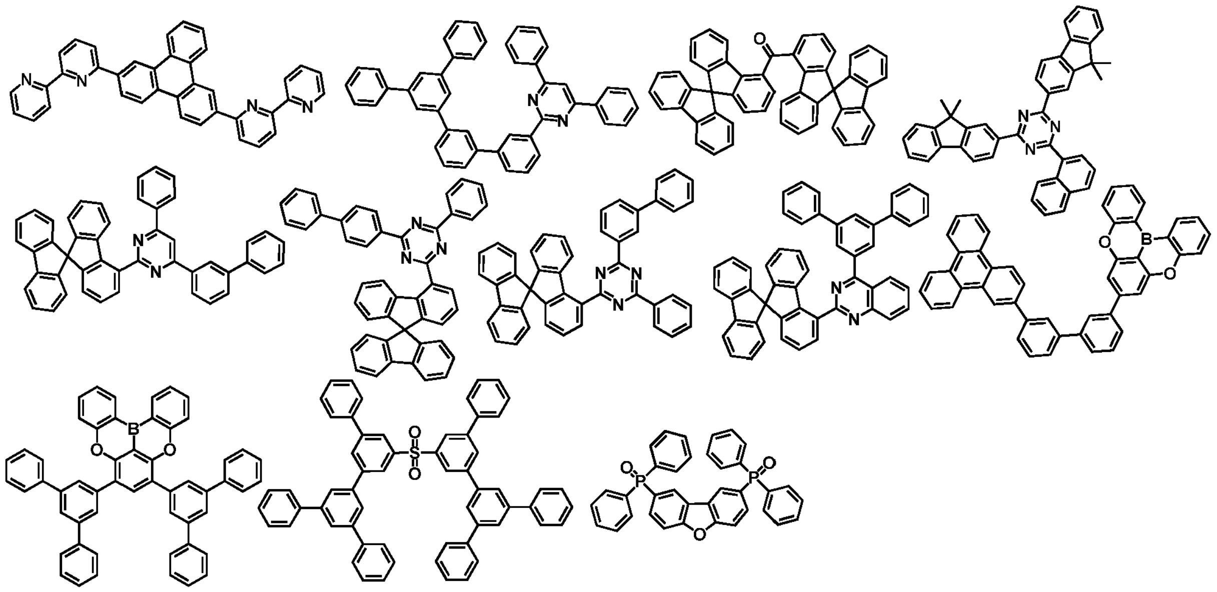

;

or the organic compound H2 is one or more of the following compounds:

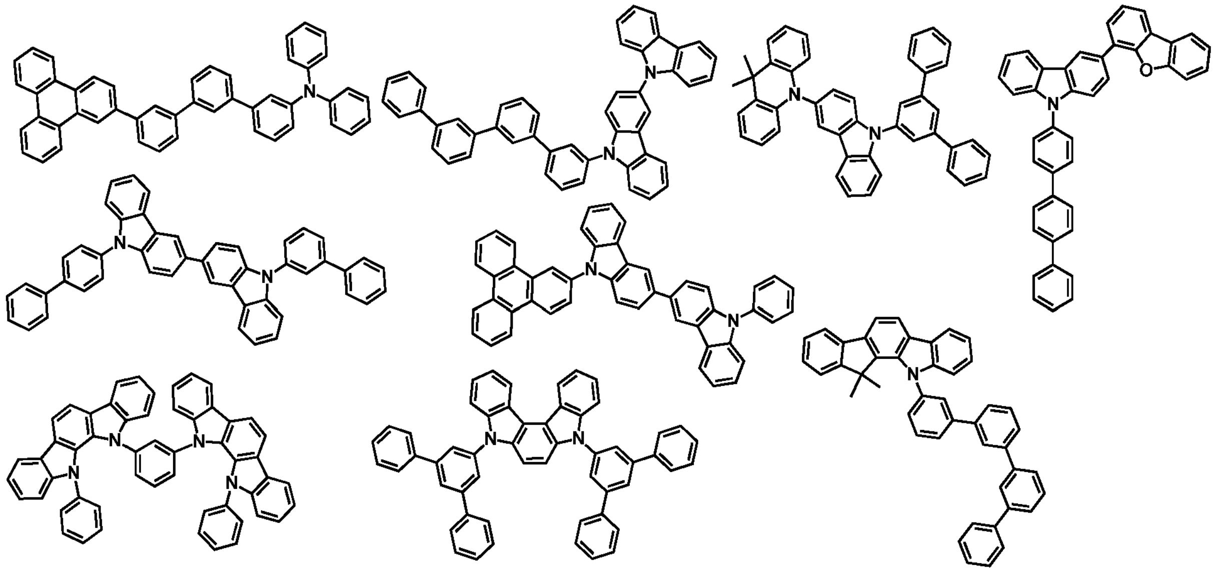

.

10. The organic mixture according to any one of claims 1 to 9, wherein the difference in molecular weight between the organic compound H1 and the organic compound H2 does not exceed 100 Dalton.

11. The organic mixture according to any one of claims 1 to 10, wherein the organic mixture further comprises a light-emitting material selected from at least one of a fluorescent emitter, a phosphorescent emitter and a TADF material.

12. A formulation **characterized by** comprising the organic mixture according to any one of claims 1 to 11 and an organic solvent.

13. An organic electronic device **characterized by** comprising a functional layer including the organic mixture according to any one of claims 1 to 11.

14. The organic electronic device according to claim 13, wherein the organic electronic device is an organic light-emitting diode (OLED), an organic photovoltaic cell (OPV), an organic light-emitting electrochemical cell (OLEEC), an organic field effect transistor (OFET), an organic light-emitting field effect transistor, an organic sensor or an organic plasmon emitting diode.

## Patentansprüche

1. Organische Mischung, umfassend eine organische Verbindung H1 und eine organische Verbindung H2, wobei 1) min((LUMO(H1)-HOMO(H2), LUMO(H2)-HOMO(H1)) $\leq$ min($E_T$(H1), $E_T$(H2))+0.1eV, wobei LUMO(H1), HOMO(H1) und $E_T$(H1) jeweils die niedrigsten unbesetzten Molekülorbital-, die höchsten besetzten Molekülorbital-und Triplett-angeregten Zustand-Energieniveaus der organischen Verbindung H1 sind, und LUMO(H2), HOMO(H2) und $E_T$(H2) jeweils die niedrigsten unbesetzten Molekülorbital-, die höchsten besetzten Molekülorbital-und Triplett-angeregten Zustand-Energieniveaus der organischen Verbindung H2 sind;
**dadurch gekennzeichnet, dass** 2) (LUMO+1)(H1)-LUMO(H1)$\geq$0.1eV, wobei (LUMO+1)(H1) das zweitniedrigste unbesetzte Molekülorbital-Energieniveau der organischen Verbindung H1 ist.

2. Organische Mischung nach Anspruch 1, wobei (LUMO+1)(H1)-LUMO(H1)$\geq$0.15eV.

3. Organische Mischung nach einem der Ansprüche 1 bis 2, wobei die organische Verbindung H1 eine Verbindung ist, die durch die Formel (1) repräsentiert ist:

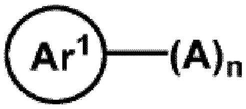

(1)

wobei $Ar^1$ aus H-Atom, einer aromatischen Gruppe enthaltend von 5 bis 90 Ringatome oder eine heteroaromatische Gruppe enthaltend von 5 bis 90 Ringatome ausgewählt ist; A eine elektronenakzeptierende Gruppe ist; n eine ganze Zahl von 1 bis 6 ist; wenn n größer als 1 ist, ist eine Vielzahl an der elektronenakzeptierenden Gruppen gleich oder verschieden.

**4.** Organische Mischung nach Anspruch 3, wobei die elektronenakzeptierende Gruppe F oder Cyano ist, während $Ar^1$ kein H-Atom ist; oder die elektronenakzeptierende Gruppe eine Gruppe umfasst, die durch eine oder mehrere der folgenden Strukturen gebildet ist:

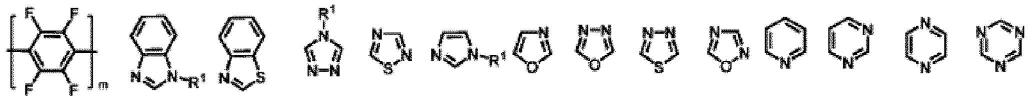

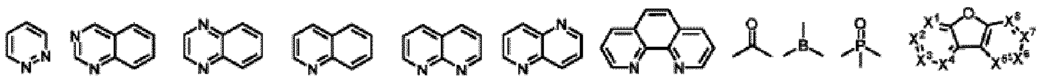

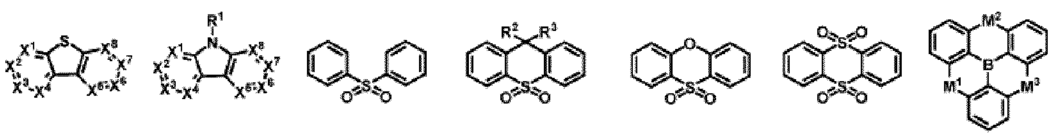

wobei m 1, 2 oder 3 ist; von $X^1$ bis $X^8$ aus CR oder N ausgewählt sind, und mindestens eines von $X^1$ bis $X^8$ N ist; $M^1$, $M^2$ und $M^3$, jeweils unabhängig, N(R), $C(R)_2$, $Si(R)_2$, O, C=N(R), $C=C(R)_2$, P(R), P(=O)R, S, S=O, $SO_2$ oder keine repräsentieren; wobei $R^1$ $R^2$, $R^3$ und R, jeweils unabhängig, Alkyl, Alkoxy, Amino, Alkenyl, Alkinyl, Aralkyl, Heteroalkyl, Aryl oder Heteroaryl repräsentieren.

**5.** Organische Mischung nach einem der Ansprüche 1 bis 4, wobei die organische Verbindung H2 eine Verbindung ist, die durch die Formel (2) repräsentiert ist:

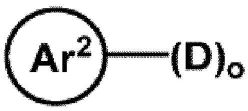

(2)

wobei $Ar^2$ aus H-Atom, einer aromatischen Gruppe enthaltend von 5 bis 90 Ringatome oder eine heteroaromatische Gruppe enthaltend von 5 bis 90 Ringatome ausgewählt ist; D eine elektronenspendende Gruppe ist; o eine ganze Zahl von 1 bis 6 ist; wenn o größer als 1 ist, ist eine Vielzahl an der elektronenspendenden Gruppen gleich oder verschieden.

**6.** Organische Mischung nach Anspruch 5, wobei die elektronenspendende Gruppe eine Gruppe umfasst, die durch eine oder mehrere der folgenden Strukturen gebildet ist:

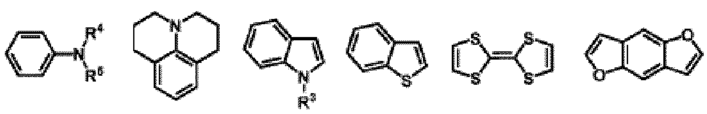

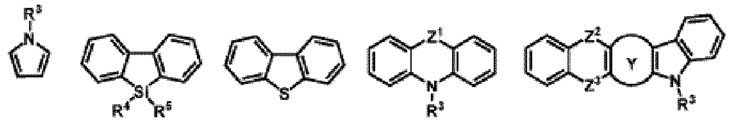

wobei Y eine aromatische Gruppe enthaltend von 5 bis 40 Kohlenstoffatome oder eine heteroaromatische Gruppe enthaltend von 5 bis 40 Kohlenstoffatome repräsentiert; $Z^1$, $Z^2$ und $Z^3$, jeweils unabhängig, eine Einfachbindung, N(R), $C(R)_2$, $Si(R)_2$, O, S, C=N(R), $C=C(R)_2$ oder P(R) repräsentiert; wobei R, $R^3$, $R^4$ und $R^5$, jeweils unabhängig, Alkyl, Alkoxy, Amino, Alkenyl, Alkinyl, Aralkyl, Heteroalkyl, Aryl oder Heteroaryl repräsentieren.

**7.** Organische Mischung nach einem der Ansprüche 3-6, wobei $Ar^1$ oder $Ar^2$ eine Gruppe umfasst, die durch eine oder mehrere der folgenden Strukturen gebildet ist:

| | | |
|---|---|---|
| 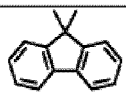 | 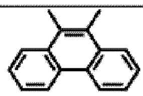 | 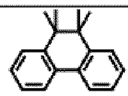 |
| 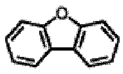 | 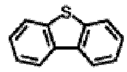 | 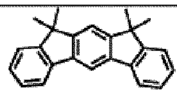 |
| 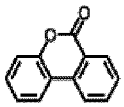 | 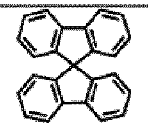 | 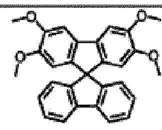 |

| | | |
|---|---|---|
| 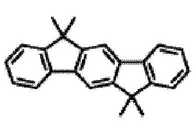 | 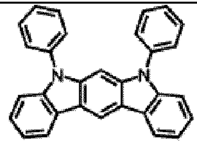 | 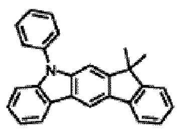 |
| 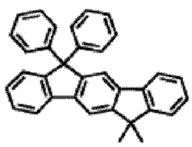 | 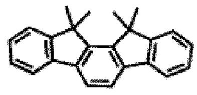 | 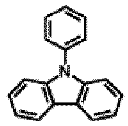 |
| 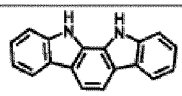 | 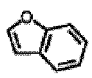 | 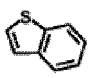 |
| 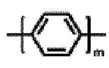 | 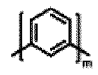 | 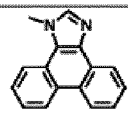 |
| 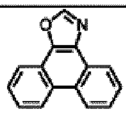 | 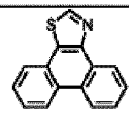 | 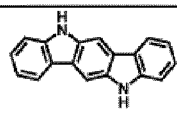 |
| 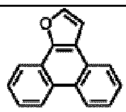 | 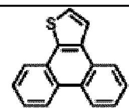 | 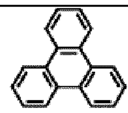 |
| 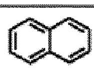 | 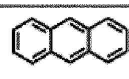 | 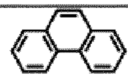 |
| 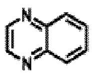 | 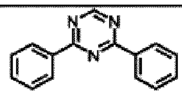 | 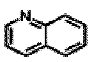 |

wobei m 1, 2 oder 3 ist.

**8.** Organische Mischung nach einem der Ansprüche 2 bis 7, wobei die organische Verbindung H1 aus einer oder mehreren der Verbindungen ausgewählt ist, die durch die folgenden allgemeinen Formeln (3) bis (6) repräsentiert sind:

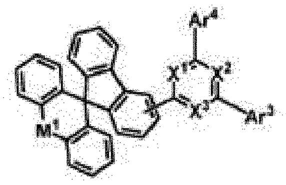 (3),
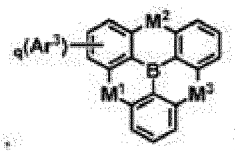 (4),

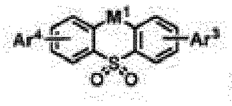 (5) und 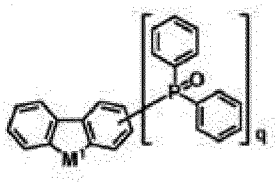 (6),

wobei $Ar^3$ und $Ar^4$, jeweils unabhängig, eine aromatische Gruppe enthaltend von 5 bis 60 Ringatome oder eine heteroaromatische Gruppe enthaltend von 5 bis 60 Ringatome repräsentieren; q eine ganze Zahl von 1 bis 6 ist; $X^1$, $X^2$ und $X^3$, jeweils unabhängig, aus CR oder N ausgewählt sind, und mindestens eines von $X^1$, $X^2$ und $X^3$ N ist; $M^1$, $M^2$ und $M^3$, jeweils unabhängig, N(R), $C(R)_2$, $Si(R)_2$ , O, C=N(R), $C=C(R)_2$, P(R), P(=O)R, S, S=O, $SO_2$ oder keine repräsentieren; wobei R Alkyl, Alkoxy, Amino, Alkenyl, Alkinyl, Aralkyl, Heteroalkyl, Aryl oder Heteroaryl repräsentiert.

**9.** Organische Mischung nach einem der Ansprüche 2 bis 8, wobei die organische Verbindung H1 aus einer oder mehreren der Verbindungen ausgewählt ist:

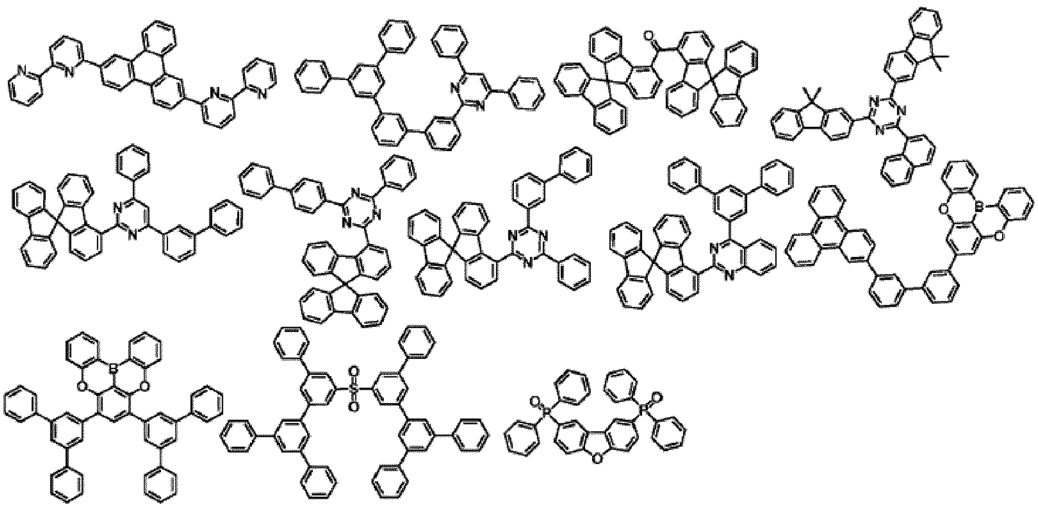

oder die organische Verbindung H2 eine oder mehrere der folgenden Verbindungen ist:

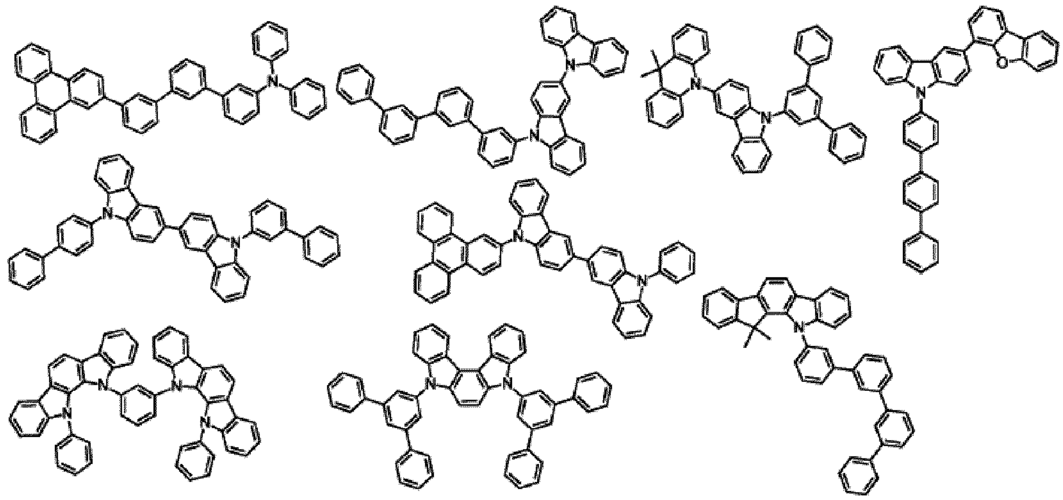

**10.** Organische Mischung nach einem der Ansprüche 1 bis 9, wobei der Unterschied im Molekulargewicht zwischen der organischen Verbindung H1 und der organischen Verbindung H2 100 Dalton nicht überschreitet.

**11.** Organische Mischung nach einem der Ansprüche 1 bis 10, wobei die organische Mischung ferner ein lichtemittierendes Material umfasst, das aus mindestens einem fluoreszierenden Emitter, einem phosphoreszierenden Emitter und einem TADF-Material ausgewählt ist.

**12.** Formulierung, die **dadurch gekennzeichnet ist, dass** sie die organische Mischung nach einem der Ansprüche 1 bis 11 und ein organisches Lösungsmittel umfasst.

**13.** Organische elektronische Vorrichtung, die **dadurch gekennzeichnet ist, dass** sie eine Funktionsschicht umfasst, die die organische Mischung nach einem der Ansprüche 1 bis 11 beinhaltet.

**14.** Organische elektronische Vorrichtung nach Anspruch 13, wobei die organische elektronische Vorrichtung eine organische lichtemittierende Diode (OLED), eine organische Photovoltaikzelle (OPV), eine organische lichtemittierende elektrochemische Zelle (OLEEC), ein organischer Feldeffekttransistor (OPET), ein organischer lichtemittierender Feldeffekttransistor, ein organischer Sensor oder eine organische Plasmon emittierende Diode ist.

## Revendications

**1.** Mélange organique comprenant un composé organique H1 et un composé organique H2, dans lequel 1) min((LUMO(H1)-HOMO(H2), LUMO(H2)-HOMO(H1)) $\leq$ min($E_T$(H1), $E_T$(H2))+0.1eV, où LUMO(H1), HOMO(H1) et $E_T$(H1) sont, respectivement, les niveaux d'énergie de l'orbitale moléculaire inoccupée la plus basse, de l'orbitale moléculaire occupée la plus haute et de l'état triplet excité du composé organique H1, et LUMO(H2), HOMO(H2) et $E_T$(H2) sont, respectivement, les niveaux d'énergie de l'orbitale moléculaire inoccupée la plus basse, de l'orbitale moléculaire occupée la plus haute et de l'état triplet excité du composé organique H2 ;
**caractérisé en ce que** 2) (LUMO+1)(H1)-LUMO(H1) $\geq$ 0.1eV, où (LUMO+1)(H1) est le deuxième niveau d'énergie de l'orbitale moléculaire inoccupée la plus basse du composé organique H1.

**2.** Mélange organique selon la revendication 1, dans lequel (LUMO+1)(H1)-LUMO(H1) $\geq$ 0,15eV.

**3.** Mélange organique selon l'une quelconque des revendications 1 à 2, dans lequel le composé organique H1 est un composé représenté par la formule (1) :

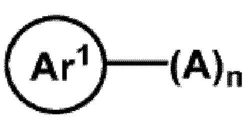

(1)

où $Ar^1$ est choisi parmi un atome d'H, un groupe aromatique contenant 5 à 90 atomes cycliques ou un groupe

hétéroaromatique contenant 5 à 90 atomes cycliques ; A est un groupe accepteur d'électrons ; n est un nombre entier de 1 à 6 ; lorsque n est supérieur à 1, plusieurs groupes accepteurs d'électrons sont identiques ou différents.

**4.** Mélange organique selon la revendication 3, dans lequel le groupe accepteur d' électrons est F ou cyano, tandis que $Ar^1$ n'est pas un atome d'H ; ou le groupe accepteur d'électrons comprend un groupe formé par une ou plusieurs des structures suivantes :

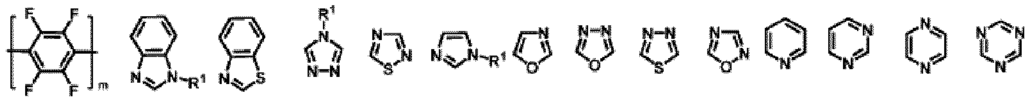

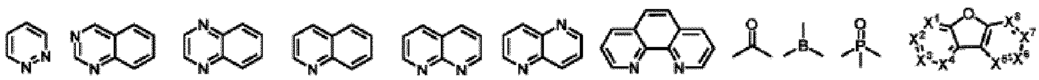

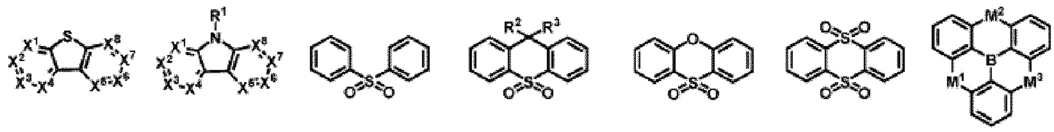

où m est égal à 1, 2 ou 3 ; $X^1$ à $X^8$ sont choisis parmi CR ou N, et au moins un des $X^1$ à $X^8$ est N ; $M^1$, $M^2$ et $M^3$ représentent chacun indépendamment N(R), $C(R)_2$, $Si(R)_2$, O, C=N(R), $C=C(R)_2$, P(R), P(=O)R, S, S=O, $SO_2$ ou aucun ; où $R^1$, $R^2$, $R^3$ et R représentent chacun indépendamment un alkyle, alcoxy, amino, alcényle, alcynyle, aralkyle, hétéroalkyle, aryle ou un hétéroaryle.

**5.** Mélange organique selon l'une quelconque des revendications 1 à 4, dans lequel le composé organique H2 est un composé représenté par la formule (2) :

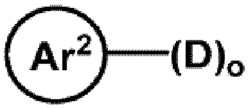
(2)

où $Ar^2$ est choisi parmi un atome d'H, un groupe aromatique contenant 5 à 90 atomes cycliques ou un groupe hétéroaromatique contenant 5 à 90 atomes cycliques ; D est un groupe donneur d'électrons ; o est un nombre entier de 1 à 6 ; lorsque o est supérieur à 1, plusieurs groupes donneurs d'électrons sont identiques ou différents.

**6.** Mélange organique selon la revendication 5, dans lequel le groupe donneur d'électrons comprend un groupe formé par une ou plusieurs des structures suivantes :

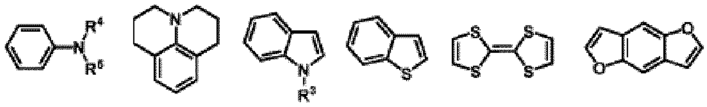

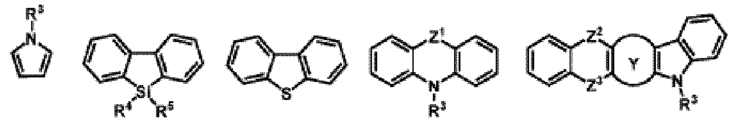

où Y représente un groupe aromatique contenant 5 à 40 atomes de carbone ou un groupe hétéroaromatique contenant 5 à 40 atomes de carbone ; $Z^1$, $Z^2$ et $Z^3$ représentent chacun indépendamment une liaison simple, N(R), $C(R)_2$, $Si(R)_2$, O, S, C=N(R), $C=C(R)_2$ ou P(R) ; où R, $R^3$, $R^4$ et $R^5$ représentent chacun indépendamment un alkyle, alcoxy, amino, alcényle, alcynyle, aralkyle, hétéroalkyle, aryle ou un hétéroaryle.

**7.** Mélange organique selon l'une quelconque des revendications 3 à 6, dans lequel $Ar^1$ ou $Ar^2$ comprend un groupe

formé par une ou plusieurs des structures suivantes :

| | | |
|---|---|---|
| 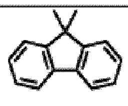 | 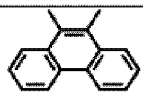 | 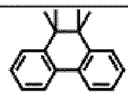 |
| 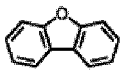 | 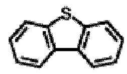 | 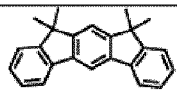 |
| 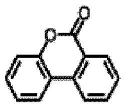 | 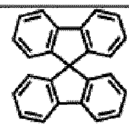 | 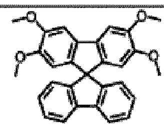 |

| | | |
|---|---|---|
| 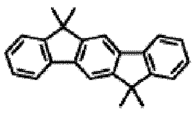 | 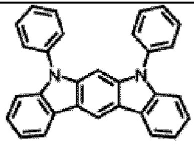 | 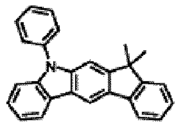 |
| 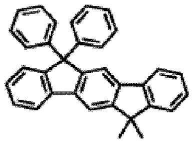 | 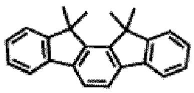 | 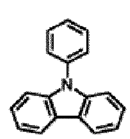 |
| 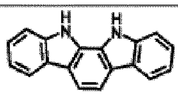 | 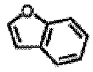 | 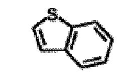 |
| 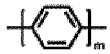 | 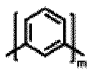 | 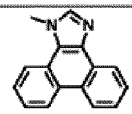 |
| 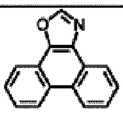 | 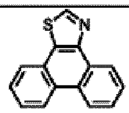 | 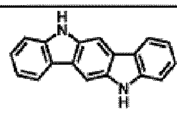 |
| 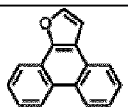 | 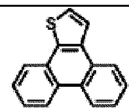 | 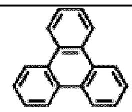 |
| 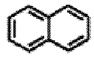 | 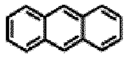 | 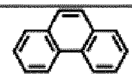 |
| 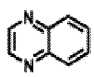 | 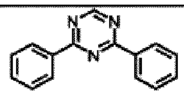 | 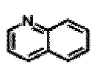 |

où m est égal à 1, 2 ou 3.

**8.** Mélange organique selon l'une quelconque des revendications 2 à 7, dans lequel le composé organique H1 est choisi parmi un ou plusieurs des composés représentés par les formules générales (3) à (6) suivantes :

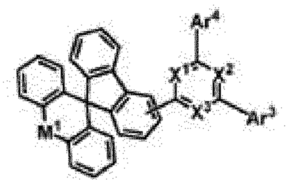 (3) , 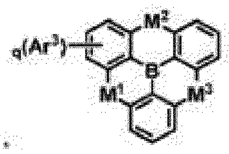 (4) ,

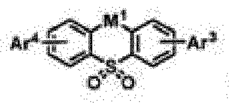 (5) et 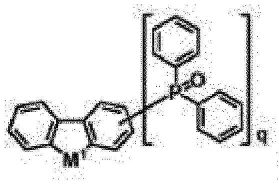 (6) ,

où $Ar^3$ et $Ar^4$ représentent chacun indépendamment un groupe aromatique contenant 5 à 60 atomes cycliques ou un groupe hétéroaromatique contenant 5 à 60 atomes cycliques ; q est un nombre entier de 1 à 6 ; $X^1$, $X^2$ et $X^3$ sont chacun indépendamment choisis à partir de CR ou N, et au moins un de $X^1$, $X^2$ et $X^3$ est N ; $M^1$, $M^2$ et $M^3$ représentent chacun indépendamment N(R), $C(R)_2$, $Si(R)_2$, O, C=N(R), $C=C(R)_2$, P(R), P(=O)R, S, S=O, $SO_2$ ou aucun ; où R représente un alkyle, alcoxy, amino, alcényle, alcynyle, aralkyle, hétéroalkyle, aryle ou un hétéroaryle.

**9.** Mélange organique selon l'une quelconque des revendications 2 à 8, dans lequel le composé organique H1 est choisi parmi un ou plusieurs des composés suivants :

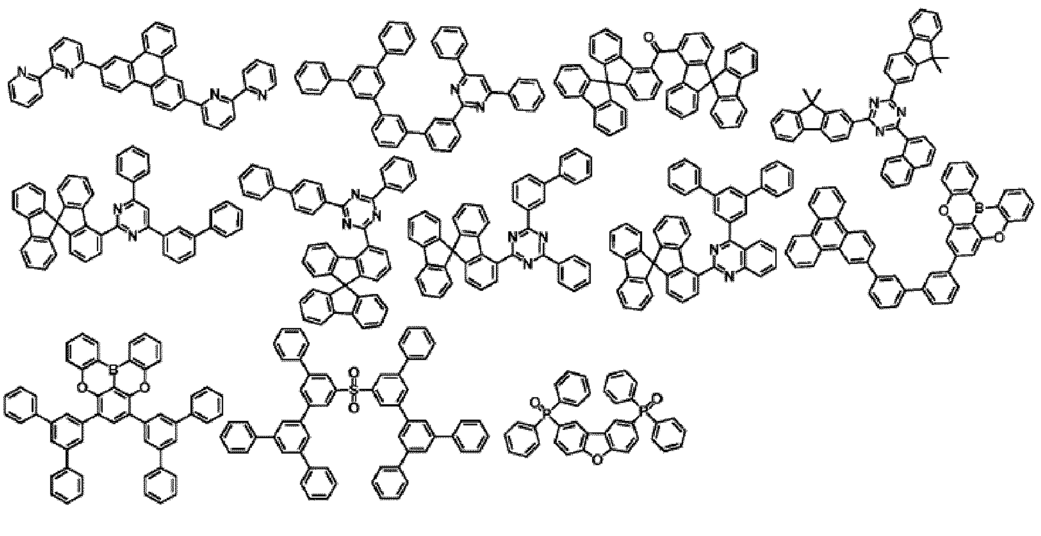

ou le composé organique H2 est un ou plusieurs des composés suivants :

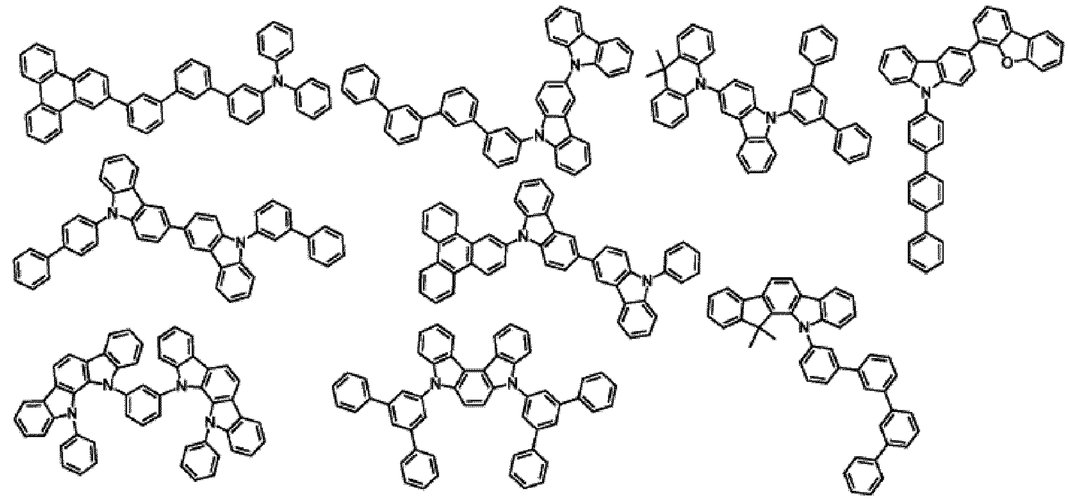

**10.** Mélange organique selon l'une quelconque des revendications 1 à 9, dans lequel la différence de poids moléculaire entre le composé organique H1 et le composé organique H2 ne dépasse pas 100 Daltons.

**11.** Mélange organique selon l'une quelconque des revendications 1 à 10, dans lequel le mélange organique comprend de plus un matériau électroluminescent choisi parmi au moins un élément parmi un émetteur fluorescent, un émetteur phosphorescent et un matériau TADF.

**12.** Formulation **caractérisée en ce qu'**elle comprend le mélange organique selon l'une quelconque des revendications 1 à 11 et un solvant organique.

**13.** Dispositif électronique organique **caractérisé en ce qu'**il comprend une couche fonctionnelle incluant le mélange organique selon l'une quelconque des revendications 1 à 11.

**14.** Dispositif électronique organique selon la revendication 13, dans lequel le dispositif électronique organique 10 est une diode électroluminescente organique (DELO), une cellule photovoltaïque organique, une cellule électrochimique électroluminescente organique, un transistor à effet de champ organique, un transistor à effet de champ électroluminescent organique, un capteur organique ou une diode organique émettrice de plasmon.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2017112713 W
- CN 201611046904
- WO 2011141110 A
- WO 2010135519 A1
- US 20090134784 A1
- WO 2011110277 A1
- JP 2913116 B
- WO 2001021729 A1
- WO 2008006449 A
- WO 2007140847 A
- WO 2006000388 A
- WO 2006058737 A
- WO 2006000389 A
- WO 2007065549 A
- WO 2007115610 A
- US 7250532 B2
- DE 102005058557 A1
- CN 1583691 A
- JP 08053397 A
- US 6251531 B1
- US 2006210830 A
- EP 1957606 A1
- US 20080113101 A1
- US 5121029 A
- WO 2006122630 A
- US 20060222886 A
- US 5130603 A
- US 20070092753 A1
- US 20070252517 A1
- US 4769292 A
- US 6020078 A
- CN 103483332 A
- TW 201309696 A
- TW 201309778 A
- TW 201343874 A
- TW 201350558 A
- US 20120217869 A1
- WO 2013133359 A1
- WO 2013154064 A1
- WO 200070655 A
- WO 200141512 A
- WO 200202714 A
- WO 200215645 A
- EP 1191613 A
- EP 1191612 A
- EP 1191614 A
- WO 2005033244 A
- WO 2005019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- US 20070087219 A1
- US 20090061681 A1
- US 20010053462 A1
- US 6824895 B
- US 7029766 B
- US 6835469 B
- US 6830828 B
- WO 2007095118 A1
- US 2012004407 A1
- WO 2012007088 A1
- WO 2012007087 A1
- WO 2012007086 A1
- US 2008027220 A1
- WO 2011157339 A1
- CN 102282150 A
- WO 2009118087 A1
- WO 2015041428 A


### Non-patent literature cited in the description


- **KIM et al.** *Adv. Func. Mater.,* 2013
- **SATOSHI SEO et al.** *JAPANESE JOURNAL OF APPLIED PHYSICS,* 2014
- Dendrimers and Dendrons. Wiley-VCH Verlag GmbH & Co. KGaA, 2002
- **ADACHI.** *Adv. Mater.,* 2009, vol. 21, 4802
- **ADACHI.** *Appl. Phys. Lett.,* 2011, vol. 98, 083302
- **ADACHI.** *Appl. Phys. Lett.,* 2012, vol. 101, 093306
- **ADACHI.** *Chem. Commun.,* 2012, vol. 48, 11392
- **ADACHI.** *Nature Photonics,* 2012, vol. 6, 253
- **ADACHI.** *Nature,* 2012, vol. 492, 234
- **ADACHI.** *J. Am. Chem. Soc,* 2012, vol. 134, 14706

- **ADACHI.** *Angew. Chem. Int. Ed,* 2012, vol. 51, 11311
- **ADACHI.** *Chem. Commun.,* 2012, vol. 48, 9580
- **ADACHI.** *Chem. Commun.,* 2013, vol. 48, 10385
- **ADACHI.** *Adv. Mater.,* 2013, vol. 25, 3319
- **ADACHI.** *Adv. Mater.,* 2013, vol. 25, 3707
- **ADACHI.** *Chem. Mater.,* 2013, vol. 25, 3038
- **ADACHI.** *Chem. Mater.,* 2013, vol. 25, 3766
- **ADACHI.** *J. Mater. Chem. C.,* 2013, vol. 1, 4599
- **ADACHI.** *J. Phys. Chem. A.,* 2013, vol. 117, 5607
- **BALDO, THOMPSON et al.** *Nature,* 2000, vol. 403, 750-753
- **ADACHI et al.** *Appl. Phys. Lett.,* 2001, vol. 78, 1622-1624
- **J. KIDO et al.** *Appl. Phys. Lett.,* 1994, vol. 65, 2124
- **KIDO et al.** *Chem. Lett.,* 1990, vol. 657
- **JOHNSON et al.** *JACS,* 1983, vol. 105, 1795
- **WRIGHTON.** *JACS,* 1974, vol. 96, 998
- **MA et al.** *Synth. Metals,* 1998, vol. 94, 245
- Handbook of Print Media: Technologies and Production Methods. Helmut Kipphan
- **BULOVIC et al.** *Nature,* 1996, vol. 380, 29
- **GU et al.** *ppl. Phys. Lett.,* 1996, vol. 68, 2606